(19) Europäisches Patentamt
European Patent Office
Office européen des brevets

(11) EP 4 483 880 A1

(12) **EUROPEAN PATENT APPLICATION**
published in accordance with Art. 153(4) EPC

(43) Date of publication:
**01.01.2025 Bulletin 2025/01**

(21) Application number: **23759416.3**

(22) Date of filing: **23.02.2023**

(51) International Patent Classification (IPC):
***A61K 31/517*** *(2006.01)* ***A61K 31/5377*** *(2006.01)*
***A61K 31/4725*** *(2006.01)* ***A61K 31/496*** *(2006.01)*
***A61P 31/12*** *(2006.01)*

(52) Cooperative Patent Classification (CPC):
**A61K 31/4725; A61K 31/496; A61K 31/517; A61K 31/5377; A61K 45/00; A61P 31/12; A61P 31/14; A61P 31/16; A61P 31/18; A61P 31/20; A61P 31/22; A61P 37/04;** Y02A 50/30

(86) International application number:
**PCT/IB2023/051675**

(87) International publication number:
**WO 2023/161844 (31.08.2023 Gazette 2023/35)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **23.02.2022 CN 202210168693**

(71) Applicant: **Asclepieion Pharmaceutical Co., Ltd**
**Suzhou, Jiangsu 215000 (CN)**

(72) Inventors:
- **WEI, Wenjuan**
**Suzhou, Jiangsu 215000 (CN)**
- **PENG, Hong**
**Suzhou, Jiangsu 215000 (CN)**
- **HUANG, Tao**
**Suzhou, Jiangsu 215000 (CN)**
- **HU, Xianjing**
**Suzhou, Jiangsu 215000 (CN)**

(74) Representative: **Mewburn Ellis LLP**
**Aurora Building**
**Counterslip**
**Bristol BS1 6BX (GB)**

# (54) USE OF HPK1 INHIBITOR IN TREATMENT OF INTERFERON-RELATED DISEASES

(57) Disclosed in the present invention is a class of completely-new HPK1 inhibitory active compounds. In the present invention, the intrinsic mechanistic link of HPK1 kinase to viral infection is explained for the first time. The compounds of the present invention are useful in the treatment of interferon-related diseases, particularly viral infections. Further, provided in the present invention is a method for treating interferon-related diseases using the compounds of the present invention.



Processed by Luminess, 75001 PARIS (FR)

## Description

### Technical field

The present invention relates to the field of biomedicine. Specifically, the present invention relates to the use of HPK1 inhibitors in the treatment of interferon-related diseases, particularly in the treatment of viral infections by restoring or enhancing the IFN-β signalling pathway.

### Background

Haematopoietic Progenitor Kinase 1 (HPK1), also known as mitogen-activated protein kinase kinase kinase kinase (MAP4K1), is a serine/threonine protein kinase. It is involved in various cellular events, including mitogen-activated protein kinase signalling, nuclear factor-κB signalling, cytokine signalling, cell proliferation and apoptosis, T-cell receptor/B-cell receptor signalling, and T/B/dendritic cell-mediated immune responses. HPK1 is mainly expressed in blood cells. In T cells, HPK1 is a negative regulator of T cell receptor (TCR) signalling and is responsible for suppressing the immune response. TCR activation leads to the recruitment of HPK1 to the cell membrane, followed by the activation via phosphorylation of the Tyr281\Ser171\Thr165 site. Activated HPK1 destabilises the TCR signalling complex through phosphorylation of the Ser276 site of SLP76 protein, thereby inhibiting T cell activation and proliferation. And HPK1 activation also inhibits the of interferon gamma (IFN-γ). In a series of tumor-immune animal models, the suppression of HPK1 on the immune system can be effectively blocked by HPK1 knockout/knockdown, or using HPK1 inhibitors, thereby improving the body's immunity, and thus enhancing anti-tumor effects (Hernandez et al., Cell Reports, 2018). On the other hand, in mice infected with lymphocytic choroid plexus meningitis virus (LCMV), the viral titre in the plasma of HPK1 knockdown mice was significantly lower than that of wild-type mice at Day 21 post-infection, suggesting that inhibiting HPK1 activity may be a new antiviral therapeutic pathway. WO2020255022 filed by Janssen Sciences Ireland Unlimited Company suggests that the combination of a hepatitis B vaccine (HBV) and a small molecule inhibitor of HPK1 may be an effective therapy for curing hepatitis B.

Despite the above research, an intrinsic mechanism between HPK1 kinase and viral infection has not been elucidated to date; and there have been no reports exploring the use of a HPK1 small molecule inhibitor as a broad-spectrum antiviral agent, alone or in combination (non-vaccine adjuvants), for treating virus-associated diseases.

### Summary of the invention

The purpose of the present invention is to provide a series of novel HPK1 inhibitors which can be used as therapeutic agents for interferon-related diseases, particularly viral infections.

In a first aspect of the present invention, the use of a HPK1 inhibitor in the preparation of a drug for treating an interferon-related disease, or for boosting immunity is provided.

In a preferred embodiment, said interferon is IFN-β and/or IFN-γ.

In a preferred embodiment, said drug is an enhancer of IFN-β and/or IFN-γ signalling pathway.

In a specific embodiment, said interferon-related disease is a viral infection.

In preferred embodiments, said viral infection is a viral infection on which IFN-β/IPN-γ has therapeutic and/or prophylactic effects.

In specific embodiments, the HPK1 inhibitor is a compound shown in Formula I or a pharmaceutically acceptable salt, solvate, stereoisomer, tautomer, prodrug, metabolite or derivative thereof,

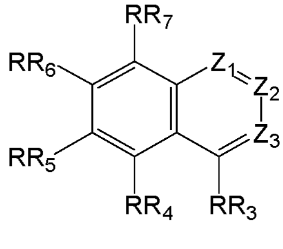

I

wherein $Z_1$ is selected from Nor C-$RR_1$;

$Z_2$ and $Z_3$ are each selected from N or C-$RR_2$, wherein $Z_2$ and $Z_3$ are not identical;

$RR_1$ is selected from a hydrogen, halogen, cyano, hydroxy, substituted or unsubstituted $C_{1-6}$ alkyl, substituted or unsubstituted $C_{3-6}$ cycloalkyl, substituted or unsubstituted $C_{1-6}$ alkoxy, substituted or unsubstituted amino or substituted or unsubstituted acylamino;

$RR_2$ is selected from a substituted or unsubstituted hydroxyl, substituted or unsubstituted amino, or substituted or unsubstituted sulfhydryl;

$RR_3$ is selected from H, hydroxy, halogen, cyano, substituted or unsubstituted $C_{1-6}$ alkyl, substituted or unsubstituted $C_{3-8}$ cycloalkyl, substituted or unsubstituted $C_{1-6}$ alkoxy, substituted or unsubstituted amino, or substituted or unsubstituted acylamino;

$RR_4$, $RR_5$, $RR_6$, $RR_7$ are each independently selected from H, hydroxy, halogen, cyano, substituted or unsubstituted $C_{1-6}$ alkyl, substituted or unsubstituted $C_{3-8}$ cycloalkyl, substituted or unsubstituted $C_{1-6}$ alkoxy, substituted or unsubstituted amino, substituted or unsubstituted acylamino, substituted or unsubstituted $C_{5-10}$ heteroaryl, or $RR_8$-$Z_4$-, respectively;

$RR_8$ is selected from a substituted or unsubstituted alkyl, substituted or unsubstituted cycloalkyl, substituted or unsubstituted heterocyclyl, or substituted or unsubstituted hydroxy;

$Z_4$ is selected from -O-, -NH-, -S-, -SO-, -SO2-, carbonyl, carbonylamino or aminocarbonyl.

In specific embodiments, the compound shown in Formula I is a compound shown in Formula 1-1:

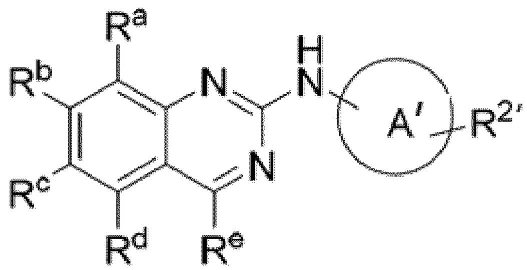

I-1

wherein one of $R^a$, $R^b$, $R^c$, $R^d$ is $R^{1'}$-X'- and the rest are each independently selected from H, hydroxyl, halogen, cyano, substituted or unsubstituted $C_{1-6}$ alkyl, substituted or unsubstituted $C_{3-8}$ cycloalkyl, or substituted or unsubstituted $C_{1-6}$ alkoxy;

$R^e$ is selected from H, hydroxy, halogen, cyano, substituted or unsubstituted $C_{1-6}$ alkyl, substituted or unsubstituted $C_{3-8}$ cycloalkyl, or substituted or unsubstituted $C_{1-6}$ alkoxy;

X' is selected from -O-, -NH-, -S-, -SO-, -SO2-, carbonyl, carbonylamino or aminocarbonyl;

$R^{1'}$ is a substituted or unsubstituted cycloalkyl, substituted or unsubstituted heterocycloalkyl or $R^{14'}$-O-$(CH_2)_m$-, wherein m is 0, 1, 2, 3, 4 or 5, and $R^{14'}$ is a substituted or unsubstituted $C_{1-6}$ alkyl or substituted or unsubstituted $C_{3-8}$ cycloalkyl;

ring A' is a substituted or unsubstituted aryl (preferably a substituted or unsubstituted phenyl) or substituted or unsubstituted 5-6-membered heteroaryl, wherein said heteroaryl has 1-4 heteroatoms selected from O, S or N;

$R^{2'}$ is selected from following substituents:

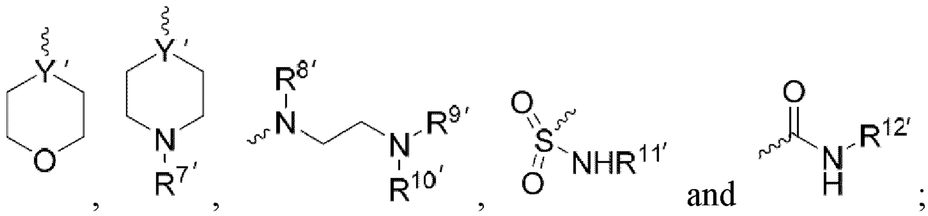

wherein Y' is C or N, $R^{7'}$ is H, substituted or unsubstituted $C_{1-6}$ alkyl or substituted or unsubstituted $C_{3-8}$ cycloalkyl, $R^{8'}$ is H, substituted or unsubstituted $C_{1-6}$ alkyl or substituted or unsubstituted $C_{3-8}$ cycloalkyl, $R^{9'}$ and $R^{10'}$ are independently H, substituted or unsubstituted $C_{1-6}$ alkyl or substituted or unsubstituted $C_{3-8}$ cycloalkyl, $R^{11'}$ is H, substituted or unsubstituted amino, substituted or unsubstituted $C_{1-6}$ alkyl or substituted or unsubstituted $C_{3-8}$ cycloalkyl, and $R^{12'}$ is a substituted or unsubstituted 4-6-membered heterocyclyl.

In a preferred embodiment, said $R^{1'}$ is a substituted or unsubstituted 5-8-membered cycloalkyl or a substituted or unsubstituted 5-8-membered heterocyclyl, wherein said heterocyclyl has 1-4 heteroatoms selected from O, S or N.

In a preferred embodiment, when $R^{1'}$ is a substituted 5-8-membered cycloalkyl, $R^{1'}$ is

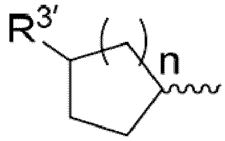,

wherein n is 1, 2, 3 or 4;

$R^{3'}$ is $-O^{R4'}$ or $-NR^{5'}R^{6'}$, wherein $R^{4'}$ is H,

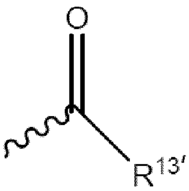,

substituted or unsubstituted alkyl, or substituted or unsubstituted cycloalkyl, $R^{5'}$ and $R^{6'}$ are each independently H,

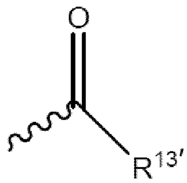,

substituted or unsubstituted alkyl, or substituted or unsubstituted cycloalkyl, wherein $R^{5'}$ and $R^{6'}$ are not both a substituted or unsubstituted cycloalkyl.

Said $R^{13'}$ is H, substituted or unsubstituted $C_{1-6}$ alkyl, substituted or unsubstituted $C_{3-8}$ cycloalkyl.

In a preferred embodiment, said A ring is selected from following groups:

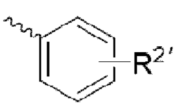, 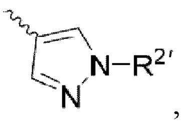, 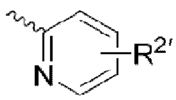, 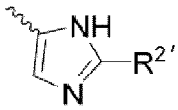, 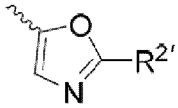 and 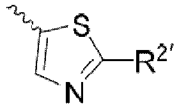.

In a specific embodiment, the compound shown in Formula I-1 is shown in Formula I-1-1,

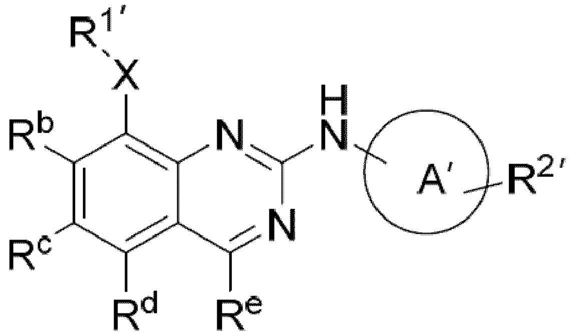

I-1-1

wherein $R^b$, $R^c$, $R^d$ and $R^e$ are each independently selected from H, hydroxyl, halogen, cyano, substituted or unsubstituted $C_{1-6}$ alkyl, substituted or unsubstituted $C_{3-8}$ cycloalkyl, or substituted or unsubstituted $C_{1-6}$ alkoxy;

X' is selected from -O-, -NH-, -S-, -SO-, $-SO_2-$, carbonyl, carbonylamino or aminocarbonyl;

$R^{1'}$ is a substituted or unsubstituted cycloalkyl, substituted or unsubstituted heterocycloalkyl or $R^{14'}-O-(CH_2)_m-$, wherein m is 0, 1, 2, 3, 4 or 5, and $R^{14'}$ is a substituted or unsubstituted $C_{1-6}$ alkyl or substituted or unsubstituted $C_{3-8}$ cycloalkyl;

ring A' is a substituted or unsubstituted aryl (preferably a substituted or unsubstituted phenyl) or substituted or unsubstituted 5-6-membered heteroaryl, wherein said heteroaryl has 1-4 heteroatoms selected from O, S or N; $R^{2'}$ is selected from following substituents:

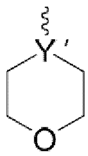 , 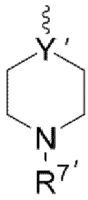 , 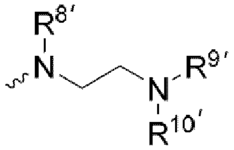 , 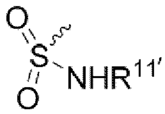 and 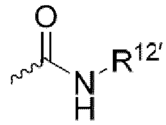 ;

wherein Y' is C or N, $R^{7'}$ is H, substituted or unsubstituted $C_{1-6}$ alkyl or substituted or unsubstituted $C_{3-8}$ cycloalkyl, $R^{8'}$ is H, substituted or unsubstituted $C_{1-6}$ alkyl or substituted or unsubstituted $C_{3-8}$ cycloalkyl, $R^{9'}$ and $R^{10'}$ are independently H, substituted or unsubstituted $C_{1-6}$ alkyl or substituted or unsubstituted $C_{3-8}$ cycloalkyl, $R^{11'}$ is H, substituted or unsubstituted amino, substituted or unsubstituted $C_{1-6}$ alkyl or substituted or unsubstituted $C_{3-8}$ cycloalkyl, and $R^{12'}$ is a substituted or unsubstituted 4-6-membered heterocyclyl.

In a specific embodiment, the compound shown in Formula I-1 is shown in Formula I-1-1-1 or 1-1-1-2,

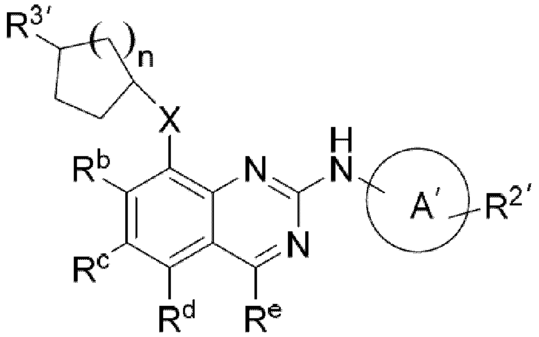

I-1-1-1

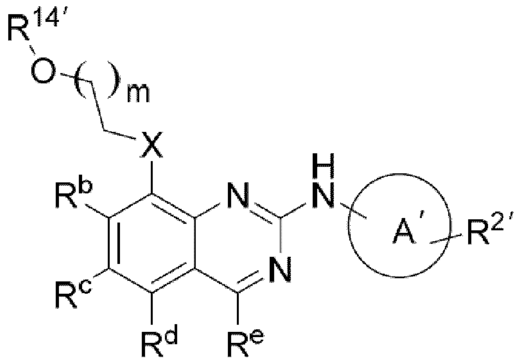

I-1-1-2

wherein $R^b$, $R^c$, $R^d$ and $R^e$ are each independently selected from H, hydroxyl, halogen, cyano, substituted or unsubstituted $C_{1-6}$ alkyl, substituted or unsubstituted $C_{3-8}$ cycloalkyl, or substituted or unsubstituted $C_{1-6}$ alkoxy;
X is selected from -O-, -NH-, -S-, -SO-, $-SO_2-$, carbonyl, carbonylamino or aminocarbonyl;
n is 1, 2, 3 or 4;
m is 0, 1, 2, 3, 4 or 5,
$R^{14'}$ is a substituted or unsubstituted $C_{1-6}$ alkyl or substituted or unsubstituted $C_{3-8}$ cycloalkyl;
ring A' is a substituted or unsubstituted aryl (preferably a substituted or unsubstituted phenyl) or substituted or unsubstituted 5-6-membered heteroaryl, wherein said heteroaryl has 1-4 heteroatoms selected from O, S or N;
$R^{2'}$ is selected from following substituents:

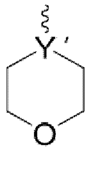 , 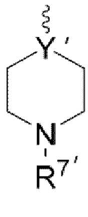 , 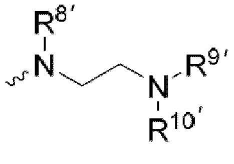 , 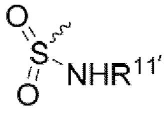 and 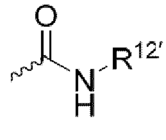 ;

wherein Y' is C or N, $R^{7'}$ is H, substituted or unsubstituted $C_{1-6}$ alkyl or substituted or unsubstituted $C_{3-8}$ cycloalkyl, $R^{8'}$ is H, substituted or unsubstituted $C_{1-6}$ alkyl or substituted or unsubstituted $C_{3-8}$ cycloalkyl, $R^{9'}$ and $R^{10'}$ are independently H, substituted or unsubstituted $C_{1-6}$ alkyl or substituted or unsubstituted $C_{3-8}$ cycloalkyl, $R^{11'}$ is H, substituted or unsubstituted amino, substituted or unsubstituted $C_{1-6}$ alkyl or substituted or unsubstituted $C_{3-8}$ cycloalkyl, and $R^{12'}$ is a substituted or unsubstituted 4-6-membered heterocyclyl.

In a specific embodiment, the compound is selected from the following group:

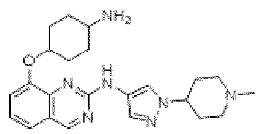 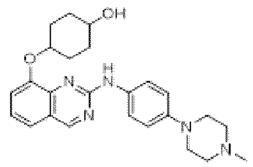 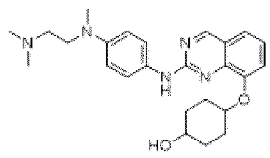 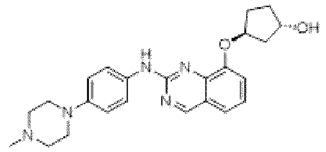

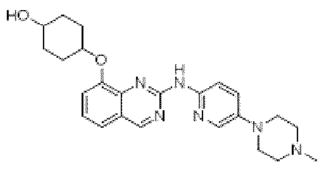 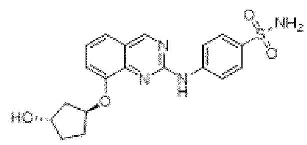 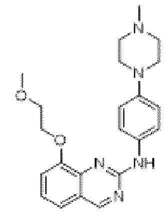 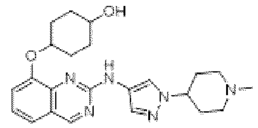

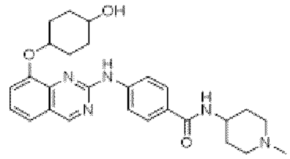 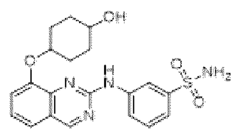 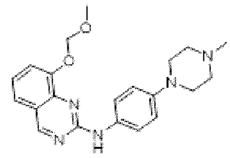 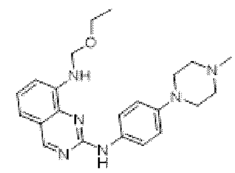

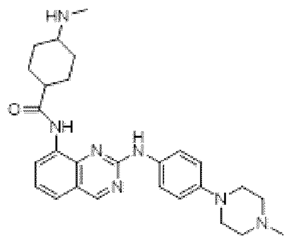 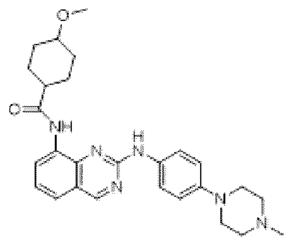 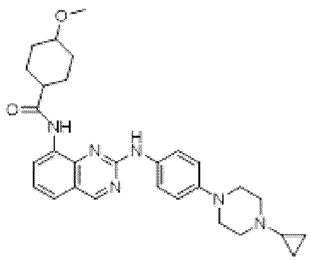 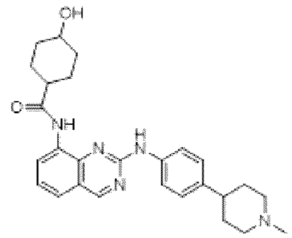

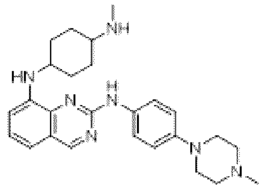 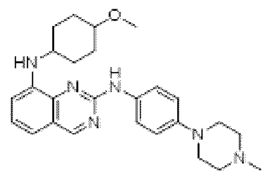 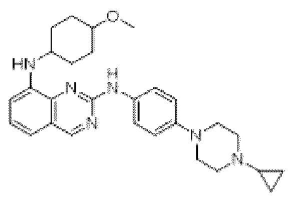 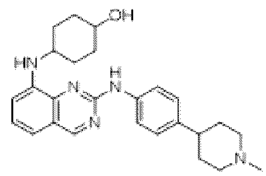

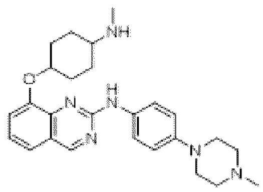 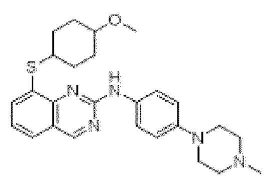 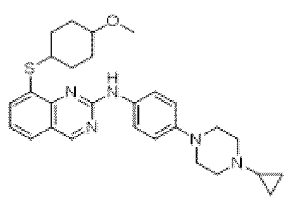 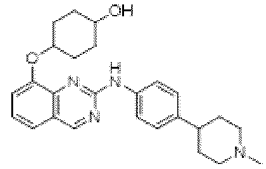

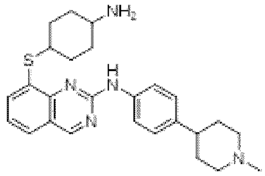 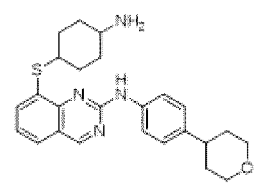 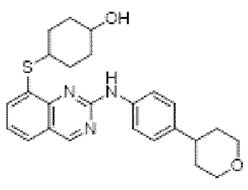 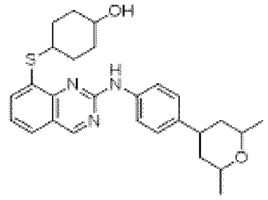

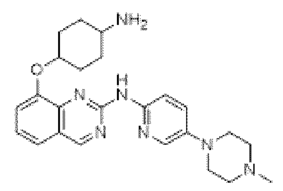
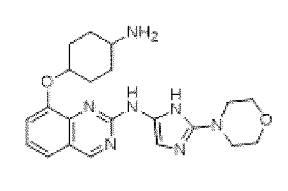
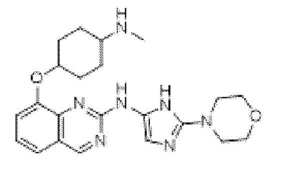
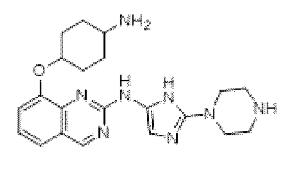
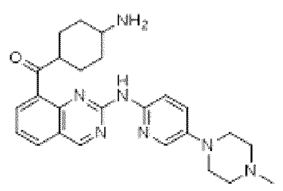
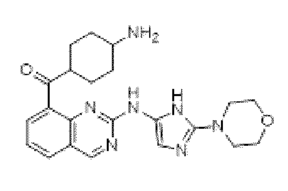
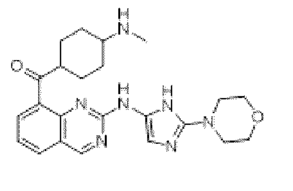
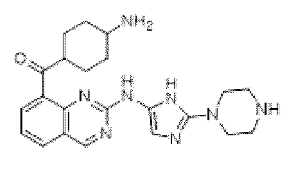
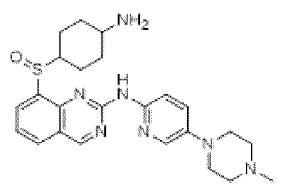
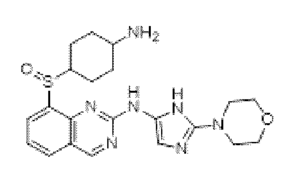
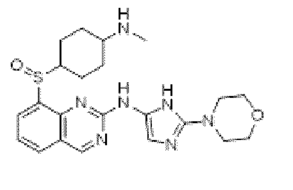
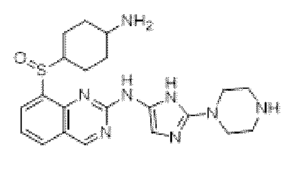
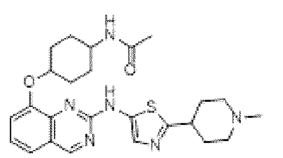
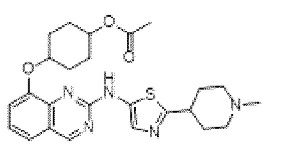
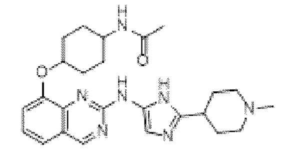
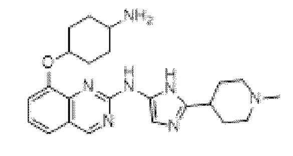
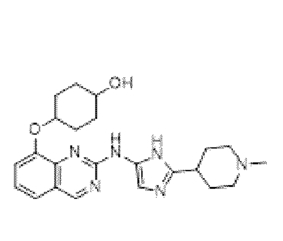
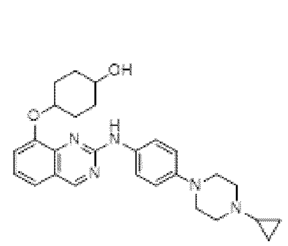
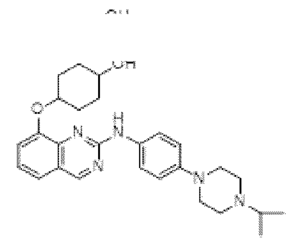
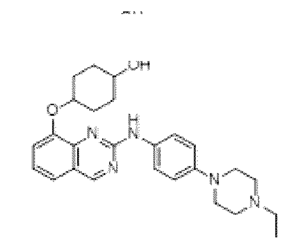
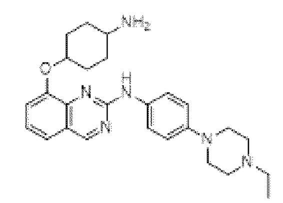
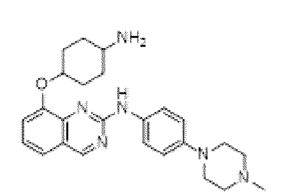
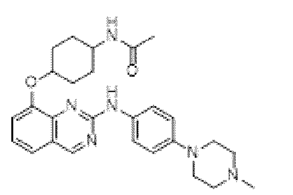
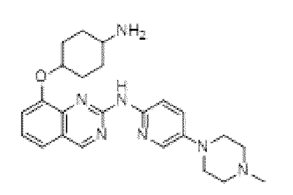
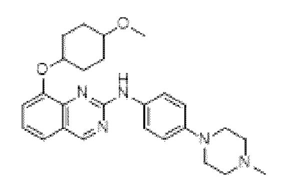
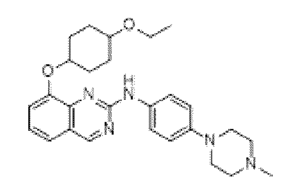
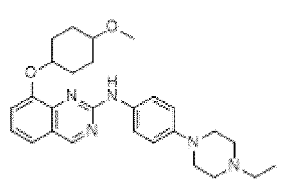
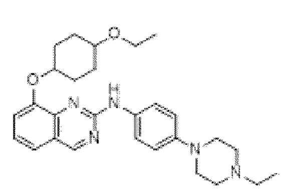
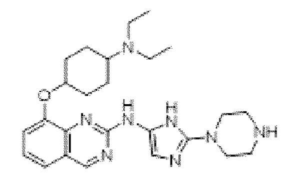
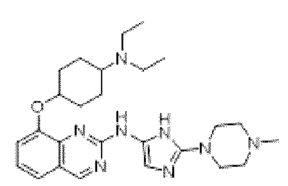
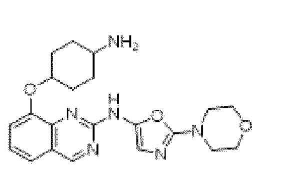
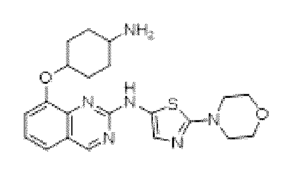

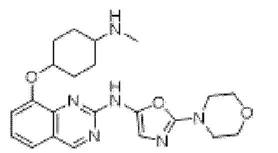 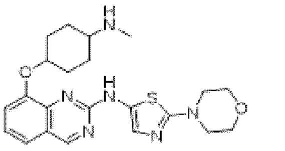 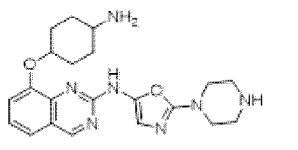 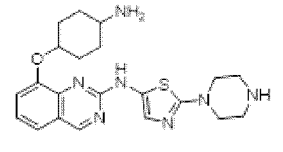

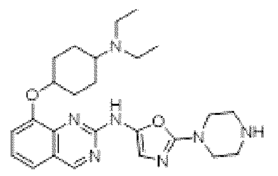 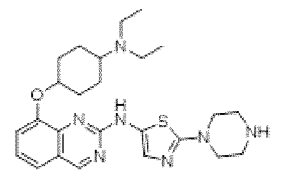 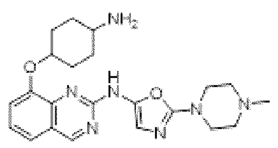 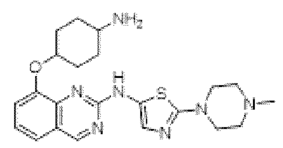

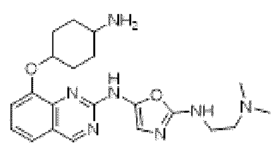 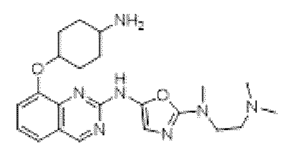 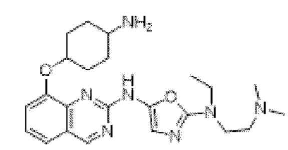 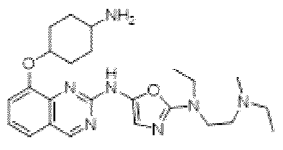

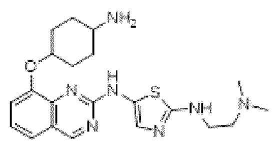 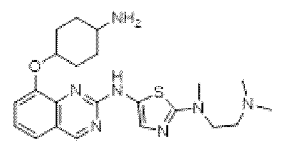 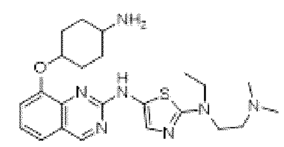 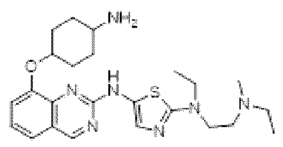

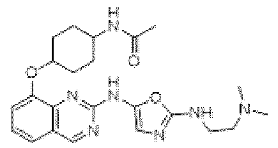 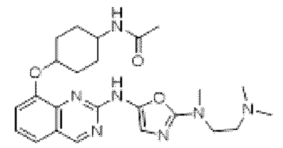 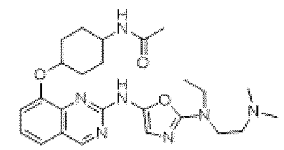 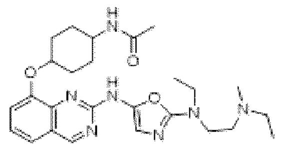

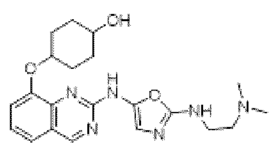 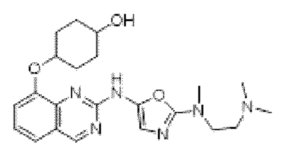 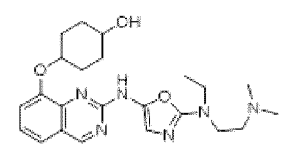 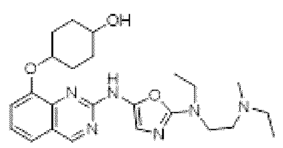

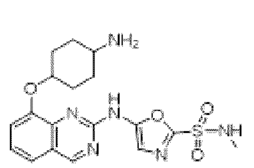 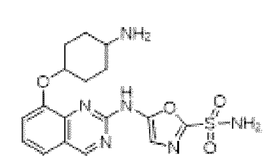 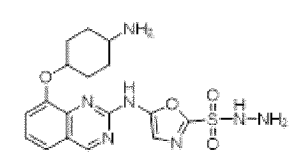 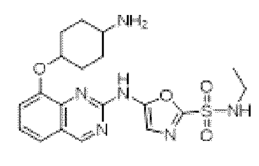

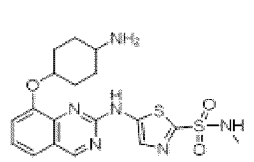 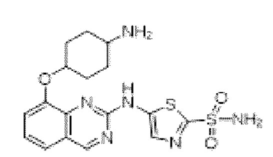 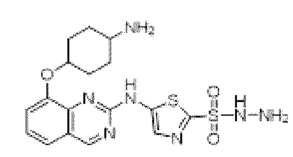 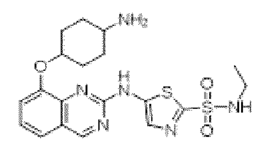

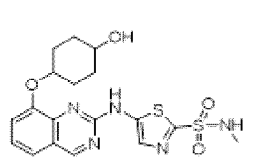 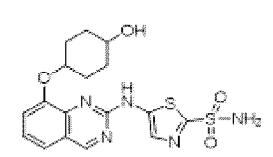 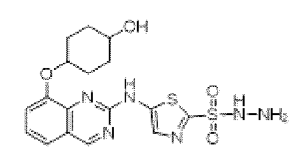 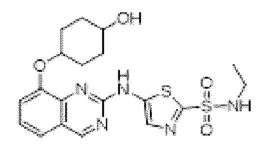

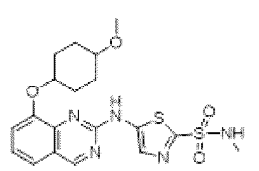
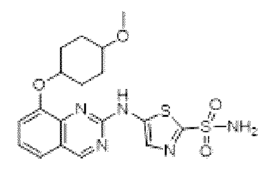
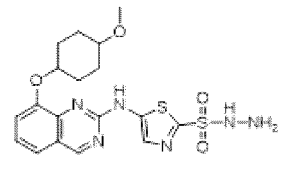
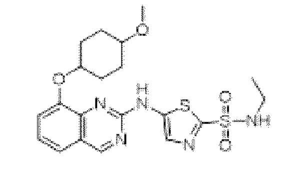

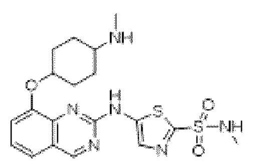
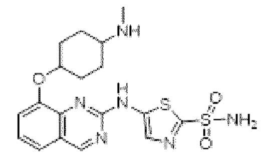
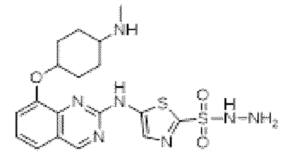
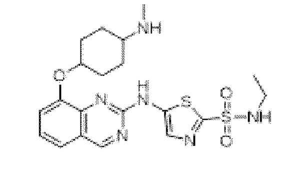

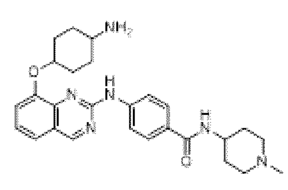
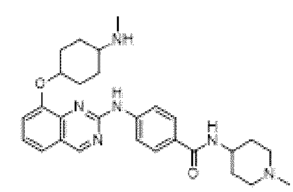
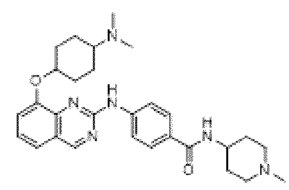
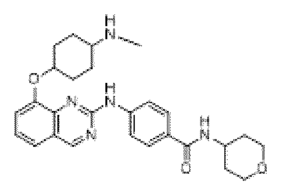

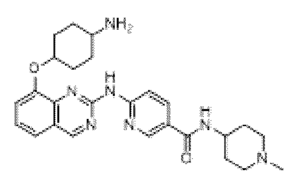
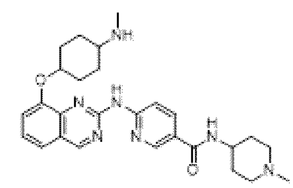
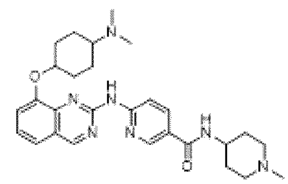
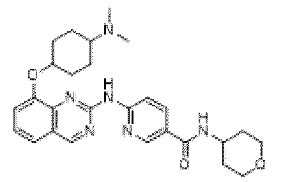

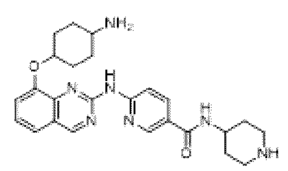
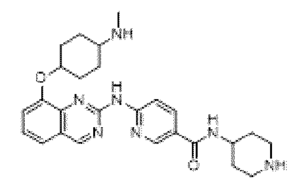
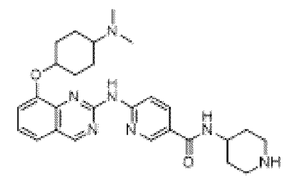
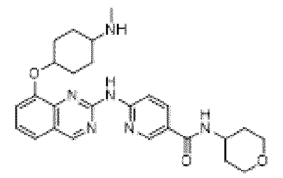

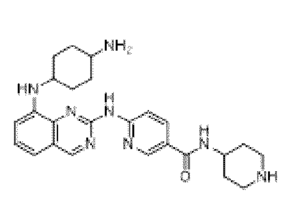
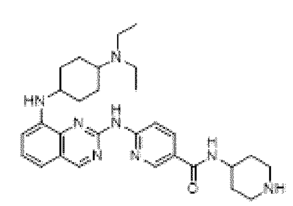
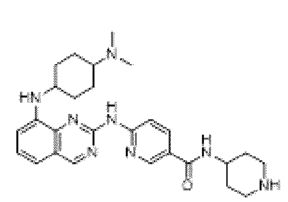
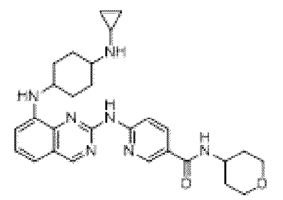

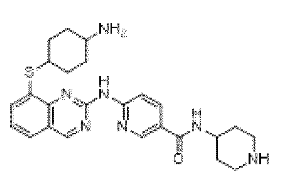
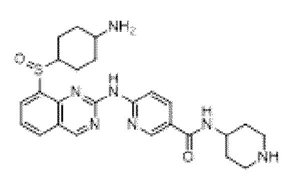
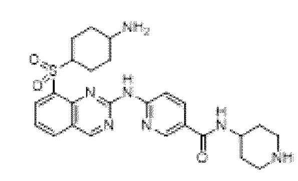
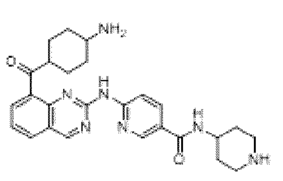

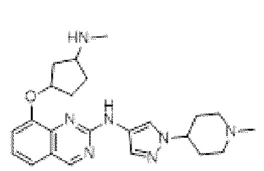
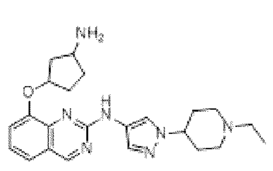
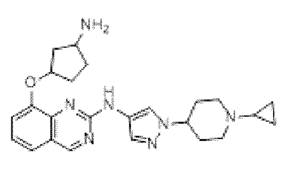
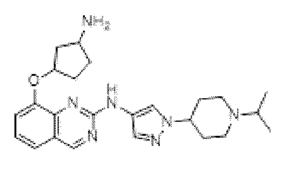

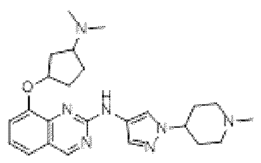
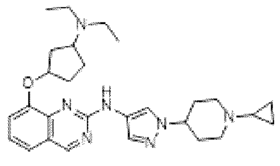
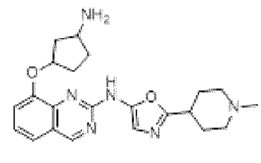
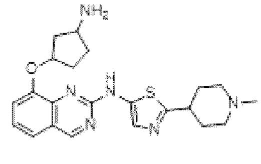

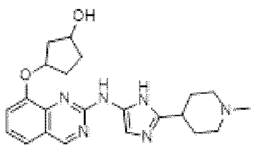
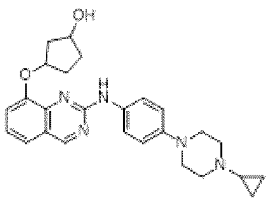
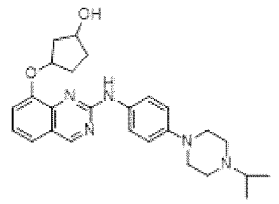
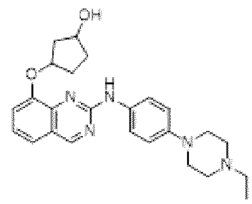

In a specific embodiment, the compound shown in Formula I is a compound shown in Formula I-2,

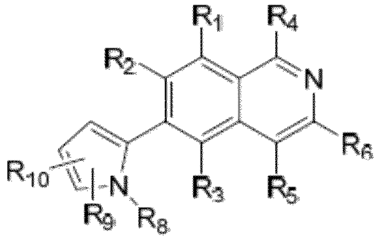

I-2

wherein $R_1$, $R_2$, $R_3$, $R_4$ and $R_5$ are each independently selected from: a hydrogen, halogen, cyano, hydroxy, substituted or unsubstituted $C_{1-6}$ alkyl, substituted or unsubstituted $C_{3-6}$ cycloalkyl, substituted or unsubstituted $C_{1-6}$ alkoxy, substituted or unsubstituted amino, or substituted or unsubstituted acylamino, and none of $R_1$ and $R_2$ is a hydrogen;

$R_6$ is:

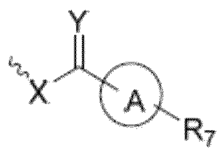

X is O or NH or S, Y is O or NH or S, A ring is a substituted or unsubstituted $C_{3-6}$ cycloalkyl or a substituted or unsubstituted $C_{6-10}$ aryl or a substituted or unsubstituted $C_{5-10}$ heteroaryl, said heteroaryl means an aryl comprising one or more N, O, or S heteroatoms, $R_7$ is a mono- or multi-substitution on various types of rings, including a halogen, cyano, hydroxyl, amino, amido, branched or straight chain $C_{1-6}$ alkyl, $C_{2-6}$ alkenyl, $C_{2-6}$ alkynyl, $C_{3-6}$ heterocyclyl, $C_{6-10}$ aryl, $C_{5-10}$ heteroaryl or -C(O)R', wherein said R' is a $C_{3-6}$ heterocyclyl, $C_{6-10}$ aryl, or $C_{5-10}$ heteroaryl, and said $C_{1-6}$ alkyl, $C_{2-6}$ alkenyl, $C_{2-6}$ alkynyl, $C_{3-6}$ heterocyclyl, $C_{6-10}$ aryl, and $C_{5-10}$ heteroaryl further comprise various types of mono- or multi-substitution including a hydrogen, hydroxyl, halogen, cyano, amino, $-CHF_2$, $-CF_3$, $-CH_3$, $-CH_2CH_2OH$, or $-CONH_2$; alternatively, $R_7$, together with the ring A, forms a substituted or unsubstituted fused ring group, bridged ring group, heterobridged ring group, spiro ring group or hetero spiro ring group;

$R_8$, $R_9$ and $R_{10}$ are each independently selected from:

i. a hydrogen, hydroxyl, halogen, cyano, amino, di($C_{1-6}$ alkyl)amino, mono($C_{1-6}$ alkyl)amino or $C_{1-6}$ alkoxy;
ii. a branched or straight chain $C_{1-6}$ alkyl, $C_{2-6}$ alkenyl, or $C_{2-6}$ alkynyl, and the alkyl, alkenyl and alkynyl further comprise various mono- or multi-substitution, including a hydrogen, hydroxyl, halogen, cyano, amino, $-CHF_2$, $-CF_3$, di($C_{1-6}$ alkyl)amino, mono($C_{1-6}$ alkyl)amino, $C_{3-6}$ cycloalkyl, or $C_{1-6}$ alkoxy;
iii. $C_{3-6}$ cycloalkyl, $C_{3-6}$ heterocycloalkyl, $C_{6-10}$ aryl or $C_{5-10}$ heteroaryl, wherein the cycloalkyl, heterocycloalkyl, aryl or heteroaryl comprises various mono- or multi-substitution, including a hydrogen, $C_{1-6}$ alkyl, hydroxyl, halogen, cyano, amino, $-CHF_2$ or $-CF_3$.

In a specific embodiment, the compound shown in Formula I-2 is shown in Formula I-2-1,

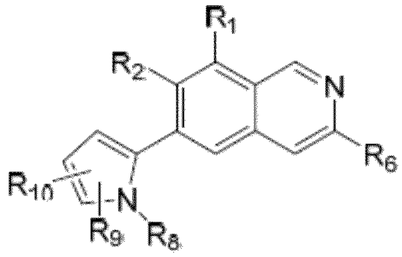

I-2-1

wherein $R_1$, $R_2$, $R_6$, $R_8$, $R_9$, and $R_{10}$ are each defined as above.

In a specific embodiment, the compound shown in Formula I-2 is shown in Formula I-2-2,

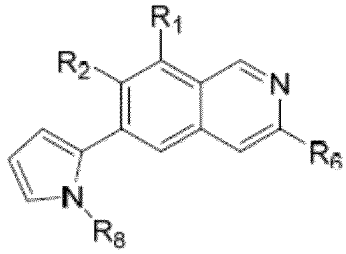

I-2-2

wherein $R_1$, $R_2$, $R_6$ and $R_8$ are each defined as above.

In a preferred embodiment, X is NH.

In a specific embodiment, the compound shown in Formula I-2 is shown in Formula I-2-3,

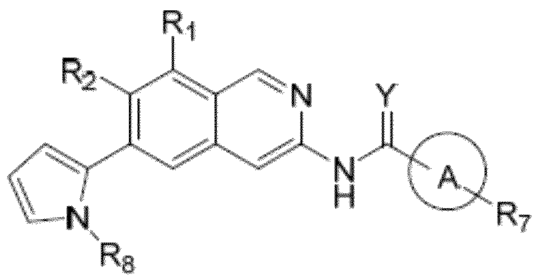

I-2-3

wherein $R_1$, $R_2$, $R_8$ and A are each defined as above.

In a preferred embodiment, the A ring is selected from the following group:

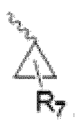 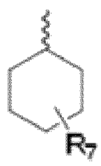 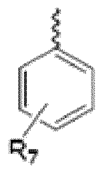 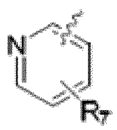 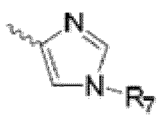 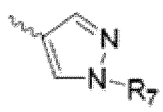 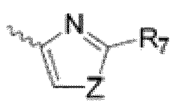 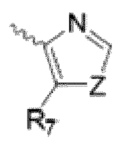 ,

wherein Z is O or S.

In a preferred embodiment, $R_7$ is selected from: a halogen, cyano, hydroxyl, amino, amide, methyl, ethyl, isopropyl, cyclopropyl, vinyl, acetylene,

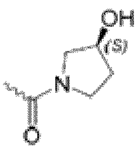 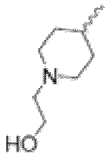 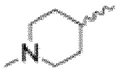 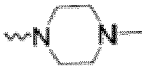 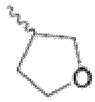 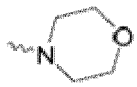 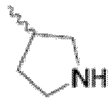 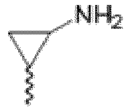 .

In a preferred embodiment, the A ring, together with $R_7$, forms an optionally substituted fused ring group, bridged ring group, heterobridged ring group, spiro ring group, or hetero spiro ring group selected from the following group:

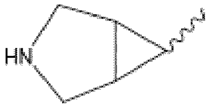 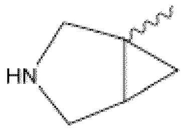 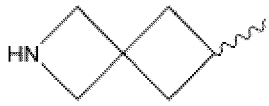 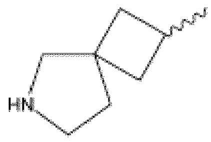 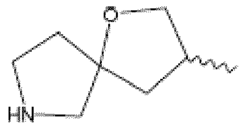

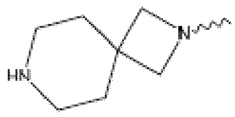 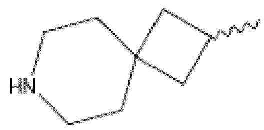 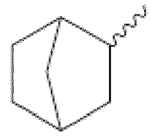 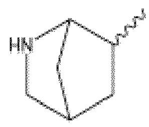 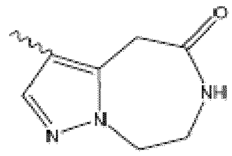

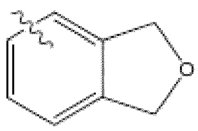 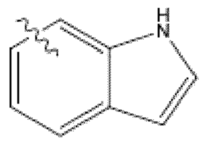 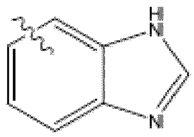 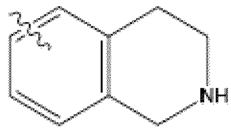 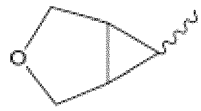

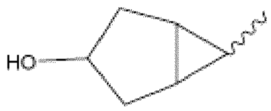 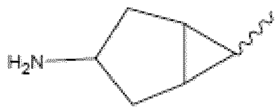 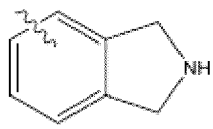

In a preferred embodiment, $R_1$ is an amino, hydroxyl, cyano, methoxy, or halogen substituent.

In a preferred embodiment, $R_8$ is a methyl, ethyl, isopropyl, cyclopropyl, ethenyl, ethynyl, or substituted or unsubstituted aryl (preferably phenyl) or 5-membered heteroaryl.

In a preferred embodiment, $R_8$ is a methyl, ethyl, isopropyl, cyclopropyl, substituted or unsubstituted phenyl.

In a specific embodiment, the compound is selected from:

| No. | Structure | Name |
|---|---|---|
| 1 | 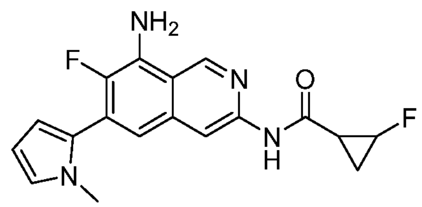 | N-(8-amino-7-fluoro-6-(1-methyl-1H-pyrrole-2-yl)isoquinolin-3-yl)-2-fluorocyclopropan-1-carboxamide |
| 2 | 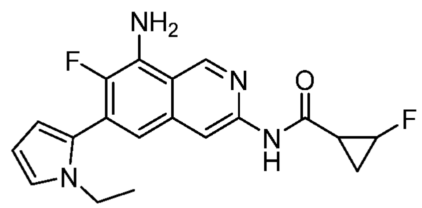 | N-(8-amino-6-(1-ethyl-1H-pyrrole-2-yl)-7-fluoroi soquinolin-3-yl)-2-fluorocyclopropan-1-carboxamide |

(continued)

| No. | Structure | Name |
| --- | --- | --- |
| 3 | 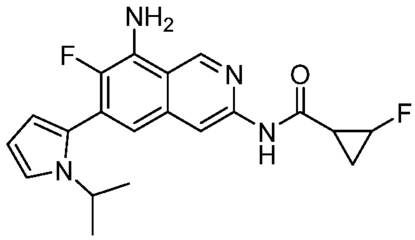 | N-(8-amino-7-fluoro-6-(1-isopropyl-1H-pyrrole-2-yl) isoquinolin-3-yl)-2-fluorocyclopropan-1-carboxamide |
| 4 | 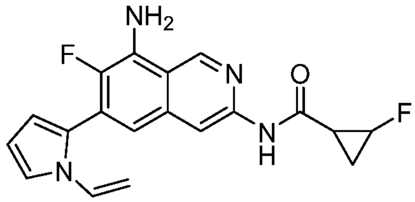 | N-(8-amino-7-fluoro-6-(1-ethenyl-1H-pyrrole-2-yl) isoquinolin-3-yl)-2-fluorocyclopropan-1-carboxamide |
| 5 | 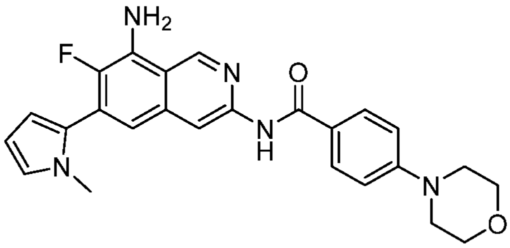 | N-(8-amino-7-fluoro-6-(1-methyl-1H-pyrrole-2-yl)isoquinolin-3-yl)-4-morpholinobenzamide |
| 6 | 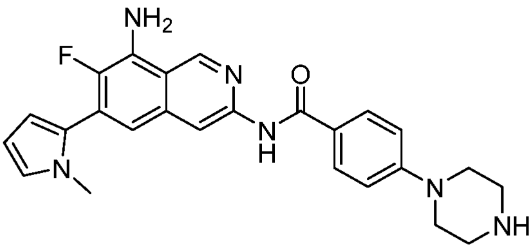 | N-(8-amino-7-fluoro-6-(1-methyl-1H-pyrrole-2-yl)isoquinolin-3-yl)-4-(piperazin-1-yl)benzamide |
| 7 | 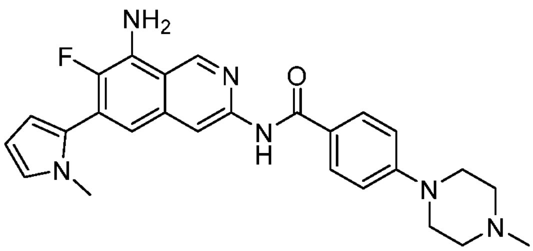 | N-(8-amino-7-fluoro-6-(1-methyl-1H-pyrrole-2-yl)isoquinolin-3-yl)-4-(4-methylpiperazin-1-yl)benzamide |
| 8 | 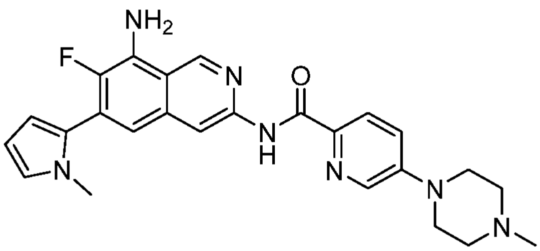 | N-(8-amino-7-fluoro-6-(1-methyl-1H-pyrrole-2-yl)isoquinolin-3-yl)-5-(4-methylpiperazin-1-yl)pyridinolinoamide |

(continued)

| No. | Structure | Name |
|---|---|---|
| 9 | 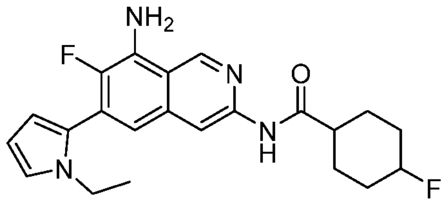 | N-(8-amino-6-(1-ethyl-1H-pyrrole-2-yl)-7-fluoroisoquinolin-3-yl)-4-fluorocyclohexan-1-carboxamide |
| 10 | 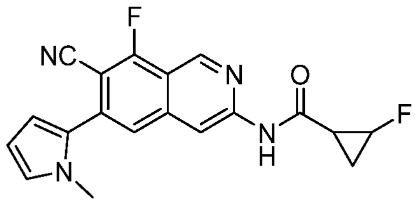 | N-(7-cyano-8-fluoro-6-(1-methyl-1H-pyrrole-2-yl)isoquinolin-3-yl)-2-fluorocyclopropan-1-carboxamide |
| 11 | 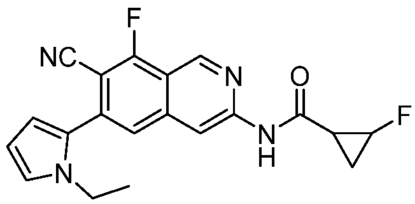 | N-(7-cyano-6-(1-ethyl-1H-pyrrole-2-yl)-8-fluoroi soquinolin-3-yl)-2-fluorocyclopropan-1-carboxamide |
| 12 | 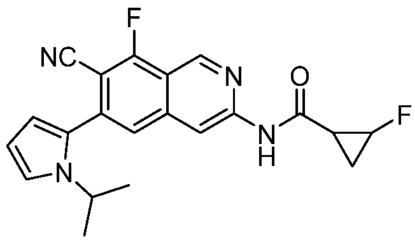 | N-(7-cyano-8-fluoro-6-(1-isopropyl-1H-pyrrole-2-yl) isoquinolin-3-yl)-2-fluorocyclopropan-1-carboxa-mide |
| 13 | 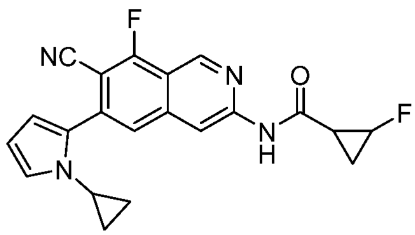 | N-(7-cyano-6-(1-cyclopropyl-1H-pyrrole-2-yl)-8-fluoroisoquinolin-3-yl)-2-fluorocyclopropan-1-car-boxamide |
| 14 | 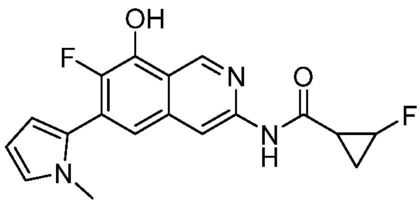 | 2-fluoro-N-(7-fluoro-8-hydroxy-6-(1-methyl-1H-pyrrole-2-yl)isoquinolin-3-yl)cyclopropan-1-carboxa-mide |
| 15 | 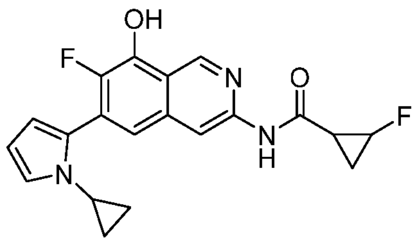 | N-(6-(1-cyclopropyl-1H-pyrrole-2-yl)-7-fluoro-8-hydroxyisoquinolin-3-yl)-2-fluorocyclopropan-1-car-boxamide |

(continued)

| No. | Structure | Name |
|---|---|---|
| 16 | 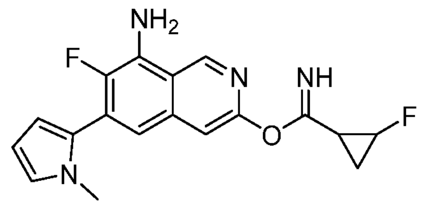 | 8-amino-7-fluoro-6-(1-methyl-1H-pyrrole-2-yl)isoquinolin-3-yl 2-fluorocyclopropan-1-carbamate |
| 17 | 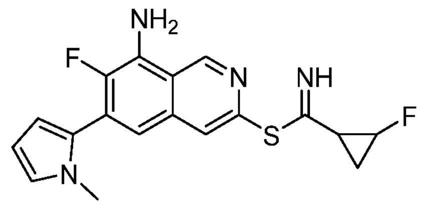 | 8-amino-7-fluoro-6-(1-methyl-1H-pyrrole-2-yl)isoquinolin-3-yl 2-fluorocyclopropan-1-carbonimidothioate |
| 18 | 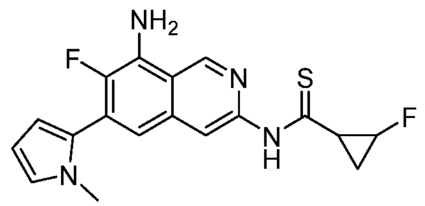 | N-(8-amino-7-fluoro-6-(1-methyl-1H-pyrrole-2-yl)isoquinolin-3-yl)-2-fluorocyclopropan-1 -carbothioamide |
| 19 | 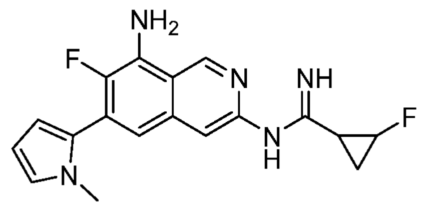 | N-(8-amino-7-fluoro-6-(1-methyl-1H-pyrrole-2-yl)isoquinolin-3-yl)-2-fluorocyclopropan-1 -carboximide |
| 20 | 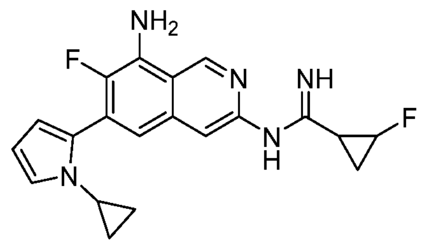 | N-(8-amino-6-(1-cyclopropyl-1H-pyrrole-2-yl)-7-fluoroisoquinolin-3-yl)-2-fluorocyclopropan-1 -carboximide |
| 21 | 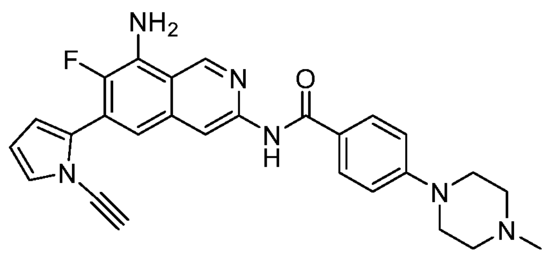 | N-(8-amino-6-(1-ethynyl-1H-pyrrole-2-yl)-7-fluoroisoquinolin-3 -yl)-4-(4-methylpiperazin-1-yl)benzamide |
| 22 | 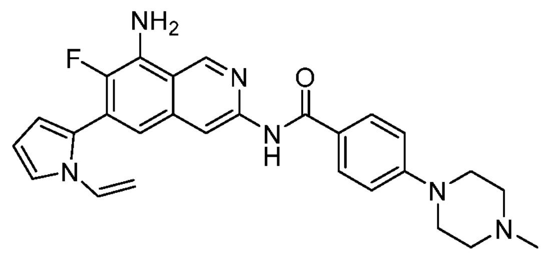 | N-(8-amino-7-fluoro-6-(1-ethenyl-1H-pyrrole-2-yl) isoquinolin-3-yl)-4-(4-methylpiperazin-1-yl)benzamide. |

In a specific embodiment, the compound is

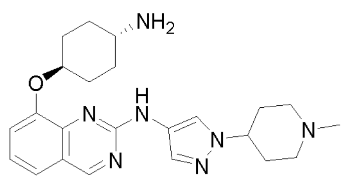

**C1**

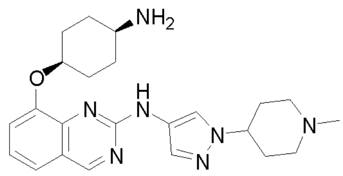

**C2**

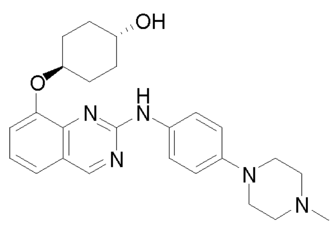

**C3**

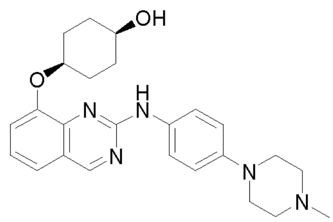

**C4**

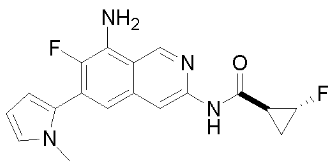

**C5**

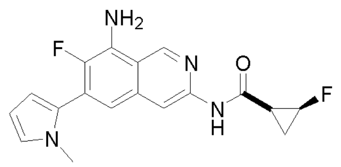

**C6**

.

In a specific embodiment, the virus is selected from Hepatitis B virus, Measles Virus, Sindbis Virus, West Nile Virus, Dengue Virus, Herpes Simplex Virus, Human Cytomegalovirus HCMV, Ebola Virus, hepatitis C virus, Influenza A Virus, SARS-CoV, Zika Virus, HIV, Feline Infectious Peritonitis Virus, Mouse Hepatitis Virus, Canine Coronavirus, Feline Calicivirus, Feline Leukemia Virus, Feline Immunodeficiency Virus, Feline Panleukopenia Virus, Avian Infectious Bronchitis Virus, Transmissible Gastroenteritis Virus, Porcine Epidemic Diarrhea Virus, Porcine Hemagglutinating Encephalomyelitis Virus, Bovine Coronavirus, and the like; preferably, Feline Infectious Peritonitis Virus, Mouse Hepatitis Virus, Herpes Simplex Virus, SARS-CoV, Zika Virus, Feline Infectious Peritonitis Virus, Canine Coronavirus, Feline Calicivirus, Avian Infectious Bronchitis Virus, Porcine Epidemic Diarrhea Virus.

In the second aspect of the present invention, a pharmaceutical composition is provided comprising a compound of the present invention or a pharmaceutically acceptable salt, solvate, stereoisomer, tautomer, prodrug, metabolite or derivative thereof, and one or more other antiviral drugs, as well as optionally a pharmaceutically acceptable excipient.

In the third aspect of the present invention, a compound of the present invention for use as a therapeutic drug for interferon related diseases is provided.

In the fourth aspect of the present invention, a method for treating interferon related diseases is provided, comprising a step of administering a therapeutically effective amount of a compound or pharmaceutical composition of the present invention to a subject in need thereof.

It should be understood that, within the scope of the present invention, each of the above technical features of the present invention and each of the technical features specifically described hereinafter (e.g., in the Examples) may be combined with each other, thereby constituting a new or preferred technical solution, which will not be repeated one by one herein due to the limited contents of the specification.

## Description of drawings

Figure 1. Overexpression of HPK1 significantly inhibited SeV virus-induced IFN-β signalling pathway.

Figure 2. Overexpression of different doses of HPK1 inhibited SeV-induced IFN-β expression in a dose-dependent manner.

Figure 3. Overexpression of different doses of HPK1 inhibited SeV-induced ISRE expression in a dose-dependent manner.
Figure 4. HPK1 small molecule inhibitor restored HPK1-suppressed IFN-β expression.
Figure 5. Compound C2 restored HPK1-inhibited IFN-β expression in a dose-dependent manner.
Figure 6. HPK1 inhibitor significantly reduced viral expression in liver tissue of MHV-infected C57 mouse model.
Figure 7. Oral administration of different doses of C1 compounds (twice a day) exhibited inhibitory effects on the viral load in the liver of MHV-infected C57 mice in a dose-dependent manner.
Figure 8. Oral administration of different doses of C1 compounds (twice a day) exhibited inhibitory effects on alanine aminotransferase (ALT) in the serum of MHV-infected C57 mice in a dose-dependent manner.
Figure 9 shows the synthetic route for compound DD02001H.
Figure 10 shows the synthetic route for compound DD02013H.
Figure 11 shows the synthetic route for compound DD02014H.
Figure 12 shows the synthetic route for compound DD02006H.
Figure 13 shows the synthetic route for compound DD02008H.
Figure 14 shows the synthetic route for compound DD02015H.
Figure 15 shows the synthetic route for compound DD02021H.
Figure 16 shows the synthetic route for compound DD02018H.
Figure 17 shows the synthetic route for compound DD02002H.
Figure 18 shows the synthetic route for compound DD02019H.

## Modes for carrying out the invention

After extensive and intensive research, the inventors have unexpectedly discovered a series of highly active and selective HPK1 small molecule inhibitors. The inventors have further elucidated the intrinsic mechanistic link between HPK1 kinase and viral infections, and have also evaluated the antiviral efficacy of these HPK1 small molecule inhibitors as a monotherapy at the cellular as well as animal model level. Findings suggest that HPK1 small molecule inhibitors as a monotherapy or composition are expected to be used as a novel broad-spectrum antiviral therapy for treating a virus-associated disease, based on which the present invention has been completed.

## Definition on terms

The terms used herein are the same or similar to those conventionally understood by a skilled person. For clarity, some of the terms used herein are defined as follows.

The term "alkyl" as used herein refers to a branched and straight chain saturated aliphatic hydrocarbon group containing, for example, 1 to 12, 1 to 6, or 1 to 4 carbon atoms. Examples of an alkyl include, but are not limited to, methyl, ethyl, propyl (e.g., n-propyl and isopropyl), butyl (e.g., n-butyl, iso-butyl, sec-butyl, and tert-butyl), and amyl (e.g., n-pentyl, iso-pentyl, neo-pentyl), n-hexyl, 2-methylpentyl, 2-ethylbutyl, 3-methylpentyl, and 4-methylpentyl. Numbers appearing in subscript form after the symbol 'C' indicate the number of carbon atoms which may be contained in a particular group. For example, "$C_{1-6}$ alkyl" denotes a straight and branched alkyl with 1-6 carbon atoms.

The term "alkenyl" as used herein refers to a straight or branched hydrocarbon group having at least one double bond. The alkenyl may optionally be substituted with one or more substituents and includes a group having a 'cis' and 'trans' orientation, or 'E' and 'Z' orientation. Z' orientation. Examples include, but are not limited to, vinyl, allyl, and the like.

The term "alkynyl" as used herein generally refers to a branched or straight chain hydrocarbon group having at least one triple bond. The alkynyl group may optionally be substituted. In the present application, the terms "C2-3 alkynyl", "C2-4 alkynyl", "C2-5 alkynyl", "C2-6 alkynyl", "C2-7 alkynyl" and "C2-8 alkynyl" generally refer to an alkynyl containing at least 2 and at most 3, 4, 5, 6, 7 or 8 carbon atoms, respectively. Non-limiting examples of an alkynyl include ethynyl, propynyl, butynyl, pentynyl, hexynyl, heptynyl, octynyl, nonynyl, decynyl and the like.

The term "cycloalkyl" as used herein refers to a group obtained by the loss of a hydrogen atom from a cyclic hydrocarbon molecule. Representative examples of a cycloalkyl include, but are not limited to, cyclopropyl, cyclopentyl, and cyclohexyl. Examples of a monocyclic cycloalkyl are cyclopropyl, cyclobutyl, cyclopentyl, cyclohexyl or cycloheptyl.

The term "heteroatom" as used herein refers to oxygen (O), sulfur (S) or nitrogen (N). The terms "halo" and "halogen" refer to F, Cl, Br or I. Accordingly, the term "haloalkyl" refers to an alkyl substituted with one or more halogens.

As used herein, the term 'cyano' refers to the group -CN, and the term 'amino' refers to the group $-NH_2$.

The term "heterocycloalkyl group" or 'heterocycloalkyl' as used herein refers to a cycloalkyl comprising 1-4 heteroatoms (in the case of a monocyclic ring), 1-6 heteroatoms (in the case of a bicyclic ring), or 1-9 heteroatoms (in the case of a tricyclic ring), wherein the heteroatom is selected from O, S or N. The heterocycloalkyl may optionally be substituted with one or more substituents. In one embodiment, 0, 1, 2, 3 or 4 atoms of each ring of a heterocycloalkyl may be substituted with a substituent. Representative heterocycloalkyl include piperidinyl, piperazinyl, tetrahydropyranyl,

morpholinyl, thiomorpholinyl, 1,3-dioxolyl, THF group, tetrahydrothienyl, thiophenyl, and the like.

The term "aromatic ring" or "aryl" as used herein refers to a hydrocarbon monocyclic, bicyclic, or tricyclic aromatic ring system obtained by the loss of a hydrogen atom. The aryl may optionally be substituted with one or more substituents. In one embodiment, 0, 1, 2, 3, 4, 5 or 6 atoms of each ring of an aryl may be substituted with a substituent. Examples of an aryl include phenyl, naphthyl, anthracenyl, fluorenyl, indenyl, azulenyl, and the like.

The term "aromatic heterocycle" or "heteroaryl" as used herein refers to an "aromatic ring" or "aryl" having at least one heteroatom (O, S or N) in at least one ring, and the heteroatom-containing ring preferably has 1, 2 or 3 heteroatoms independently selected from O, S or N. Each ring in the heteroaryl containing an heteroatom may contain 1 or 2 oxygen or sulphur atoms and/or 1-4 nitrogen atoms, provided that the total number of heteroatoms in each ring is 4 or less and each ring has at least one carbon atom. A heteroaryl may be attached to any available nitrogen or carbon atom of any ring. The heteroaryl ring system may be unsubstituted or may contain one or more substituents. Non-limiting examples of a heteroaryl include pyridyl, furanyl, thienyl, pyrrolyl, oxazolyl, oxadiazolyl, imidazolyl, thiazolyl, isoxazolyl, quinolyl, pyrazolyl, isothiazolyl, pyridazinyl, pyrimidinyl, pyrazinyl, triazinyl, isoquinolyl, indazolyl and the like.

The term "alkoxy" as used herein refers to -O-alkyl. An alkoxy may optionally be substituted with one or more substituents.

As used herein, "(CO)" and "C(O)" denote a carbonyl moiety. Examples of suitable carbonyl moiety include, but are not limited to, ketone and aldehyde moiety.

The term "alkylamino" as used herein refers to an amino substituted with one or two alkyl groups. The term "aminoalkyl" refers to an alkyl substituted with one or more amino groups. The term "hydroxyalkyl" refers to an alkyl substituted with one or more hydroxyl groups. The alkyl moiety optionally has one or more substituents.

As used herein, the term "spiro-cycloalkyl" denotes a polycyclic group in which two carbon rings share one carbon atom. The term "hetero-spiro-cycloalkyl" refers to a polycyclic group in which two monocyclic rings share a carbon atom, wherein the two rings may contain one or more heteroatoms.

The term "bridged ring group" as used herein refers to a ring group in which any two carbon rings share two carbon atoms that are not directly connected to each other, and which can be classified as dicyclic, tricyclic, tetracyclic, and so on, depending on the number of constituent rings. "Heterobridged ring group" refers to a polycyclic heterocycle group, in which two rings share two non-adjacent carbon atoms or heteroatoms.

The term "fused ring group" as used herein refers to a polycyclic organic compound formed by two or more carbon rings sharing a common ring edge. "Hetero-fused ring group" refers to a polycyclic heterocycle group, in which two rings share two adjacent carbon atoms or heteroatoms.

The term "isomer" or "stereoisomer" as used herein refers to compounds with the same chemical composition but different arrangements of atoms or groups in space.

The term "tautomer" or "tautomeric form" refers to structural isomers with different energies that can be transformed into each other through low energy barriers. For example, proton tautomers (also known as interconverting isomers) involve interconversion through proton transfer, such as ketone-enol and imine-enamine isomerization. Valence tautomers involve the mutual conversion of some bonding electrons through recombination.

The term "diastereomer" refers to stereoisomers with two or more asymmetric centers, molecules of which are not mirror images of each other. The term "enantiomer" refers to two stereoisomers of a compound that are mirror images and cannot overlap with each other. The equimolar mixture of two enantiomers is named as a "racemic mixture" or "racemate"

The term "prevention and/or treatment" as used herein not only includes the prevention and/or treatment of a disease, but also typically includes preventing the onset of a disease, slowing down or reversing the progression of a disease, preventing or slowing down the onset of one or more symptoms related to a disease, reducing and/or alleviating one or more symptoms related to a disease, reducing the severity and/or duration of a disease and/or any symptoms related to them, preventing further increase in the severity of diseases and/or any relevant symptoms, preventing, reducing or reversing any physiological damage caused by a disease, and any pharmacological effects that are usually beneficial to a patient being treated. As a viable therapeutic agent, it is not necessary for the compound or composition of the present application to achieve complete cure or eradication of any symptoms or manifestations of a disease. As recognized in relevant fields, a drug used as a therapeutic agent can reduce the severity of a given disease state, however, it is not necessary to eliminate every manifestation of the disease, so that it can be considered as an effective therapeutic agent. Similarly, as a feasible prophylactic agent, it is not necessary for a prophylactic treatment to be completely effective in preventing the onset of symptoms. Simply reducing the impact of a disease in a subject (for example, by reducing the number or severity of symptoms, or by increasing the effectiveness of another treatment, or by producing another beneficial effect), or reducing the likelihood of a disease to occur or worsen, is sufficient. The term "prevention and/or treatment" as used herein includes the prevention and/or treatment of a disease through various means, such as enhancing the immune system of a subject to achieve the prevention and/or treatment of the disease.

As used herein, the term "administering" or "administration" include the route by which said compound is introduced into a subject to achieve intended functions. Examples of routes of administration that may be used include

injectable (subcutaneous, intravenous, parenteral, intraperitoneal, intrathecal), topical, oral, inhalation, rectal and transdermal route.

The term "effective amount" as used herein includes the amount that effectively achieves the desired results within the necessary dosage and period of time. The effective amount of a compound can vary depending on factors such as the subject's disease status, age, and weight, as well as the ability of the compound to elicit the desired response in the subject. A dosage regimen can be adjusted to provide optimal therapeutic response. The "therapeutically effective amount" refers to the amount of the compound of the present application, which can (i) treat or prevent a specific disease, condition or disorder, (ii) attenuate, improve or eliminate one or more symptoms of a specific disease, condition or disorder, or (iii) prevent or delay the onset of one or more symptoms of a specific disease, condition or disorder described in the present application.

The term "subject" or "patient" as used herein refers to an animal, e.g., a mammal, including, but not limited to, a primate (e.g., human), cattle, sheep, goat, horse, dog, cat, rabbit, rat, mice, and the like. In certain embodiments, the subject is a human.

The term "inhibit" as used herein means to reduce the activity of a target enzyme compared with the activity of the enzyme in the absence of an inhibitor. In some embodiments, the term "inhibit" means a reduction in HPK1 activity of at least about 5%, at least about 10%, at least about 20%, at least about 25%, at least about 50%, at least about 60%, at least about 70%, at least about 80%, at least about 90%, or at least about 95%; or a reduction in HPK1 activity of about 5% to about 25%, about 25% to about 50%, about 50% to about 75%, or about 75% to about 100%; or about 95% to about 100% reduction in HPK1 activity, e.g., a reduction in activity of 95%, 96%, 97%, 98%, 99%, or 100%.

**Compounds of the present invention**

In the present invention, a series of compounds having HPK1 inhibitory activities are provided. The compounds of the present invention are capable of enhancing or restoring interferon expression or production in a subject, thereby enhancing a subject's immunity and thus treating and/or preventing a disease. For example, the compounds of the present invention with HPK1 inhibitory activities can enhance IFN-β-mediated antiviral effects and/or enhance IFN-γ-mediated T-cell activation, thereby treating and/or preventing viral infections. In another aspect, the compounds of the present invention with HPK1 inhibitory activities can also be used to enhance a subject's immunity.

In a specific embodiment of the present invention, a compound shown in Formula I is provided,

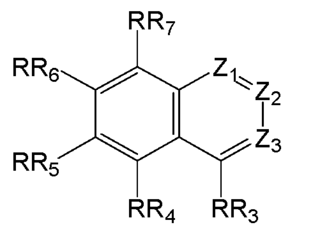

I

wherein substituents in the formula are described as above, respectively.

In a preferred embodiment, the compound of the present invention is a compound shown in Formula 1-1:

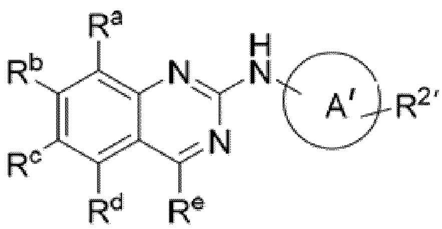

I-1

wherein substituents in the formula are described as above, respectively.

In a further preferred embodiment, the compound shown in Formula I-1 is a compound shown in Formula I-1-1:

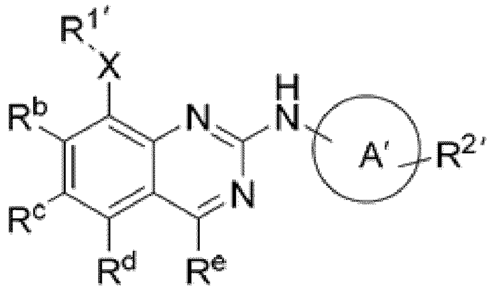

I-1-1

wherein substituents in the formula are described as above, respectively.

In a further preferred embodiment, the compound shown in Formula 1-1-1 is a compound shown in Formula 1-1-1-1 or 1-1-1-2:

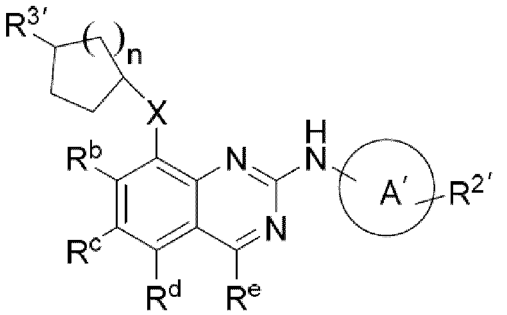

I-1-1-1

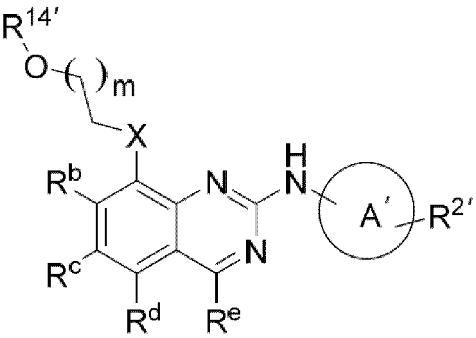

I-1-1-2

wherein substituents in the formula are described as above, respectively.

In other preferred embodiments, the compound of the present invention is a compound shown in Formula I-2:

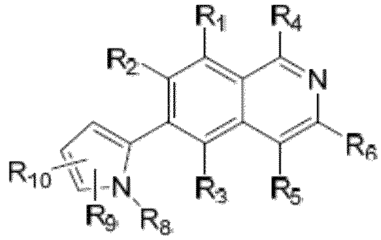

I-2

wherein substituents in the formula are described as above, respectively.

In a further preferred embodiment, the compound of the present invention is a compound shown in Formula I-2-1, I-2-2 or I-2-3:

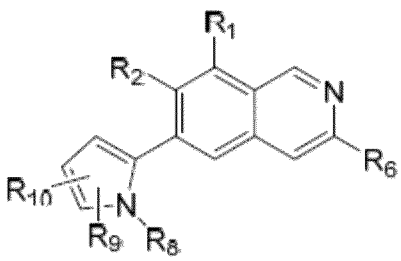

I-2-1

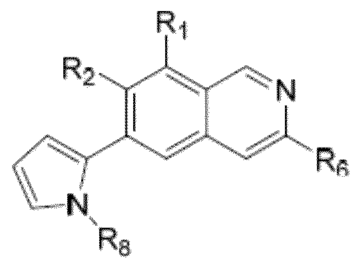

I-2-2

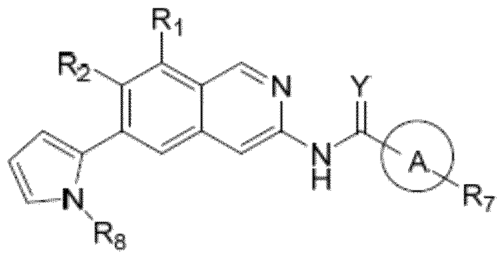

I-2-3

wherein substituents in the formula are described as above, respectively.

The compound of the invention may form a pharmaceutically acceptable salt, such as an organic or inorganic salt formed from the compound of the invention. Exemplary salts include, but are not limited to, sulfates, citrates, acetates, oxalates, chlorides, bromides, iodides, nitrates, bisulfates, phosphates, acid phosphates, isonicotinic acid salts, lactates, salicylates, acid citrates, tartrates, oleates, tannates, pantothenates, hydrotartrates, ascorbates, succinate, maleate, gentiate, fumarate, gluconate, glucuronide, saccharate, formate, benzoate, glutamate, methanesulfonate, ethanesulfonate, benzenesulfonate, toluene sulfonate, bis(hydroxynaphthalene) (i.e., 1,1'-methylene-bis-(2-hydroxy-3-naphthalenecarboxylate), alkali metal (e.g., sodium and potassium) salts, alkaline earth metal (e.g., magnesium) salts, and ammonium salts. A pharmaceutically acceptable salt may involve the inclusion of another molecule which is, for example, an acetate ion, succinate ion or other counter ions. The counter ions may be any organic or inorganic moiety which can stabilise the charge on the parent compound. Furthermore, the pharmaceutically acceptable salt may have more than one charged atom in its structure. In instances where the plurality of charged atoms are part of a pharmaceutically acceptable salt, the salt may have a plurality of counter ions. Therefore, the pharmaceutically acceptable salt may have one or more charged atoms and/or one or more counter ions.

The compounds of the invention may also form solvates (e.g., hydrates). The term "solvate" refers to the physical association of a compound with one or more solvent molecules (organic or inorganic). This physical association includes hydrogen bonding. In some cases, the solvate cab be separated, for example when one or more solvent molecules are incorporated into the lattice of a crystalline solid. Exemplary solvates include hydrates, ethanolates, methanolates, isopropanolates, acetonitrile solvates, and ethyl acetate solvates. Methods of solvation are known in the art.

The compounds of the present invention can be metabolised *in vivo.* Therefore, 'metabolites', i.e., products resulting from the metabolism of a particular compound or salt thereof in the body, should also be included in the protection scope of the present invention.

The compound of the present invention can also be prepared into a "prodrug" or "precursor drug", i.e., a compound which is a precursor of a drug, which undergo chemical transformation through metabolism or a chemical process when given to a subject so as to obtain a compound of formula I or a salt thereof. Prodrugs are well-known in the art (see, e.g., Berge et al. (1977) "Pharmaceutical Salts", J. Pharm. Sci. 66: 1-19).

The compounds of the present invention can also form esters, therefore, esters also fall within the scope of this application. Representative examples of specific esters include, but are not limited to, formate, acetate, propionate, butyrate, acrylate, and ethyl succinate.

The compounds of the present application are intended to include all isotopes of the atoms appearing in the compounds of the present application. For example, isotopes of hydrogen include deuterium (D) and tritium (T). Isotopes of carbon include 13C and 14C. Isotopically labelled compounds of the present application may generally be prepared by conventional techniques known to a skilled person or by methods similar to those described in the present application using suitable isotopically labelled reagents in place of unlabelled reagents employed.

The term "derivative" means a compound formed when atoms or groups of atoms in the molecule of the parent compound are replaced by other atoms or groups of atoms.

**Interferon-related disease**

Interferon-related diseases described herein are diseases that can be treated and/or prevented by administering a compound of the present invention to enhance or restore interferon expression or production in a subject. In a specific embodiment, the interferon is IFN-β and/or IFN-γ; therefore, the compound of the present invention can act as an enhancer of IFN-β and/or IFN-γ signalling pathway, and the "interferon-related disease" as described herein includes all viral infections for which IFN-β/IPN-γ has a therapeutic and/or preventive effects.

In a specific embodiment, the interferon-related disease is viral infection, including but not limited to Hepatitis B virus, Measles Virus, Sindbis Virus, West Nile Virus, Dengue Virus, Herpes Simplex Virus, Human Cytomegalovirus HCMV, Ebola Virus, hepatitis C virus, Influenza A Virus, SARS-CoV, Zika Virus, HIV, Feline Infectious Peritonitis Virus, Mouse Hepatitis Virus, Canine Coronavirus, Feline Calicivirus, Feline Leukemia Virus, Feline Immunodeficiency Virus,

Feline Panleukopenia Virus, Avian Infectious Bronchitis Virus, Transmissible Gastroenteritis Virus, Porcine Epidemic Diarrhea Virus, Porcine Hemagglutinating Encephalomyelitis Virus, Bovine Coronavirus, and the like; preferably, Feline Infectious Peritonitis Virus, Mouse Hepatitis Virus, Herpes Simplex Virus, SARS-CoV, Zika Virus, Feline Infectious Peritonitis Virus, Canine Coronavirus, Feline Calicivirus, Avian Infectious Bronchitis Virus, Porcine Epidemic Diarrhea Virus.

**Pharmaceutical composition**

Since the compounds of the present invention can be used to treat interferon-related diseases, the compounds of the present invention can be prepared into pharmaceutical compositions. In the pharmaceutical composition, the compound of the present invention or a pharmaceutically acceptable salt, solvate, stereoisomer, tautomer, prodrug, metabolite or derivative thereof, and an optional pharmaceutically acceptable excipient is included.

The term "pharmaceutically acceptable" means that the described compounds, substances, compositions and/or dosage forms are suitable for use in contact with human and animal tissues without causing undue toxicity, irritation, allergic reactions, or other problems or complications, under the sound pharmaceutical judgement, and therefore proportional to a reasonable benefit/risk ratio.

The term "excipient" or "carrier" refers to a carrier, excipient or stabiliser that is non-toxic to cells or mammals at the used dosage and concentration. Non-limiting examples include buffers, such as phosphates, citrates, and other organic acids; antioxidants, including ascorbic acid; low molecular weight (less than about 10 residues) polypeptides; proteins, such as serum albumin, gelatin, or immunoglobulins; hydrophilic polymers, such as polyvinylpyrrolidone; amino acids, such as glycine, glutamine, asparagine, arginine, or lysine; and monosaccharides, disaccharides and other carbohydrates (including glucose, mannose, or dextrin); chelating agents such as EDTA; sugar alcohols such as mannitol or sorbitol; salt-forming counterbalancing ions such as sodium; and/or nonionic surfactants such as TWEEN™, polyethylene glycol (PEG), and PLURONICS™. In certain embodiments, the pharmaceutically acceptable carrier is a non-naturally occurring pharmaceutically acceptable carrier.

The compounds of the present invention can treat interferon-related diseases, in particular viral infections. Therefore, in addition to the compounds of the present invention, one or more other antiviral drugs may be included in the pharmaceutical compositions of the present invention, thereby enhancing the efficacy of said pharmaceutical compositions.

**Advantages of the present invention:**

1. the present invention provides novel HPK1 small molecule inhibitors with high activity and selectivity;
2. the present invention elucidates, for the first time, the intrinsic mechanistic link between HPK1 kinase and viral infection;
3. the HPK1 small molecule inhibitors of the present invention can exhibit antiviral effects at the cellular as well as animal model level as a monotherapy, thereby laying a novel material basis for the development of drugs for interferon-related diseases, especially viral infections;
4. The HPK1 small molecule inhibitors of the present invention can exhibit antiviral effects at the cellular as well as animal model level as an adjuvant/co-adjuvant agent for enhancing immunity, thereby laying a novel material basis for the development of drugs for interferon-related diseases, in particular viral infections.

The technical solutions of the present invention will be further described below in connection with specific embodiments, however, following embodiments do not constitute a limitation of the present invention, and all the various embodiments based on the principles and technical means of the present invention shall fall within the scope of the present invention. Experimental methods for which specific conditions are not indicated in the following embodiments are generally in accordance with conventional conditions, or in accordance with conditions recommended by the manufacturer. Percentages and portions are calculated by weight unless otherwise indicated.

## Examples

### Example 1. Synthesis of the compounds of the present invention

### 1. Synthesis and assay on HPK1 inhibitory activity of compounds of formula I-1

1.1 Synthesis of Compound DD02001H: 8-(((1s,4s)-4-aminocyclohexyl)oxo)-N-(1-(1-methylpiperidin-4-yl)-1H-pyrazol-4-yl)quinazolin-2-amine

The synthetic route of compound DD02001H is shown in Figure 9.

1.1.1 Synthesis of Compound 2

At 0 °C, $BH_3$/THF (1.00 M, 329 mL, 2.20 eq) was added dropwise to a solution of compound 1 (25.0 g, 150 mmol, 1.00 eq) in THF (300 mL) under $N_2$. After addition, the mixture was stirred at 0 °C for 30 min, then heated to 50 °C and stirred for 12 h. TLC (petroleum ether: ethyl acetate = 1: 1, raw material Rf = 0.1, product Rf = 0.3) showed the formation of a new spot and complete disappearance of the raw material spots, so that the reaction was completed. Afterwards, the mixture was cooled to 0 °C, and MeOH (400 mL) was added dropwise to the mixture until no bubbles formed. The mixture was then cooled to 0 °C, MeOH (400 mL) was added dropwise until no bubbles were formed. Then 40.0 mL of $H_2O$ was added and extracted with ethyl acetate (300 mL*2). The organic layer was washed with brine (100 mL*2), dried over anhydrous $Na_2SO_4$ and filtered, and then concentrated under reduced pressure to obtain Compound 2 (45.0 g, 294 mmol, 98.2% yield), which was a yellowish oily substance, the structure of which was confirmed by $^1$H NMR.

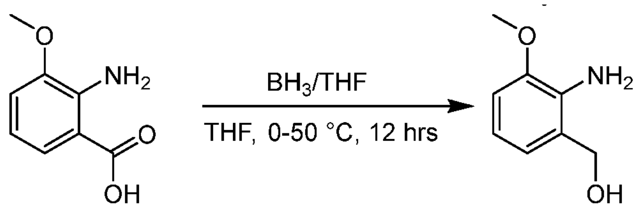


1 2

**$^1$H NMR**(400 MHz, $CDCl_3$) $\delta$ 6.65-6.85 (m, 3H), 4.63 (s, 2H), 3.86 (s, 3H).

1.1.2 Synthesis of Compound 3

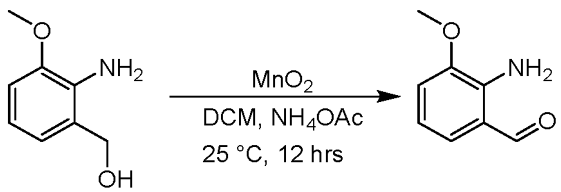


2 3

To a solution of compound 2 (45.0 g, 294 mmol, 1.00 eq.) in dichloromethane (DCM) (500 mL) was added $MnO_2$ (128 g, 1.47 mol, 5.00 eq.), and the resulting mixture was stirred for 12 hr at 25 °C. Thin layer chromatography (petroleum ether: ethyl acetate = 1: 1, raw material Rf = 0.45, product Rf = 0.8) showed the formation of a new spot and disappearance of the raw material, so that the reaction was completed. The mixture was filtered through diatomaceous earth, and the filtrate was concentrated under reduced pressure. Compound 3 (25.0 g, 166 mmol, 56.3% yield) was isolated and purified by column chromatography ($SiO_2$, petroleum ether/ethyl acetate = 30/1 to 5/1), the structure of which was confirmed by $^1$H NMR.

**$^1$H NMR**(400 MHz, DMSO-$d_6$)$\delta$ 9.85 (s, 1H), 7.18 (dd, $J$=8.0, 1.2 Hz, 1H), 7.02 (dd, $J$ = 8.0, 0.8 Hz, 1H), 6.80 (br s, 2H), 6.64 (t, $J$ = 8.0 Hz, 1H), 3.77-3.87 (m, 3H).

1.1.3 Synthesis of Compound 4

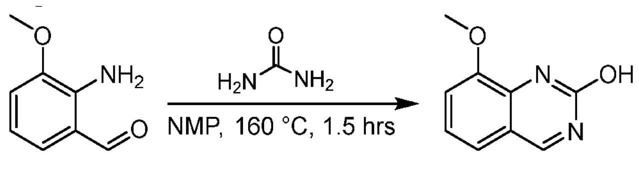


3

4

A mixture of compound 3 (23.0 g, 152 mmol, 1.00 eq), urea (101 g, 1.67 mol, 89.7 mL, 11.0 eq) and $NH_4OAc$ (586 mg, 7.61 mmol, 0.05 eq) was stirred at 160 °C for 0.5 h, and the mixture began to precipitate from the hot solution. NMP (100 mL) was added to dissolve the solid precipitate, afterwards the reaction was stirred at 160 °C for 1 h. LC-MS detection showed the target product 4 (RT = 0.247 min) and the disappearance of the raw material, so that the reaction was completed. And then the mixture was cooled to 25 °C and poured into 100 mL of $H_2O$, stirred at 25 °C for 10 min and filtered under reduced pressure. The crude product was shaken and dispersed with petroleum ether (60.0 mL) for 5 min at 25°C and filtered under reduced pressure to obtain Compound 4 (20.0 g, 114 mmol, 74.6% yield) as grey solids, the structure of which was confirmed by $^1$H NMR.

**LCMS:** product RT = 0.247 min, m/z = 177.2 $(M+H)^+$
**$^1$H NMR**(400 MHz, DMSO)$\delta$ 8.36 (s, 1H), 6.90 (br d, *J*= 4.0 Hz, 1H), 6.87 (d, *J*= 8.0 Hz, 1H), 6.79 (dd, *J* = 8.0, 4.0 Hz, 1H), 5.98 (dd, *J*= 8.0, 4.0 Hz, 1H), 3.79 (s, 3H).

1.1.4 Synthesis of Compound 5

Compound 4 (19.0 g, 108 mmol, 1.00 eq) was added into POCl3 (248 g, 1.61 mol, 150 mL, 15.0 eq) at 0 °C and the resulting mixture was stirred at 25 °C for 0.5 h, then heated to 140 °C and stirred for 2 h. LC-MS detection showed the target product (RT = 0.655 min). At the end of the reaction, the mixture was slowly poured into ice water (200 mL) at 0-10 °C and stirred, and then extracted with ethyl acetate (300 mL*4). The organic layer was washed with brine (100 mL*2), dried over anhydrous $Na_2SO_4$, filtered and concentrated under reduced pressure. The crude product was shaken and dispersed with petroleum ether (60.0 mL) at 25 °C for 5 min and filtered to obtain Compound 5 (7.60 g, 39.1 mmol, 36.2% yield) as yellow solids, the structure of which was confirmed by $^1$H NMR.

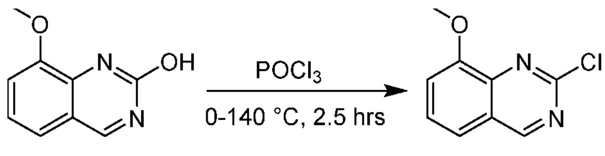


4

5

**LCMS:** product RT = 0.655 min, m/z = 195.0 $(M+H)^+$
**$^1$H NMR**(400 MHz, DMSO) $\delta$ 9.56 (s, 1H), 7.69 - 7.79 (m, 2H), 7.54 (br d, *J*= 4.0 Hz, 1H), 3.99 (s, 3H).

1.1.5 Synthesis of Compound 6

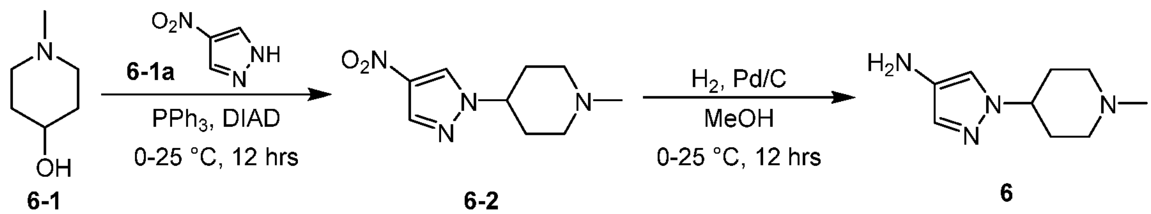


6-1

6-2

6

At 25 °C, to a solution of Compound 6-1 (10.0 g, 86.8 mmol, 10.1 mL, 1.00 eq), Compound 6-1a (11.7 g, 104 mmol, 1.20 eq) and triphenylphosphine alkane (34.2 g, 130 mmol, 1.50 eq) in THF (300 mL) was added DIAD (26.3 g, 130 mmol,

25.3 mL, 1.50 eq) and the resulting mixture was stirred under $N_2$ for 12 h. LCMS showed the complete reaction of the raw material and formation of the product. After the reaction was completed, the reaction mixture was adjusted to pH = 4 with 1 M HCl and extracted with ethyl acetate (300 mL), and the aqueous phase was adjusted to pH = 8 with $NaHCO_3$ (saturated) and extracted again with ethyl acetate (100 mL*2). The organic layer was washed with saturated brine (100 mL), the organic layer was dried over anhydrous sodium sulfate, filtered and concentrated under reduced pressure to give Compound 6-2 (8.00 g, 38.1 mmol, 43.8% yield) as a yellow oily substance, the structure of which was confirmed by $^1$H NMR.

**LCMS** product RT = 0.150 min, m/z = 211.1 $(M+H)^+$
**$^1$H NMR**(400 MHz, $CDCl_3$)$\delta$ 8.15 (s, 1H), 8.04 (s, 1H), 4.08 - 4.14 (m, 1H), 2.95 - 2.98 (m, 2H), 2.31 (s, 3H), 2.09 - 2.15 (m, 6H).

Compound 6-2 (8.00 g, 38.1 mmol, 1.00 eq.), Pd/C (2.00 g, 10% purity) were stirred in methanol (50.0 mL) and the mixture was stirred under $H_2$ (15 psi) at 25 °C for 12 h. LCMS showed that Compound 6-2 was completely consumed, and at the end of the reaction, the mixture was filtered and concentrated under reduced pressure to give yellow oily Compound 6 (5.00 g, 27.7 mmol, 72.9% yield), the structure of which was confirmed by $^1$H NMR.

**LCMS:** product RT = 0.10 min, m/z = 181.1 $(M+H)^+$
**$^1$H NMR**(400 MHz, $CDCl_3$)$\delta$ 7.09 (s, 1H), 7.01 (s, 1H), 3.92 - 3.98 (m, 1H), 2.88 - 2.91 (m, 4H), 2.26 (s, 3H), 1.85 - 2.26 (m, 6H).

1.1.6 Synthesis of Compound 7

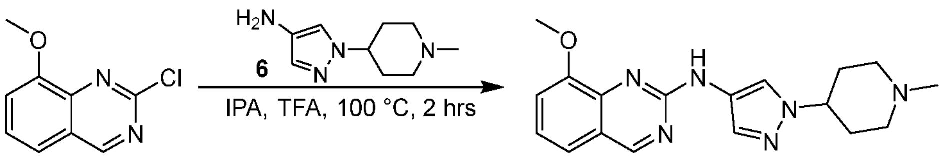


5 7

TFA (23.4 mg, 205 umol, 15.2 uL, 0.10 eq) was added to a solution of Compound 5 (400 mg, 2.06 mmol, 1.00 eq) and Compound 6 (444 mg, 2.47 mmol, 1.20 eq) in IPA (10.0 mL), and the resulting mixture was stirred for 2 h at 100 °C. LCMS detection showed the target product (RT = 0.653 min). The mixture was diluted with ethyl acetate (100 mL) and washed with saturated $NaHCO_3$ solution (50.0 mL), and then the organic phase was washed with brine (50.0 mL), dried over anhydrous sodium sulfate, filtered and concentrated to give a solid, which was shaken and dispersed with petroleum ether (25.0 mL) to give Compound 7 (570 mg, 1.68 mmol, 81.9% yield) as yellow solids, the structure of which was confirmed by LCMS and $^1$H NMR.

**LCMS:** product RT = 0.673 min, m/z = 339.3 $(M+H)^+$
**$^1$H NMR**(400 MHz, $CDCl_3$)$\delta$ 9.05 (s, 1H), 8.22 (s, 1H), 7.60 (s, 1H), 7.31 - 7.35 (m, 2H), 7.21 - 7.25 (m, 1H), 7.10 - 7.12 (m, 1H), 4.12 - 4.14 (m, 1H), 4.05 (s, 3H), 2.98 - 3.01 (m, 2H), 2.34 (s, 3H), 2.14 - 2.23 (m, 6H).

1.1.7 Synthesis of Compound 8

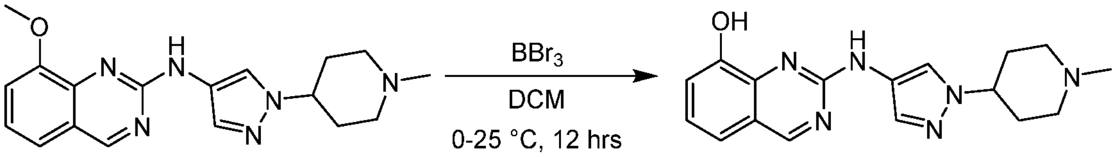


7 8

$BBr_3$ (1.05 g, 4.21 mmol, 405.7 uL, 2.50 eq) was added dropwise to a solution of Compound 7 (570 mg, 1.68 mmol, 1.00 eq) in DCM (15.0 mL). The mixture was stirred at 25 °C for 12 h. LCMS (EW20001-101-P1A1) detection

showed the target product (RT = 0.622 min). At the end of the reaction, the mixture was diluted with DCM (100 mL) and washed with saturated $NaHCO_3$ solution (50.0 mL), and the organic phase was washed with brine (50.0 mL), dried over anhydrous sodium sulfate, filtered and concentrated to give Compound 8 (500 mg, crude) as yellow solids, which was used directly in the next step.

**LCMS:** product RT= 0.847 min, m/z = 325.2 $(M+H)^+$

1.1.8 Synthesis of Compound 10

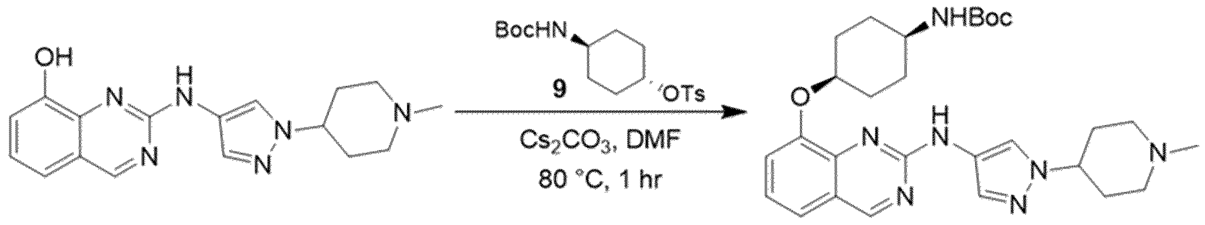


8 10

$Cs_2CO_3$ (502 mg, 1.54 mmol, 2.50 eq) was added to a solution of Compound 8 (200 mg, 616 umol, 1.00 eq) and Compound 9 (273 mg, 740 umol, 1.20 eq) in DMF (3.00 mL) and the resulting mixture was stirred for 1 hr at 80 °C. The LCMS detection showed the target product (RT = 0.799 min). At the end of the reaction, the mixture was quenched by adding water (50.0 mL) and extracted with ethyl acetate (50.0 mL*2), and the organic phase was washed with brine (50.0 mL), dried over anhydrous sodium sulfate, filtered and concentrated to give Compound 10 (320 mg, 613 umol, 99.5% yield) as a yellow oily substance, which was used without further purification in the next step.

**LCMS** product RT = 0.799 min, m/z = 522.3 $(M+H)^+$.

1.1.9 Synthesis of Compound DD02001H

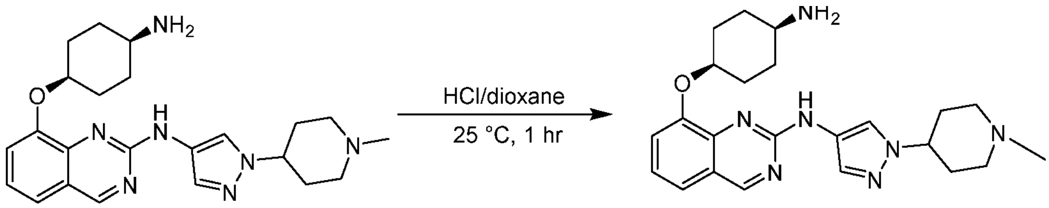


10 **DD02001H**

A mixture of Compound 10 (320 mg, 613 umol, 1.00 eq) with HCl/dioxane (4 M, 10.0 mL, 65.2 eq) was stirred at 25 °C for 1 h. LCMS detection showed the target product (RT = 0.651 min). The mixture was concentrated to obtain a residue, which was purified by preparative HPLC. The mixture was adjusted to pH = 8 with saturated $NaHCO_3$ solution and extracted with DCM (50.0 mL*2), and the organic phase was washed with brine (50.0 mL), dried over anhydrous sodium sulfate, filtered and concentrated to give Compound DD02001H as yellow solids (27.2 mg, 91.7 umol, 14.9% yield, 96.7% purity), the structure of which was confirmed by LCMS, HPLC (EW20001-106-P1A2) and $^1$H-NMR.

**LCMS** product RT = 0.644 min, m/z = 422.3 $(M+H)^+$

**$^1$H NMR**(400 MHz, $CDCl_3$)$\delta$ 8.94 (s, 1H), 8.14 (s, 1H), 7.68 (s, 1H), 7.23 - 7.25 (m, 1H), 7.12 - 7.13 (m, 2H), 7.06 (s, 1H), 4.67 - 4.68 (m, 1H), 4.05 - 4.09 (m, 1H), 2.89 - 2.92 (m, 2H), 2.78 - 2.80 (m, 1H), 2.26 (s, 3H), 2.05 - 2.15 (m, 8H), 1.62 - 1.74 (m, 8H).

1.2 Synthesis of Compound DD02013H: 4-((2-((4-((2-(dimethylamino)ethyl)(methyl)amino)phenyl)amino)quinazolin-8-yl)oxo)cyclohexan-1-ol

The synthetic route of compound DD02013H is shown in Figure 10.

1.2.1 Synthesis of Compound 11

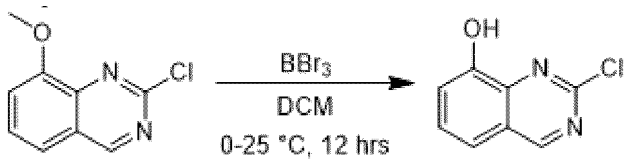


5 11

A solution of $BBr_3$ (1.42 g, 5.65 mmol, 545 uL, 2.20 eq) in DCM (5.00 mL)was added dropwise to a solution of Compound 5 (500 mg, 2.57 mmol, 1.00 eq) in DCM (10.0 mL) at 0 °C. After the addition, the mixture was stirred at 25 °C for 12 h. LCMS detection showed the target product molecular weight (RT = 0.585 min). At the end of the reaction, the mixture was quenched with water (50.0 mL) and extracted with DCM (50.0 mL). The organic phase was washed with brine (50.0 mL), dried over anhydrous sodium sulfate, filtered and concentrated to give a residue which was purified by column chromatography ($SiO_2$, petroleum ether/ethyl acetate = 20/1 to 10/1, TLC (petroleum ether: ethyl acetate = 3:1, Rf = 0.3) to give Compound 11 (400 mg, 2.21 mmol. 86.2% yield) as yellow solids, the structure of which was confirmed by LCMS and $^1H$ NMR.

**LCMS:** product RT = 0.575 min, m/z = 181.0 $(M+H)^+$
**$^1$H NMR**(400 MHz, $CDCl_3$)$\delta$ 9.30 (s, 1H), 7.59-7.64 (m, 1H), 7.49-7.52 (m, 1H), 7.42-7.45 (m, 2H).

1.2.2 Synthesis of Compound 12

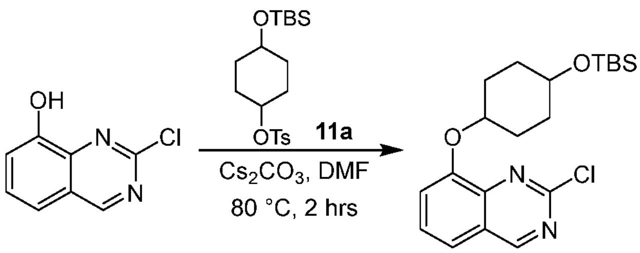


11 12

$Cs_2CO_3$ (721.6 mg, 2.21 mmol, 2.00 eq) was added to a solution of Compound 11 (200 mg, 1.11 mmol, 1.00 eq) and Compound 11a (639 mg, 1.66 mmol, 1.50 eq) in DMF (5.00 mL) and the resulting mixture was stirred for 2 hr at 80°C. The LCMS detection showed the target product (RT = 1.229 min). After the reaction was completed, the reaction was quenched by the addition of water (50.0 mL), extracted with ethyl acetate (50.0 mL*2), and the organic phase was washed with brine (50.0 mL), dried over anhydrous sodium sulfate, filtered and concentrated to obtain the residue, which was purified by column chromatography ($SiO_2$, petroleum ether/ethyl acetate = 20/1 to 10/1, TLC (petroleum ether/ethyl acetate = 5: 1, Rf = 0.4)) to give Compound 12 (100 mg, 254 umol, 22.9% yield) as yellow solids, the structure of which was confirmed by $^1H$ NMR.

**LCMS:** product RT = 1.229 mins, m/z = 393.2 $(M+H)^+$
**$^1$H NMR**(400 MHz, $CDCl_3$)$\delta$ 9.25 (s, 1H), 7.55-7.60 (m, 1H), 7.49-7.51 (m, 1H), 7.34-7.37 (m, 1H), 4.53-4.57 (m, 1H), 3.90-3.92 (m, 1H), 2.15-2.20 (m, 2H), 1.86 -1.89 (m, 4H), 1.60-1.64 (m, 2H), 0.93 (s, 9H), 0.08 (s, 6H).

1.2.3 Synthesis of Compound 12a

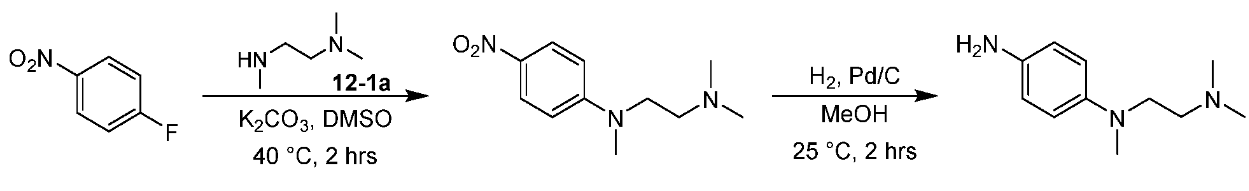


12-1 12-2 12a

$K_2CO_3$ (3.92 g, 28.4 mmol, 2.00 eq) was added to a solution of Compound 12-1 (2.00 g, 14.2 mmol, 1.50 mL, 1.00 eq) and Compound 12-1a (1.59 g, 15.6 mmol, 2.03 mL, 1.10 eq) in dimethylsulfoxide (10.0 mL) and the resulting mixture was was stirred at 40 °C for 2 h. TLC (petroleum ether: ethyl acetate = 5:1) showed the retention of Compound 12-1 (Rf = 0.6) and the formation of a new product. At the end of the reaction, the reaction mixture was diluted with $H_2O$ (100 mL) and extracted with ethyl acetate (100 mL*2), and the combined organic layers were washed with saturated brine (200 mL), dried over anhydrous sodium sulphate, filtered and concentrated under reduced pressure to give Compound 12-2 (3.20 g, crude oil), the structure of which was confirmed by $^1$H NMR and used in the next step without further purification.

**$^1$H NMR**(400 MHz, $CDCl_3$)$\delta$ 8.09-8.12 (m, 2H), 6.59-6.62 (m, 2H), 3.55 (t, *J* = 7.2 Hz, 2H), 3.10 (s, 1H), 2.50 (t, *J* = 7.2 Hz, 2H), 2.30 (s, 6H).

To a solution of Compound 12-2 (3.20 g, 14.3 mmol, 1.00 eq.) in MeOH (30.0 mL) was added Pd/C (1.00 g, 10% purity, 1.00 eq.). The suspension was degassed under vacuum and purged several times with hydrogen, afterwards the resulting solution was stirred under an atmosphere of $H_2$ (15 psi) at 25°C for 2 h. TLC (dichloromethane: methanol = 10:1) detection showed the disappearance of the raw material of Compound 12-2 (Rf = 0.5) and the formation of a new compound. After the reaction was completed, the mixture was filtered and concentrated to give Compound 12a (2.30 g, 11.9 mmol, 83.0% yield) as a reddish-brown oil, the structure of which was confirmed by $^1$H NMR without further purification.

**$^1$H NMR**(400 MHz, DMSO)$\delta$ 6.47 - 6.54 (m, 4H), 4.34 (br. s, 2H), 3.19 (t, *J* = 7.2 Hz, 2H), 2.72 (s, 1H), 2.30 (t, *J* = 7.2 Hz, 2H), 2.14 (s, 6H).

1.2.4 Synthesis of Compound 13

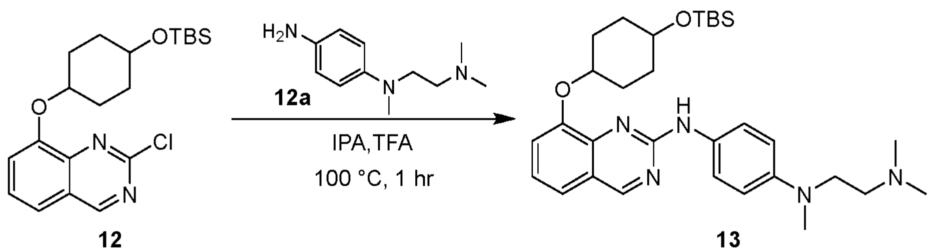


12 13

TFA (29.0 mg, 255 umol, 18.8 uL, 1.00 eq) was added to a solution of Compound 12 (100 mg, 254 umol, 1.00 eq) and Compound 12a (59.0 mg, 305 umol, 1.20 eq) in IPA (3.00 mL). The resulting mixture was stirred at 100 °C for 1 h. LCMS detection showed the target product (RT = 0.913 min). After the reaction was completed, the mixture was extracted with ethyl acetate (100 mL), and the organic phase was washed successively with saturated $NaHCO_3$ solution (50.0 mL) and brine (50.0 mL), dried over anhydrous sodium sulfate, filtered and concentrated to give Compound 13 (130 mg, crude) as yellow solids, which was not purified and used directly in the next step.

1.2.5 Synthesis of Compound DD02013H

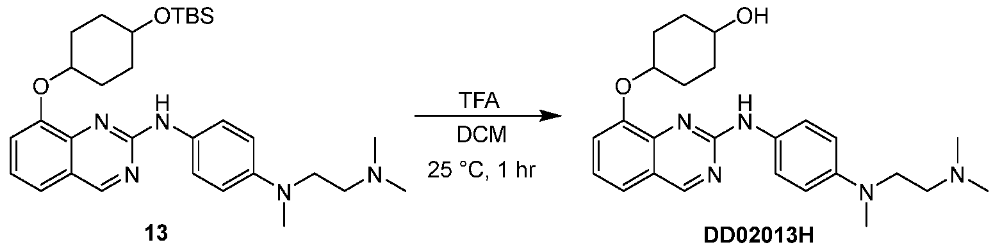


13 DD02013H

To a solution of Compound 13 (130 mg, 236 umol, 1.00 eq.) in DCM (5.00 mL) was added TFA (0.50 mL) and the resulting mixture was stirred at 25 °C for 1 h. LCMS detection showed the target product (RT = 0.701 min). After the reaction was completed, the mixture was concentrated to obtain a residue solid, which was purified by preparative high performance liquid chromatography (HPLC).The resulting solid was adjusted to pH = 8 with saturated $NaHCO_3$ solution, extracted with DCM (50.0 mL*2), washed with brine (50.0 mL), dried over anhydrous sodium sulphate, filtered and concentrated to give a yellow solid, the target compound DD02013H (47.6 mg. 130.7 umol, 55.3% yield, 94.9% purity), the structure of which was confirmed by LCMS, HPLC and $^1$H-NMR.

**LCMS** product RT = 0.997 min, m/z = 436.3 $(M+H)^+$
**HPLC** product RT = 2.951 mins, purity: 95.6%
**$^1$H NMR**(400 MHz, $CDCl_3$)$\delta$ 9.00 (s, 1H), 7.83 - 7.85 (m, 2H), 7.29 - 7.31 (m, 1H), 7.17 - 7.19 (m, 3H), 6.85 - 6.87 (m, 2H), 4.78 - 4.79 (m, 1H), 3.76 - 3.81 (m, 1H), 3.49 (t, *J*= 7.2 Hz, 2H), 2.95 (s, 3H), 2.54 (t, *J* = 7.2 Hz, 2H), 2.33 (s, 6H), 2.16 - 2.21 (m, 2H), 2.03 - 2.06 (m, 2H), 1.80 - 1.83 (m, 2H), 1.69 - 1.73 (m, 2H).

1.3 Synthesis of Compound DD02014H: 4-((2-((1-(1-methylpiperidin-4-yl)-1H-pyrazol-4-yl)amino)quinazolin-8-yl)oxo) cyclohexan-1-ol

The synthetic route of compound DD02014H is shown in Figure 11.

$Cs_2CO_3$ (1.00 g, 3.08 mmol, 2.50 eq) was added to a solution of Compound 8 (400 mg, 1.23 mmol, 1.00 eq) and Compound 11a (569.1 mg, 1.48 mmol, 1.20 eq) in DMF (10.0 mL) and the resulting mixture was stirred for 1 hr at 80 °C. LCMS detection showed the target product (RT = 0.958 min). After the reaction was completed, ethyl acetate (100 mL) was added and the organic phase was washed successively with saturated $NaHCO_3$ solution (50.0 mL) and brine (50.0 mL), dried over anhydrous sodium sulfate, filtered and concentrated to obtain the residue, which was shaken and dispersed with petroleum ether: ethyl acetate (3:1, 20.0 mL), filtered and dried to obtain Compound 14 ( 300 mg, 559 umol, 45.3% yield) as yellow solids, the structure of which was characterised by LCMS.

**LCMS** product RT = 0.912 min, m/z = 537.3 $(M+H)^+$.

A mixture of Compound 14 (300 mg, 559 umol, 1.00 eq) and HCl/MeOH (4 M, 10.0 mL, 71.6 eq) was stirred at 25 °C for 1 h. LCMS (EW20001-117-P1A1) detection showed the disappearance of the product and the appearance of the target product (RT = 0.729 min). After the reaction was completed, the mixture was concentrated and the resulting solids were purified by preparative HPLC. Afterwars, the mixture was adjusted to pH = 8 with a saturated $NaHCO_3$ solution, extracted with DCM (50.0 mL*2). The organic phase was washed with brine (50.0 mL), dried over anhydrous sodium sulfate, filtered and concentrated to give Compound DD02014H (35.5 mg, 138 umol. 24.6% yield, 96.9% purity) as yellow solids, the structure of which was confirmed by LCMS, HPLC and $^1$H NMR.

**LCMS** product RT = 0.730 min, m/z = 423.2 $(M+H)^+$
**HPLC** product RT = 1.192 mins, purity: 98.0%
**$^1$H NMR**(400 MHz, $CDCl_3$)$\delta$ 9.01 (s, 1H), 8.61 (s, 1H), 7.49 (s, 1H), 7.28 - 7.30 (m, 1H), 7.17 - 7.22 (m, 3H), 4.66 - 4.68 (m, 1H), 4.23 - 4.30 (m, 1H), 3.85 - 3.89 (m, 1H), 2.99 - 3.03 (m, 2H), 2.33 (s, 3H), 2.25 - 2.29 (m, 4H), 2.15 - 2.17 (m, 4H), 1.95 - 2.06 (m, 2H), 1.84 - 1.88 (m, 4H).

1.4 Synthesis of Compound DD02006H: 4-((2-((4-(4-(4-methylpiperazin-1-yl)phenyl)amino)quinazolin-8-yl)oxo)cyclohexan-1-ol

The synthetic route of compound DD02006H is shown in Figure 12.

1.4.1 Synthesis of Compound 16

TFA (586 mg, 5.14 mmol, 380 uL, 1.00 eq) was added to a solution of Compound 5 (1.00 g, 5.14 mmol, 1.00 eq) and Compound 16a (1.08 g, 5.65 mmol, 1.10 eq) in IPA (20.0 mL) at 25 °C, mixed well, and the reaction mixture was heated

to 100 °C and stirred for 3 hours.

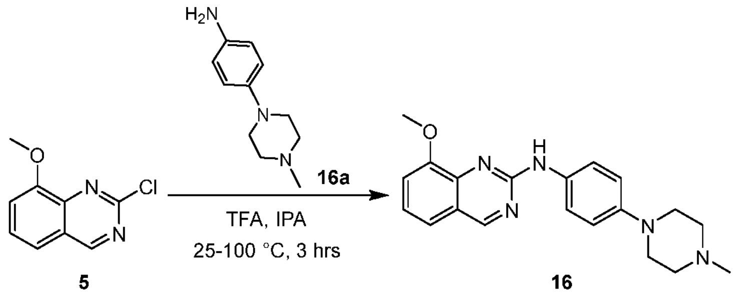


5 16

LCMS detection showed the target product (RT = 0.691 min). After the reaction was completed, the mixture was poured into 30.0 mL of $H_2O$, then extracted with ethyl acetate (60.0 mL*4), and the organic layer was washed with brine (60.0 mL*2), dried over anhydrous $Na_2SO_4$, filtered and concentrated under reduced pressure to give Compound 16 (1.50 g, crude) as yellow solids. The product was used directly in the next step without purification.

**LCMS:** product RT = 0.691 min, m/z = 350.2 $(M+H)^+$

1.4.2 Synthesis of Compound 17

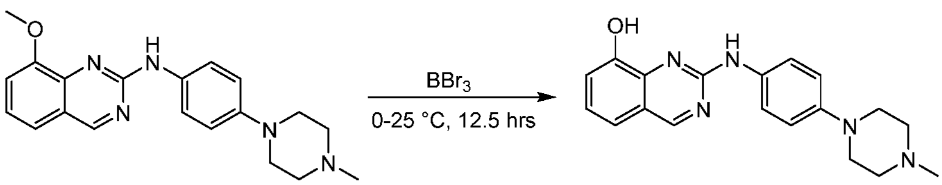


16 17

$BBr_3$ (2.80 g, 11.2 mmol, 1.08 mL, 3.00 eq) dissolved in DCM (10.0 mL) was added dropwise to a solution of Compound 16 (1.30 g, 3.72 mmol, 1.00 eq) in DCM (20.0 mL) at 0 °C. After the dropwise addition was completed, the mixture was stirred at 0 °C for 30 min, and subsequently heated to 25 °C and stirred for 12 h. LCMS detection showed the target product (RT = 0.677 min). After the reaction was completed, the mixture was poured into ice water (30.0 mL), extracted with ethyl acetate (60.0 mL*3), and the organic layer (30.0 mL*2) was washed with brine, dried over anhydrous $Na_2SO_4$, filtered and concentrated under reduced pressure to give Compound 17 (1.20 g, crude) as grey solids. The product was not purified and used directly in the next step.

**LCMS:** product RT = 0.677 min, m/z = 336.2 $(M+H)^+$

1.4.3 Synthesis of Compound 18

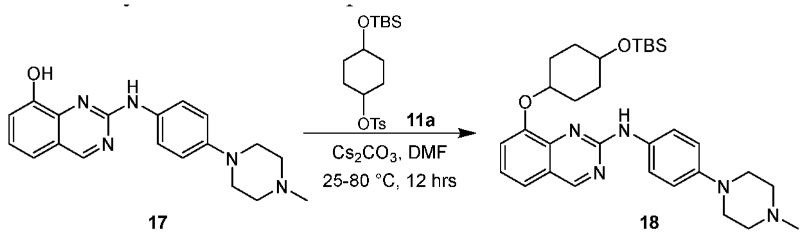


17 18

Compound 17 (150 mg, 447 umol, 1.00 eq), Compound 11a (276 mg, 894 umol, 2.00 eq), and $Cs_2CO_3$ (364 mg, 1.12 mmol, 2.50 eq) were dissolved in DMF (10.0 mL), and the mixture was subsequently heated to 80 °C for a 12 h. LCMS detection showed the target product (RT = 0.934 min). After the reaction was completed, the mixture was poured into 30.0 mL of $H_2O$ and then extracted with ethyl acetate (30.0 mL*4), and the organic layer was washed with brine (30.0 mL*2), dried over anhydrous $Na_2SO_4$, filtered and concentrated under reduced pressure to give Compound 18 (200 mg, crude) as yellow solids. The product was used directly in the next step without purification.

**LCMS** product: RT = 0.934 min, m/z = 548.4 $(M+H)^+$

1.4.4 Synthesis of Compound DD02006H

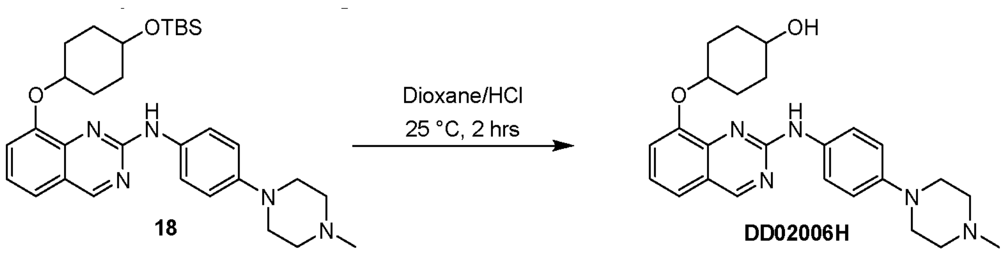


18

DD02006H

HCl/1,4-dioxane (4 M, 2.00 mL, 21.9 eq) was added dropwise to a solution of Compound 18 (200 mg, 365 umol, 1.00 eq) in DCM (6.00 mL) at 25 °C, and the resulting mixture was stirred for 2 h. LCMS detection showed the target product (RT = 0.746 min). Thin layer chromatography (DCM: MeOH = 8: 1, raw material Rf = 0.5, product Rf = 0.2) showed the formation of a new spot and no residual material. The mixture was poured into 30.0 mL of $H_2O$ and the pH was adjusted with saturated $NaHCO_3$ to about 8. The mixture was then extracted with ethyl acetate (30.0 mL*4), and the organic layer was washed with brine (30.0 mL*2), dried over anhydrous Na2SO4, filtered and concentrated under reduced pressure. The crude product was subjected to pre-HPLC and concentrated under reduced pressure to give target product (68.6 mg, 157 umol, 43.1% yield, 99.4% purity) as yellow solids, the structure of which was confirmed by $^1$H NMR, LCMS and HPLC.

> **LCMS** product: RT = 0.734 min, m/z = 434.3 $(M+H)^+$,
> **HPLC** product RT = 1.373 mins, 99.4% purity;
> **$^1$H NMR**(400 MHz, DMSO)$\delta$ 9.57 - 9.71 (m, 1H), 9.12 - 9.27 (m, 1H), 7.90 - 8.06 (m, 2H), 7.40 - 7.47 (m, 1H), 7.28 - 7.34 (m, 1H), 7.18 - 7.26 (m, 1H), 6.86 - 6.99 (m, 2H), 4.75 (br s, 1H), 4.52 - 4.64 (m, 1H), 3.63 (br s, 1H), 3.07 (br d, *J*= 4.0 Hz, 4H), 2.24 (s, 3H), 1.91 - 2.05 (m, 2H), 1.74 - 1.87 (m, 2H), 1.56 - 1.72 (m, 4H).

1.5 Synthesis of Compound DD02008H: 8-(2-methoxyethoxy)-N-(4-(4-methylpiperazin-1 -yl)phenyl)quinazolin-2-amine

The synthetic route of compound DD02008H is shown in Figure 13.

Compound 17 (150 mg, 447 umol, 1.00 eq), Compound 17a (68.4 mg, 492 umol, 46.2 uL, 1.10 eq), and $Cs_2CO_3$ (437 mg, 1.34 mmol, 3.00 eq) were dissolved in DMF (10.0 mL), and then the mixture was heated to 80 °C and stirred for 12 h. LCMS detection showed the target product (RT = 0.703 min). Thin layer chromatography (DCM: MeOH = 10: 1, raw material Rf = 0.1, product Rf = 0.3) showed the formation of a new spot and no raw material. After the reaction was completed, the mixture was poured into 30.0 mL $H_2O$, then extracted with ethyl acetate (30.0 mL*4), and the organic layer was washed with brine (30.0 mL*2), dried over anhydrous $Na_2SO_4$, filtered and concentrated under reduced pressure. The mixture was purified by column chromatography ($SiO_2$, DCM/MeOH = 20/1 to 5/1) to yield the target compound DD02008H (71.94 mg, 183 umol, 40.9% yield, 98.6% purity) as yellow solids, the structure of which was confirmed by $^1$H NMR, LCMS and HPLC.

> **LCMS** product: RT = 0.710 min, m/z = 416.2 $(M+H+Na)^+$
> **HPLC** product: RT = 1.267 min, 98.7% purity
> **$^1$H NMR**(400 MHz, $CDCl_3$)$\delta$ 9.04 (s, 1H), 7.72 (br d, *J*= 8.0 Hz, 2H), 7.33 (dd, *J* = 8.0, 4.0 Hz, 1H), 7.15 - 7.24 (m, 2H), 6.97 (d, *J* = 8.0 Hz, 2H), 4.33 - 4.41 (m, 2H), 3.91 - 3.99 (m, 2H), 3.56 (s, 3H), 3.13 - 3.25 (m, 4H), 2.58 - 2.65 (m, 4H), 2.38 (s, 3H).

1.6 Synthesis of Compound DD02015H: 3-((2-((4-(4-(4-methylpiperazin-1-yl)phenyl)amino)quinazolin-8-yl)oxo)cyclopentan-1-ol

The synthetic route of compound DD02015H is shown in Figure 14.

Compound 17 (150 mg, 447 umol, 1.00 eq), Compound 18a (331 mg, 894 umol, 2.00 eq) and $Cs_2CO_3$ (364 mg, 1.12 mmol, 2.50 eq) were dissolved in DMF (10.0 mL), then the mixture was heated to 80 °C and stirred for 12 h. LCMS detection showed the target product (RT = 0.893 min) and TLC (DCM: MeOH = 10: 1, raw material Rf = 0.1, product Rf = 0.25) showed the formation of a new spot and no raw material. After the reaction was completed, the resulting mixture was

poured into 30 mL of water and subsequently extracted with ethyl acetate (30.0 mL * 4), washed with brine (30.0 mL * 2), dried over anhydrous sodium sulfate, filtered, and the solvent was removed under reduced pressure. The resulting residual solid was purified by column chromatography (SiO2, DCM/MeOH=20/1 to 5/1) to give Compound 19 (200 mg. 375 umol, 83.8% yield) as yellow solids.

**LCMS** product: RT = 0.893 min, m/z = 534.4 (M+H)+

Compound 19 (200 mg, 375 umol, 1.00 eq) was dissolved in DCM (6.00 mL), and HCl /1,4-dioxane (4 M, 4.00 mL, 42.7 eq) was added dropwise at 25 °C. After the dropwise addition, and the reaction mixture was stirred for 2 h. The LCMS detection showed the target product (RT = 0.721 min), and the HPLC showed that the starting material was consumed. After the reaction was completed, the mixture was poured into 30.0 mL of $H_2O$ and adjusted to pH 8 with saturated $NaHCO_3$, then extracted with ethyl acetate (30.0 mL*4), and the organic layer was washed with brine (30.0 mL*2), dried over anhydrous $Na_2SO_4$, filtered and concentrated under reduced pressure, and the resulting crude product was purified by preparative high performance liquid chromatography (HPLC) to give the target compound DD02015H (110 mg, 262 umol, 70.0% yield, 100% purity) as yellow solids, the structure of which was confirmed by $^1$H NMR, LCMS and HPLC.

**LCMS** product: RT = 0.716 min, m/z = 420.3 (M+H)+
**HPLC** product: RT = 1.286 min, 100% purity.
**$^1$H NMR**(400 MHz, $CDCl_3$)$\delta$ 9.04 (s, 1 H), 7.60 (br d, $J$ = 8.0 Hz, 2 H), 7.32 - 7.38 (m, 1 H), 7.21 - 7.24 (m, 2 H), 6.93 - 7.01 (m, 2 H), 5.15 (t, $J$ = 4.0 Hz, 1 H), 4.41 (br t, $J$= 4.0 Hz, 1 H), 3.17 - 3.26 (m, 4 H), 2.62 (m, 4 H), 2.38 (s, 3 H), 1.94 - 2.20 (m, 6 H).

1.7 Synthesis of Compound DD02021H: 4-((2-((5-(4-methylpiperazin-1-yl)pyridin-2-yl)amino)quinazolin-8-yl)oxo)cyclohexan-1-ol

The synthetic route of compound DD02021H is shown in Figure 15.

1.7.1 Synthesis of Compound 20

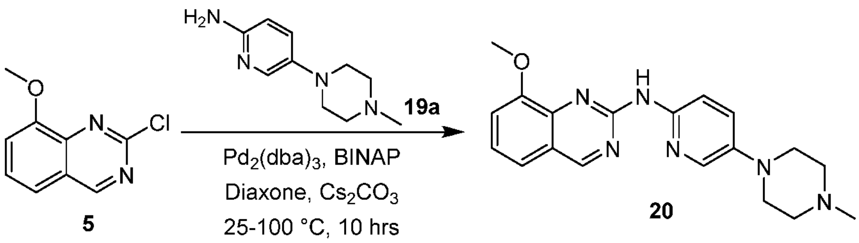


5

20

At 25 °C, Compound 5 (500 mg, 2.57 mmol, 1.00 eq), Compound 19a (988 mg, 5.14 mmol, 2.00 eq), $Pd_2(dba)_3$ (353 mg, 386 umol, 0.15 eq) and BINAP (184 mg, 295 umol, 1.15e-1 eq) were dissolved in 1,4-dioxane (30.0 mL) and $Cs_2CO_3$ (1.67 g, 5.14 mmol, 2.00 eq) was added into the resulting mixture. Then the reaction mixture was heated to 100 °C and stirred for 10 h. LCMS detection showed the target product (RT = 0.643 min). After the reaction was completed, the mixture was poured into 30.0 mL of $H_2O$ and then extracted with DCM (50.0 mL*3), and the organic layer was washed with brine (30.0 mL*3), dried over anhydrous $Na_2SO_4$, filtered and concentrated under reduced pressure to give Compound 20 (600 mg, crude) as yellow solids. The product was not purified and used directly in the next step of the reaction.

1.7.2 Synthesis of Compound 21

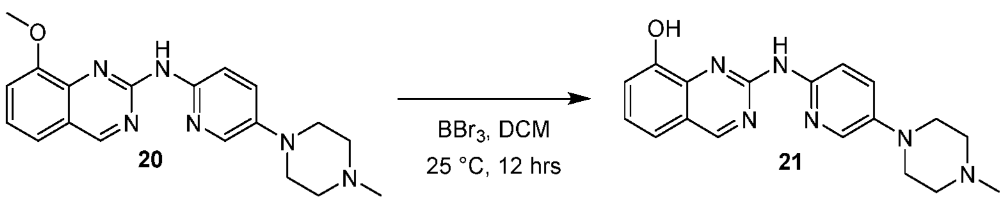


20

21

Compound 20 (600 mg, 1.71 mmol, 1.00 eq) was dissolved in DCM (10.0 mL), the resulting solution was added

dropwise into $BBr_3$ (429 mg, 1.71 mmol, 165 uL, 1.00 eq) dissolved in DCM (5.00 mL). After the dropwise addition was completed, the system was stirred for 12 hr at 25 °C. LCMS detection showed the target product (RT = 0.321 min). After the reaction was completed, the mixture was poured into ice water (40.0 mL) and the pH was adjusted to about 8 with saturated $NaHCO_3$, then extracted with DCM (50.0 mL*3), and the organic layer was washed with brine (30.0 mL*3), dried over anhydrous sodium sulfate, filtered and concentrated under reduced pressure to give Compound 21 (600 mg, crude) as yellow solids. The product was not purified and was used directly in the next step of the reaction.

**LCMS** product RT = 0.324 min, m/z = 337.2 $(M+H)^+$

1.7.3 Synthesis of Compound 22

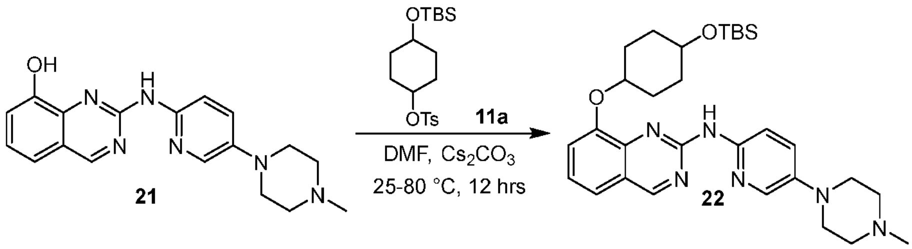


Compound 21 (600 mg, 1.78 mmol, 1.00 eq), Compound 11a (1.37 g, 3.57 mmol, 2.00 eq) and $Cs_2CO_3$ (1.45 g, 4.46 mmol, 2.50 eq) were dissolved in DMF (10.0 mL), and the resulting mixture was heated to 80 °C for 12 h. LCMS detection showed the target product (RT = 0.830 min) and thin layer chromatography (DCM: MeOH = 10: 1, raw material $R_f$ = 0.1, product Rf = 0.3) showed the formation of a new spot while no starting material. After the reaction was completed, the mixture was poured into 30.0 mL of $H_2O$ and then extracted with ethyl acetate (60.0 mL*3), and the organic layer was washed with brine (40.0 mL*2), dried over anhydrous $Na_2SO_4$, filtered and concentrated under reduced pressure, and the resulting material was purified by column chromatography ($SiO_2$, DCM/MeOH = 40/1 to 5/1) to give Compound 22 (700 mg 1.08 mmol, 60.5% yield, 84.6% purity) as yellow solids.

**LCMS** product RT = 0.830 min, m/z = 549.4 $(M+H)^+$
**HPLC** product RT = 2.563 mins, 84.6% purity.

1.7.4 Synthesis of Compound DD02021H

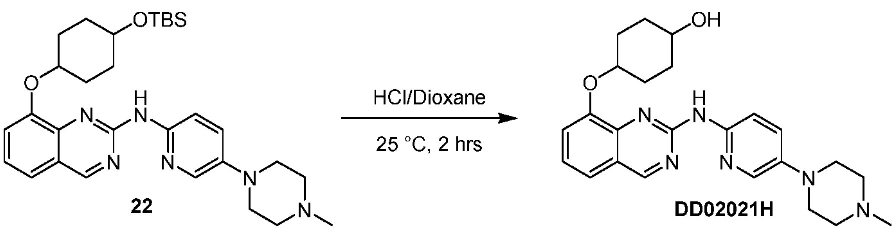


At 25 °C, Compound 22 (600 mg, 925 umol, 1.00 eq.), HCl/1,4-dioxane (4 M, 6.00 mL, 25.9 eq.) were dissolved in DCM (10.0 mL) and the resulting mixture was stirred for 1 hr. LCMS detection showed the target product (RT = 0.680 min) and thin layer chromatography (DCM: MeOH = 10: 1, raw material Rf = 0.3, product Rf = 0.15) showed the formation of a new spot while no starting material. After the reaction was completed, the mixture was slowly poured into 40.0 mL of $H_2O$ with stirring, then extracted with ethyl acetate (80.0 mL*3), and the organic layer was washed with brine (50.0 mL*2), dried over anhydrous $Na_2SO_4$, filtered and concentrated under reduced pressure. The resulting solid was purified by preparative high performance liquid chromatography, the resulting substance was adjusted pH to about 8 with saturated $NaHCO_3$, then extracted with DCM (40.0 mL*4), and the organic layer was washed with brine (40.0 mL*3), dried over anhydrous $Na_2SO_4$, filtered and concentrated under reduced pressure to obtain the target product DD02021H, (148 mg, 329 umol, 35.6% yield, 96.7% purity) as yellow solids, the structure of which was confirmed by $^1H$ NMR, LCMS and HPLC.

**LCMS** product RT = 0.717 min, m/z = 435.3 $(M+H)^+$
**HPLC** product RT = 1.232 min, 96.7% purity

**$^1$H NMR**(400 MHz, DMSO)$\delta$ 9.74 (s, 1H), 9.26 (s, 1H), 8.86 (d, *J*= 8.0 Hz, 1H), 8.02 (d, *J*= 4.0 Hz, 1H), 7.43 - 7.57 (m, 2H), 7.24 - 7.40 (m, 2H), 4.78 (br s, 1H), 4.62 - 4.73 (m, 1H), 3.54 - 3.69 (m, 1H), 3.08 - 3.16 (m, 4H), 2.47 (m, 3H), 2.23 (s, 3H), 1.93 - 2.04 (m, 2H), 1.74 - 1.88 (m, 2H), 1.58 - 1.72 (m, 4H).

1.8 Synthesis of Compound DD02018H: 4-((8-((4-hydroxycyclohexyl)oxo)quinazolin-2-yl)amino)-N-(1-methylpiperidin-4-yl)benzamide

The synthetic route of compound DD02017H is shown in Figure 16.

Compound 11 (400 mg, 2.21 mmol, 1.00 eq), Compound 11a (1.28 g, 3.32 mmol, 1.50 eq) and $Cs_2CO_3$ (1.44 g, 4.43 mmol, 2.00 eq) were dissolved in DMF (10.0 mL) at 25 °C and the resulting mixture was heated to 80 °C and stirred for 2 hr. LCMS detection showed the target product (RT = 1.218 min). After the reaction was completed, the mixture was slowly poured into 40.0 mL of $H_2O$ and then extracted with DCM (80.0 mL*3), and the organic layer was washed with brine (50.0 mL*2), dried over anhydrous $Na_2SO_4$, filtered and concentrated under reduced pressure to give Compound 15 (310 mg, crude) as grey solids. The product was not purified and used directly in the next step of the reaction.

Compound 15 (260 mg, 661 umol, 1.00 eq), Compound 15a (231.53 mg, 992.38 umol, 1.50 eq) and TFA (75.4 mg, 661 umol, 49.0 uL, 1.00 eq) were dissolved in IPA (10.0 mL), then the resulting mixture was heated to 80 °C and stirred for 2 h. The mixture was then heated to 100 °C and stirred for 2 h. LCMS detection showed the target product (RT = 0.762 min) and HPLC showed a purity of 59.7% (RT = 1.535 min). After the reaction was completed, the mixture was slowly poured into 40.0 mL of $H_2O$ under stirring, and the pH was adjusted to about 8 with saturated $NaHCO_3$, then extracted with DCM (80.0 mL*3), and the organic layer was washed with brine (50.0 mL*2), dried over anhydrous sodium sulphate, filtered and concentrated under reduced pressure. The resulting crude product was purified by preparative high-performance liquid chromatography (HPLC), and the resulting material was adjusted by a saturated $NaHCO_3$ to about pH 8, extracted with DCM (40.0 mL*4). The organic layer was washed with brine (40.0 mL*3), dried with anhydrous $Na_2SO_4$, filtered, and concentrated to obtain the target product DD02018H (17.1 mg, 35.13 umol, 5.31% yield, 97.7% purity) as light yellow solids, the structure of which was confirmed by $^1$H NMR, LCMS and HPLC.

**LCMS** product RT = 0.771 min, m/z = 476.2 $(M+H)^+$
**HPLC** product RT = 1.535 mins, 97.3% purity
**$^1$H NMR**(400 MHz, DMSO-$d_6$)$\delta$ 10.15 (br s, 1H), 9.30 (br s, 1H), 8.23 (br d, *J*= 8.0 Hz, 2H), 8.13 (br s, 1H), 7.83 (br d, *J* = 8.0 Hz, 2H), 7.49 (br s, 1H), 7.27 - 7.42 (m, 2H), 4.73 (br s, 1H), 4.60 (br s, 1H), 3.87 (br s, 1H), 3.07 (br s, 2H), 2.62 - 2.67 (m, 2H), 2.33 (br s, 3H), 1.84 - 2.08 (m, 3H), 1.73 (m, 9H).

1.9 Synthesis of Compound DD02002H: 4-((8-((3-hydroxycyclopentyl)oxo)quinazolin-2-yl)amino)benzenesulfonamide

The synthetic route of compound DD02002H is shown in Figure 17.

1.9.1 Synthesis of Compound 23

Compound 11 (200 mg, 1.11 mmol) was dissolved in anhydrous tetrahydrofuran (5 mL) under $N_2$, triphenylphosphine (581 mg, 2.22 mmol), DIAD (449 mg, 2.22 mmol) were added sequentially and stirred for 15 min, and finally 1,3-cyclopentanediol (i.e. Compound 22, 340.1 mg, 3.33 mmol) was added. The reaction solution was stirred at room temperature overnight, and TLC (PE/EA = 3: 1) analysis showed that the raw material was consumed and a new product spot was generated. After the reaction was completed, the resulting mixture was concentrated, the solvent was removed, and the crude product was separated on a silica gel column to give Compound 23 (210 mg) in 71.5% yield as light yellow solids.

**LCMS** product RT=2.76 min, m/z = 265.1 $(M+H)^+$

1.9.2 Synthesis of Compound DD02002H

Compound 23 (200 mg, 0.76 mmol) was dissolved in isopropanol (5 mL) under $N_2$, p-aminobenzenesulfonamide (260.2 mg, 1.51 mmol) was added, and the reaction was heated to 90 °C overnight. Thin layer chromatography (TLC) analysis showed that some of the raw material was left, and there was a new product spot. LCMS detection showed the target product. After the reaction was completed, the reaction mixture was concentrated and the crude product was separated on a silica gel column to yield the target molecule DD02002H (104 mg) in 34% yield as white solids, the structure of which was confirmed by $^1$H NMR and LCMS.

**LCMS** product RT = 3.46 min, m/z = 401.1 $(M+H)^+$
**$^1$H NMR**(400 MHz, MeOD)$\delta$ 9.21(s, 1H), 8.23-8.25(m, 2H), 7.87-7.89(m, 2H), 7.48(d, *J*= 7.6 Hz, 1H), 7.33-7.37(m,

2H), 4.36(m, 1H), 4.20(m,1H), 1.76-2.10(m, 6H).

1.10 Synthesis of Compound DD02019H: 3-((8-((4-hydroxycyclohexyl)oxo)quinazolin-2-yl)amino)benzenesulfonamide

The synthetic route of compound DD02019H is shown in Figure 18.

1.10.1 Synthesis of Compound 26

Compound 11 (200 mg, 1.11 mmol) was dissolved in anhydrous tetrahydrofuran (5 mL) under $N_2$, triphenylphosphine (581 mg, 2.22 mmol), DIAD (449 mg, 2.22 mmol) were added sequentially, and the resulting mixture was stirred for 15 min, and finally 1,4-cyclohexanediol (387 mg, 3.33 mmol) was added. The resulting reaction solution was stirred at room temperature overnight, TLC (PE/EA = 3: 1) analysis showed that the raw materials were consumed, and a new product spot was generated. LCMS detection showed the target product (RT = 4.03 min), the reaction solution was concentrated, and the resulting crude product was separated on a silica gel column to give Compound 26 (197 mg) in 70.7% yield as light yellow solids.

**LCMS** product RT = 4.03 min, m/z =279.1 $(M+H)^+$

1.10.2 Synthesis of Compound DD02019H

Under $N_2$, Compound 26 (150 mg, 0.54 mmol) was dissolved in isopropanol (5 mL), m-aminobenzenesulfonamide (185.4 mg, 1.1 mmol) was added, and the reaction was heated to 90 °C overnight. TLC analysis showed that the raw material was left, while there was a new product spot. LC-MS detection showed the target product, and the reaction solution was concentrated, and the resulting crude was separated on a silica gel column. The reaction solution was concentrated, and the crude product was separated on a silica gel column as as to obtain the target molecule DD02019H (27 mg) in 12.1% yield as white solids, the structure of which was confirmed by LC-MS and $^1$H NMR.

**LCMS** product RT = 3.54 min, m/z = 415.2 $(M+H)^+$
**$^1$H NMR** (400 MHz, MeOD)δ 9.19 (s, 1H), 8.51(m, 2H), 7.34-7.55(m, 5H), 4.62 (m, 1H), 3.73(m, 1H), 1.57-1.94(m, 8H).

1.11 Tests of compounds of the formula 1-1 for inhibiting HPK1 kinase activity *in vitro*

Table 1. Reagents required for the experiment and sources thereof

| Material and reagent name | Supplier | Cat# |
|---|---|---|
| HPK1 | Signalchem | M23-11G-10 |
| MBP | Signalchem | M42-52N-1MG |
| ADP-Glo Kinase Assay | Promega | V9102 |
| DMSO | Sigma | D8418 |
| Sunitinib | Selleck | C64749 |
| ATP | Promega | V915B |

The starting concentration for all tested compounds was 10 μM, and diluted in threefold concentration gradient for a total of 10 concentration points in duplicate.

1.11.1 Tests of Compounds for inhibiting HPK1 kinase activity *in vitro*

Before the experiment, 50 μM DTT and the enzymatic reaction system buffer were prepared.

The compound dilution was transferred into 384-well plate by using a pipette, afterwards, the 384-well plate was sealed and centrifuged at 1000 g for 1 min. 2-fold concentration of HPK1 solution was prepared in the kinase buffer, and then 2.5 μL of the prepared 2-fold concentration of HPK1 solution was added into the 384 wells, centrifuged at 1000 g for 30 s, and incubated for 10 min at room temperature. A mixture of 2-fold concentration of MBP and ATP was prepare in the kinase buffer, and 2.5 μL of prepared mixture of 2-fold concentration of MBP and ATP was added into the above reaction

system for the reaction, centrifuged at 1000 g for 30 s, and then incubated at room temperature for 1 h. 5 μL of ADP-Glo reagent was added into the reaction system, and incubated at room temperature for 40 min. 10 μL of kinase detection reagent was added and incubated at room temperature for 40 min. Then the luminescence signals were read on an Envision 2104 plate reader, and the inhibition rate was calculated according to the following formula:

$$\% \text{ inhibition} = 100 - (\text{Signal}_{\text{cmpd}} - \text{Signal}_{\text{Ave_PC}}) / (\text{Signal}_{\text{Ave_VC}} - \text{Signal}_{\text{Ave_PC}}) \times 100$$

In the above formula, cmpd refers to the tested compound, PC refers to the positive control, and VC refers to the negative control. The positive control used in the HPK1 experiment was Sunitinib.

Table 2. The ability of the compounds described in each Example to inhibit HPK1 kinase activity

| Example | HPK1 activity/$IC_{50}$, nM |
|---|---|
| 1 | <50 |
| 4 | <50 |
| 2 | <50 |
| 6 | <100 |
| 7 | <300 |
| 9 | <300 |
| 5 | <200 |
| 3 | <50 |
| 8 | <50 |
| 10 | >300 |
| Sunitinib | 5.20 |

**2. Synthesis and assay on HPK1 inhibitory activity of compounds of formula 1-2**

2.1 Synthesis of compound (1S,2S)-N-(8-amino-7-fluoro-6-(1-methyl-1H-pyrrole-2-yl)isoquinolin-3-yl)-2-fluorocyclopropane-1-carboxamide (i.e. DD02001A_cis) and compound (1S,2R)-N-(8-amino-7-fluoro-6-(1-methyl-1H-pyrrole-2-yl)isoquinolin-3-yl)-2-fluorocyclopropane-1-carboxamide (i.e. DD02001A_trans)

2.1.1 Synthesis of Compound 2

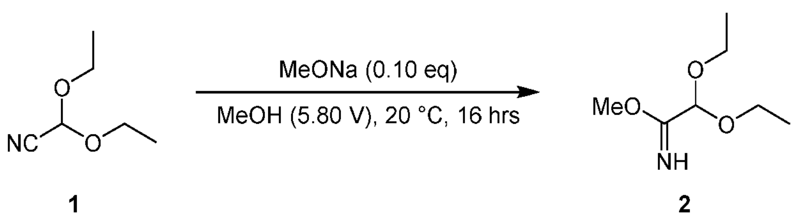


1 2

To a solution of Compound 1 (345 g, 2.67 moL, 371 mL, 1.00 eq) in MeOH (2.00 L) was added MeONa (14.4 g, 267 mmol, 0.10 eq), purged three times with $N_2$, and then stirred for 16 hr at 20 °C. TLC (petroleum ether/ethyl acetate = 5/1) showed that Compound 1 (Rf = 0.70 ) disappeared and a new spot was detected (Rf = 0.45). The resulting mixture was adjusted to about pH 9 with dry ice and then concentrated under vacuum to obtain a yellow mixture. The mixture was poured into water (1.00 L) and then extracted with ethyl acetate (1.00 L*2). The organic layer was dried over anhydrous $Na_2SO_4$, filtered and concentrated under vacuum to give Compound 2 (400 g, 2.48 moL, 92.9% yield) as a pale yellow oily substance, the structure of which was confirmed by $^1$H NMR.

$^1$H NMR(400 MHz, $CDCl_3$) δ 7.89 (s, 1H), 4.80 (s, 1H), 3.81 (s, 3H), 3.61 -3.53 (m, 4H), 1.24 (t, *J* = 7.2 Hz, 6H).

2.1.2 Synthesis of Compound 3

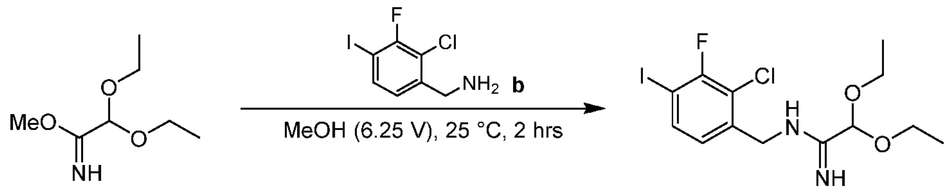


2 3

To a solution of Compound b (80.0 g, 171 mmoL, 61.0% purity, 1.00 eq) in MeOH (500 mL) was added Compound 2 (41.3 g, 256 mmol, 1.50 eq) at 25 °C and then stirred at 25 °C for 2 h. LCMS showed that Compound b disappeared and the desired mass was detected. After the reaction was completed, the resulting liquid mixture was concentrated at 45 °C under reduced pressure. The residue was dissolved in ethyl acetate (1.00 L) and washed with brine (200 mL*2). The organic phase was dried over $Na_2SO_4$, filtered and concentrated to obtain a residue. The residue was ground with petroleum ether/ethyl acetate (5/1, 150 ml) at 20 °C and filtered to obtain a filter cake so as to give Compound 3 (53.0 g, 121 mmoL, 71.0% yield, 95.0% purity) as pale yellow solids, the structure of which was confirmed by LC-MS as well as $^1$H NMR.

**LCMS:** m/z = 415.0 $(M+H)^+$
**$^1$H NMR:** (400 MHz, DMSO-$d_6$) $\delta$ 7.76 (dd, *J* = 6.2, 8.2 Hz, 1H), 7.13 (d, *J* = 8.0 Hz, 1H), 6.62 - 6.19 (m, 1H), 4.76 (s, 1H), 4.23 (s, 2H), 3.66 - 3.42 (m, 4H), 1.15 (t, *J* = 7.0 Hz, 6H).

2.1.3 Synthesis of Compound 4

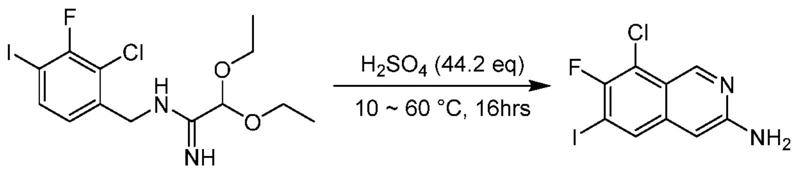


3 4

Compound 3 (94.0 g, 212 mmol, 93.5% purity, 1.00 eq.) was added to $H_2SO_4$ (920 g, 9.38 moL, 500 mL, 44.2 eq.) at 10 °C, and then heated to 60 °C for 16 h. LCMS showed that Compound 3 was consumed and the molecular weight of the target product was detected. After the reaction was completed, the reaction solution was cooled to 25 °C with ice (5.00 L), the pH was adjusted to about 13 with NaOH solids, the temperature was kept below 20 °C, a large amount of solids appeared and subjected to filtration, and a filter cake was dried in vacuum and ground with ethyl acetate (300 mL) at 25 °C to give Compound 4 (60.0 g, 186 mmol, 87.7% yield) as yellow solids, the structure of which was confirmed by LC-MS and $^1$H NMR.

**LCMS:** m/z = 322.9 $(M+H)^+$
**$^1$H NMR:** (400 MHz, DMSO-$d_6$) $\delta$ 8.96 (s, 1H), 8.21 (d, *J*= 5.8 Hz, 1H), 6.62 (s, 1H), 6.31 (s, 2H).

2.1.4 Synthesis of Compound 5

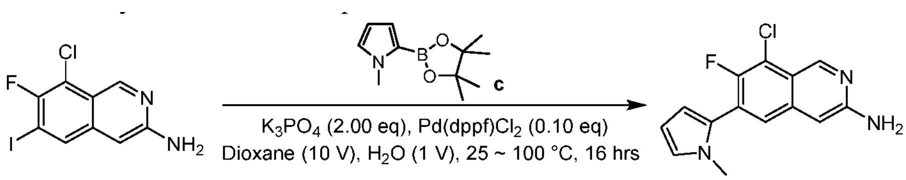


4 5

Compound 4 (41.0 g, 121 mmol, 95.6% purity, 1.00 eq.) and Compound c (30.2 g, 146 mmoL, 1.20 eq.) were dissolved in dioxane (410 mL) and $H_2O$ (41.0 mL) at 25 °C and to the resulting solution were added $K_3PO_4$ (51.6 g, 243 mmoL, 2.00 eq.) and Pd(dppf)$Cl_2$ (8.89 g, 12.1 mmol, 0.10 eq), the resulting suspension was degassed under vacuum and purged several times with $N_2$, and then stirred at 100 °C for 7 h. LCMS showed that Compound 4 was completely consumed and the molecular weight of the target product was detected. After the reaction was completed, the resulting mixture was quenched with ice water (1000 mL) and then extracted with dichloromethane (1000 mL*2), the organic layer was dried over anhydrous $Na_2SO_4$, filtered and concentrated under vacuum, and the resulting solids were ground with petroleum ether/ethyl acetate (2/1, 200 mL) at 25 °C to give Compound 5 (33.0 g, 103 mmol, 85.3% yield, 86.7% purity) as yellow solids, the structure of which was confirmed by LC-MS and $^1$H NMR.

**LCMS:** m/z = 276.1 $(M+H)^+$
**$^1$H NMR**(400 MHz, $CDCl_3$)$\delta$ 9.20 (s, 1H), 7.41 (d, *J*=6.6 Hz, 1H), 6.92 - 6.79 (m, 1H), 6.71 (s, 1H), 6.36 (ddd, *J*= 0.8, 1.6, 3.6 Hz, 1H), 6.27 (dd, *J* = 2.8, 3.6 Hz, 1H), 4.57 (s, 2H), 3.64 (d, *J*= 1.6 Hz, 3H).

2.1.5 Synthesis of Compound 6

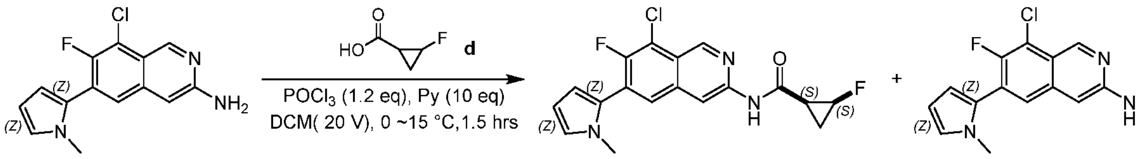


5 6_Cis 6_Trans

Compound 5 (33.0 g, 104 mmoL, 86.7% pure, 1.00 eq.), Compound d (13.0 g, 124 mmoL, 1.20 eq.) and pyridine Py (82.1 g, 1.04 mol, 83.8 mL, 10.0 eq.) were dissolved in DCM (500 mL) at 0 °C, and to the resulting solution was added $POCl_3$ ( 19.3 g, 126 mmol, 11.7 mL, 1.22 eq). And then the reaction solution was stirred at 15 °C for 1.5 h. TLC (petroleum ether/ethyl acetate = 1/1) showed that Compound 5 (Rf = 0.40) was consumed and the target product spot (Rf = 0.47, 0.75) was detected. After the reaction was completed, the reaction mixture was concentrated under reduced pressure to give a residue, which was purified by silica gel chromatography (petroleum ether/ethyl acetate = 8/1-5/1, $R_f$ = 0.47, 0.75) to give Compound 6_cis (7.00 g, 19.3 mmoL, 18.6% yield, 100% purity) as pale yellow solids and Compound 6_trans ( 5.00 g, 13.7 mmoL, 13.2% yield, 99.1%) as pale yellow solids.

**LCMS:** RT = 0.959 min, m/z = 362.1 $(M+H)^+$
**LCMS:** RT = 0.986 min, m/z = 362.1 $(M+H)^+$

Compound 6_cis: **$^1$H NMR:** (400 MHz, $CDCl_3$)$\delta$ 9.31 (s, 1H), 8.61 (s, 2H), 7.66 (d, *J* = 6.6 Hz, 1H), 6.94 - 6.80 (m, 1H), 6.43 (td, *J*= 1.6, 3.4 Hz, 1H), 6.29 (dd, *J* = 2.8, 3.6 Hz, 1H), 5.00 - 4.73 (m, 1H), 3.66 (d, *J*= 1.4 Hz, 3H), 2.08 - 1.87 (m, 2H), 1.34 - 1.24 (m, 1H).
Compound 6_trans: **$^1$H NMR:** (400 MHz, $CDCl_3$)$\delta$ 9.33 (s, 1H), 8.59 (s, 1H), 8.49 (s, 1H), 7.66 (d, *J*= 6.6 Hz, 1H), 6.93 - 6.80 (m, 1H), 6.42 (td, *J*= 1.6, 3.4 Hz, 1H), 6.29 (dd, *J* = 2.8, 3.6 Hz, 1H), 5.10 - 4.81 (m, 1H), 3.66 (d, *J* = 1.4 Hz, 3H), 2.21 - 2.08 (m, 1H), 1.60 - 1.50 (m, 2H).

2.1.6 Synthesis of Compound 7_cis

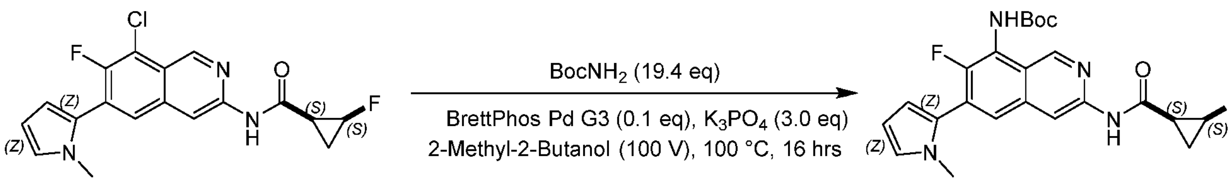


6_Cis 7_Cis

Compound 6_Cis (10.0 g, 27.6 mmol, 1.00 eq), $BocNH_2$ (62.8 g, 536 mmol, 19.4 eq), BrettPhos Pd G3 (2.51 g,

2.76 mmol, 0.10 eq) and $K_3PO_4$ (17.6 g, 82.9 mmol, 3.00 eq) were dissolved in 2-methyl-2-butanol (1.00 L). The resulting solution was degassed and purged three times with $N_2$, and then the reaction was stirred at 100 °C under $N_2$ for 16 h. LCMS showed that Compound 6_Cis was consumed and the molecular weight of the target product was detected. After the reaction was completed, the reaction mixture was filtered, the resulting filtrate was concentrated under vacuum, and the residue was purified by silica gel chromatography (petroleum ether/ethyl acetate = 10/1-3/1, Rf = 0.40) to give Compound 7_cis (3.90 g, 8.31 mmoL, 30.1% yield, 94.3% purity) as pale yellow solids, the structure of which was confirmed by LC-MS and $^1$H NMR.

**LCMS:** m/z = 443.3 $(M+H)^+$
**HPLC:** 94.3% purity under 220 nm.
**$^1$H NMR:** (400 MHz, DMSO-$d_6$) δ 10.99 (s, 1H), 9.33 (s, 1H), 9.09 (s, 1H), 8.51 (s, 1H), 7.84 (d, *J*= 6.6 Hz, 1H), 7.00 (d, *J* = 1.8 Hz, 1H), 6.52 - 6.29 (m, 1H), 6.23 - 6.13 (m, 1H), 5.15 - 4.81 (m, 1H), 3.63 (s, 3H), 2.30 - 2.15 (m, 1H), 1.77 - 1.60 (m, 1H), 1.47 (s, 9H), 1.25 - 1.02 (m, 1H).

2.1.7 Synthesis of Compound 7_trans

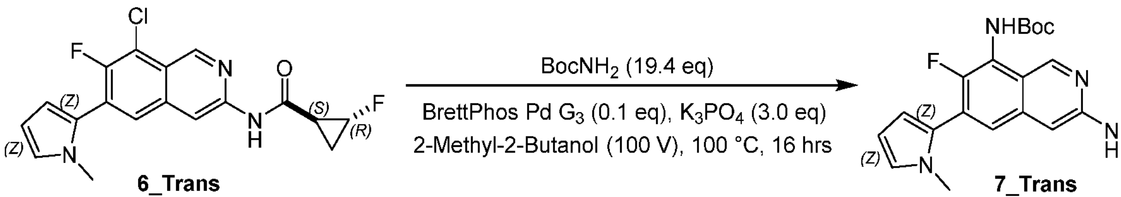


**6_Trans** **7_Trans**

Compound 6_trans (10.0 g, 27.6 mmol, 1.00 eq), $BocNH_2$ (62.8 g, 536 mmol, 19.4 eq), BrettPhos Pd $G_3$ (2.51 g, 2.76 mmol, 0.10 eq) and $K_3PO_4$ (17.6 g, 82.9 mmol, 3.00 eq) were dissolved in 2-methyl-2-butanol (1.00 L), and the resulting solution was degassed, purged with $N_2$ for three times and stirred at 100°C under $N_2$ for 16 h. LCMS showed that Compound 6_trans was consumed and the molecular weight of the target product was detected. After the reaction was completed, the reaction mixture was filtered, the filtrate was concentrated under reduced pressure, and the resulting solids were purified by silica gel chromatography (petroleum ether/ethyl acetate = 10/1-5/1, Rf = 0.50) to give Compound 7_trans (5.50 g, 10.8 mmol, 39.3% yield, 87.4% purity) as pale yellow solids, the structure of which was confirmed by LC-MS and $^1$H NMR.

**LCMS:** m/z = 443.3 $(M+H)^+$
**HPLC:** 87.4% purity under 220 nm.
**$^1$H NMR:** (400 MHz, DMSO-$d_6$) δ 11.11 (s, 1H), 9.33 (s, 1H), 9.09 (s, 1H), 8.45 (s, 1H), 7.82 (d, *J* = 6.6 Hz, 1H), 7.04 - 6.93 (m, 1H), 6.45 - 6.31 (m, 1H), 6.17 (dd, *J* = 2.6, 3.6 Hz, 1H), 5.11 - 4.77 (m, 1H), 3.62 (s, 3H), 2.74 - 2.57 (m, 1H), 1.67 - 1.42 (m, 10H), 1.27 (dt, *J* = 6.6, 13.0 Hz, 1H).

2.1.8 Synthesis of Compound DD02001A_cis

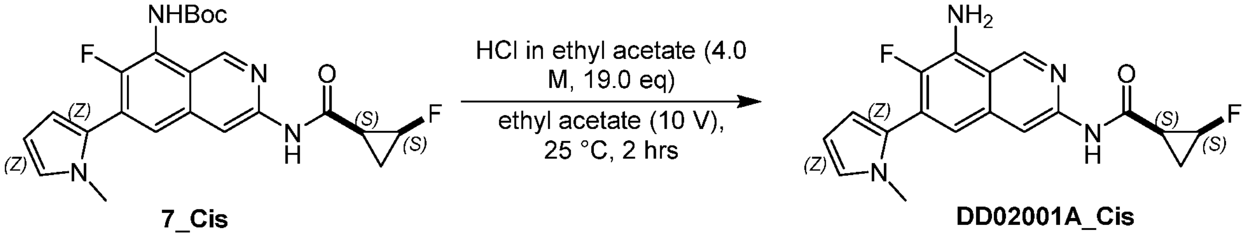


**7_Cis** **DD02001A_Cis**

Compound 7_Cis (3.90 g, 8.31 mmol, 94.3% pure, 1.00 eq.) was dissolved in ethyl acetate (40.0 mL), HCl/ethyl acetate (4 M, 39.4 mL, 18.9 eq.) was added to the above solution at 25 °C, and the reaction was stirred for 16 hr at 25 °C. LCMS showed that Compound 7_Cis was consumed completely and the molecular weight of the target product was detected. After the reaction was completed, the resulting solution was concentrated under reduced pressure to obtain a solid, the pH was adjusted with saturated aqueous $NaHCO_3$ solution to about 8, and then the residue was extracted with

dichloromethane / methanol = 10/1 (100 mL), and the resulting organic layer was dried over anhydrous sodium sulfate, filtered, and distilled under reduced pressure to obtain DD02001A_Cis (3.07 g, 8.17 mmol, yield 98.3%, purity 93.2%) as yellow solids, the structure of which was confirmed by LCMS, $^1$H NMR and F NMR.

**LCMS:** m/z = 343.2 $(M+H)^+$
**HPLC:** 93.2% purity under 220 nm.
**$^1$H NMR:**(400 MHz, DMSO-$d_6$)$\delta$ 10.80 (s, 1H), 9.36 (s, 1H), 8.28 (s, 1H), 7.06 - 6.88 (m, 2H), 6.31 - 6.10 (m, 4H), 5.08 - 4.78 (m, 1H), 3.60 (s, 3H), 2.30 - 2.17 (m, 1H), 1.74 - 1.60 (m, 1H), 1.18 - 1.09 (m, 1H)
**FNMR**:(400 MHz, DMSO-$d_6$)$\delta$ -141.11, -221.11.

2.1.9 Synthesis of Compound DD02001A_trans

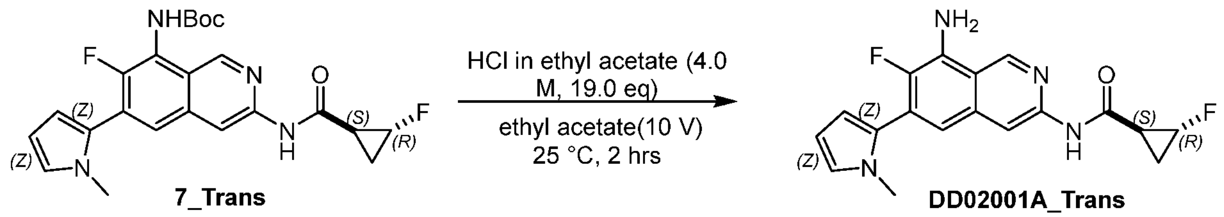


7_Trans          DD02001A_Trans

Compound 7_trans (5.50 g, 10.8 mmol, 87.0% purity, 1.00 eq.) was dissolved in ethyl acetate (40.0 mL), HCl/ethyl acetate (4 M, 51.3 mL, 19.0 eq.) was added to the above solution at 25 °C, and the resulting mixture was stirred at 25 °C for 16 h. LCMS showed that Compound 7_trans was consumed and the molecular weight of the target product was detected. After the reaction was completed, the reaction solution was concentrated under reduced pressure to obtain a solid, the pH was adjusted with saturated aqueous $NaHCO_3$ solution to about 8, and then extracted with dichloromethane/methanol = 10/1 (100 mL), and the resulting organic layer was dried over anhydrous sodium sulfate, filtered, concentrated under reduced pressure, slurried in petroleum ether/ethyl acetate = 2/1 (30 mL) at 25 °C, and filtered, and the resulting filter cake was dried under vacuum to obtain DD02001A_trans (3.30 g, 9.20 mmol, 85.0% yield, 95.4% purity) as yellow solids, the structure of which was confirmed by LCMS, $^1$H NMR and F NMR.

**LCMS:** m/z = 343.2 $(M+H)^+$
**HPLC:** 95.4% purity under 220 nm.
**$^1$H NMR:** (400 MHz, DMSO-$d_6$)$\delta$ 10.94 (s, 1H), 9.38 (s, 1H), 8.24 (s, 1H), 7.03 - 6.91 (m, 2H), 6.25 - 6.22 (m, 3H), 6.14 - 6.12 (m, 3H), 5.07 - 4.79 (m, 1H), 3.60 (s, 3H), 2.67 - 2.54 (m, 1H), 1.62 - 1.44 (m, 1H), 1.35 - 1.19 (m, 1H)
**FNMR:** (400 MHz, DMSO-$d_6$)$\delta$ -140.94, -206.78.

2.2 Tests of compounds of the formula I-2 for inhibiting kinase activity *in vitro*

Table 3. Reagents required for the experiment

| Material and reagent name | Supplier | **Cat#** |
|---|---|---|
| HPK1 | Signalchem | M23-11G-10 |
| GCK | Signalchem | M24-10G-10 |
| TNIK | Signalchem | T27-11G-10 |
| MBP | Signalchem | M42-52N-1MG |
| ADP-Glo Kinase Assay | Promega | V9102 |
| DMSO | Sigma | D8418 |
| Sunitinib | Selleck | C64749 |
| ATP | Promega | V915B |
| PDK1 | Invitrogen | P3001 |

(continued)

| Material and reagent name | Supplier | **Cat#** |
|---|---|---|
| Staurosporine | MCE | HY-15141 |

The starting concentration for all tested compounds was 10 μM, and diluted in threefold concentration gradient for a total of 10 concentration points in duplicate.

2.2.1 Tests of Compounds for inhibiting HPK1 kinase activity *in vitro*

Before the experiment, 50 μM DTT and the enzymatic reaction system buffer were prepared.

The compound dilution was transferred into 384-well plate by using a pipette, afterwards, the 384-well plate was sealed and centrifuged at 1000 g for 1 min. 2-fold concentration of HPK1 solution was prepared in the kinase buffer, and then 2.5 μL of the prepared 2-fold concentration of HPK1 solution was added into the 384 wells, centrifuged at 1000 g for 30 s, and incubated for 10 min at room temperature. A mixture of 2-fold concentration of MBP and ATP was prepare in the kinase buffer, and 2.5 μL of prepared mixture of 2-fold concentration of MBP and ATP was added into the above reaction system for the reaction, centrifuged at 1000 g for 30 s, and then incubated at room temperature for 1 h. 5 μL of ADP-Glo reagent was added into the reaction system, and incubated at room temperature for 40 min. 10 μL of kinase detection reagent was added and incubated at room temperature for 40 min. Then the luminescence signals were read on an Envision 2104 plate reader, and the inhibition rate was calculated according to the following formula:

$$\%\ \text{inhibition} = 100 - (\text{Signal}_{\text{cmpd}} - \text{Signal}_{\text{Ave_PC}}) / (\text{Signal}_{\text{Ave_VC}} - \text{Signal}_{\text{Ave_PC}}) \times 100$$

In the above formula, cmpd refers to the tested compound, PC refers to the positive control, and VC refers to the negative control.

2.2.2 Tests of Compounds for inhibiting GCK, TNIK and PDK1 kinase activity *in vitro*

The experimental procedure was identical to that for testing inhibition on HPK1 kinase activity, except that the positive control used for HPK1 assay was Sunitinib, and the positive control used for GCK, TNIK, and PDK1 assays was Staurosporine.

Table 4. Inhibiting ability of DD02001A_cis and DD02001A_trans on the above four kinase activities

| Compound | Activity/$IC_{50}$, nM | | | |
|---|---|---|---|---|
| | HPK1 | GCK | TNIK | PDK1 |
| DD02001A cis | <10 | >10 | >10 | >7000 |
| DD02001A trans | <10 | >10 | >10 | >10000 |
| Sunitinib | <10 | - | - | - |
| Staurosporine | - | 0.63 | 0.422 | 1.99 |

As shown in Table 4, DD02001A_trans exhibited efficient inhibition on HPK1 kinase activity.

2.2.3 Tests of Compounds for inhibiting other kinase activity *in vitro*

Table 5. Reagents required for the experiment and sources thereof

| Material and reagent | Supplier | **Cat#** | Material and reagent | Supplier | **Cat#** |
|---|---|---|---|---|---|
| HTRF KinEASE-TK kit | Cisbio | 62TK0PEC | AXL | Signalchem | A34-11H-10 |
| ADP-Glo Kinase Assay | Promega | V9102 | c-Kit | Carna | 08-156 |
| MAP4K1 (HPK1) | Signalchem | M23-10G-05 | c-Met | Carna | 08-151 |

(continued)

| Material and reagent | Supplier | **Cat#** | Material and reagent | Supplier | **Cat#** |
|---|---|---|---|---|---|
| GLK/MAP4K3 | Invitrogen | PV6351 | CSF 1R | Invitrogen | 4448892 |
| MAP4K4 (HGK) | Carna | 07-137 | EGFR | Signalchem | E10-112G-10 |
| MAP4K5 (KHS) | Signalchem | M27-10G-05 | FGFR1 | Invitrogen | PV3146 |
| MAP4K6 (MINK1) | Signalchem | M53-11G-10 | FGFR2 | Invitrogen | PV3368 |
| ZAP70 | Carna | 08-177 | Her2 | Invitrogen | PV3366 |
| LCK | Signalchem | L03-10G-10 | IGF1R | Signalchem | I02-11H-10 |
| JNK1 | Carna | 04-163 | ROS1 | Signalchem | R14-11G-10 |
| CK1 gamma 1, Active | Signalchem | C64-10G-10 | VEGFR1 (FLT1) | Invitrogen | PV3 666 |
| Aurora B | Carna | 05-102 | VEGFR2 | Signalchem | K01-11G-10 |
| AKT1 | Signalchem | A16-10G-10 | JAK1 | Invitrogen | PV4774 |
| MNK1 | Carna | 02-145 | Ret WT | Carna | 08-159 |
| MNK2 | Carna | 02-146 | CDk2/Cyclin E1 | Carna | 04-165 |
| TAK 1-TAB 1 | Signalchem | M15-13G | CDK9/CycT1 | Carna | 04-110 |
| MAPK14 (p38 $\alpha$) | Signalchem | M39-10BG | CDK4/CyclinD3 | Carna | 04-105 |
| SYK | Signalchem | S52-10G-10 | ERK2 | Signalchem | M28-10G-10 |
| LYN (LYN B) | Signalchem | L13-10G-10 | LRRK2 | Invitrogen | PR8604B |
| ATR | Eurofins | 14-953 | CDK7 | Invitrogen | PV3868 |
| PAK4 | Carna | 07-126 | CDK12 | Biotrus | BP1642/1648/691 |
| TBK1 | Signalchem | T02-10G-10 | BRAF | Invitrogen | PV3848 |
| IKK-BETA | Signalchem | I03-18G-10 | PI3K-alpha | Invitrogen | PV4788 |
| ERK1 | Signalchem | M29-10G-10 | PKC-alpha | Signalchem | P61-18G |

The inhibition rate of compound DD02001A_trans on 48 kinases, including HPK1, at a concentration of 10 $\mu$M (in duplicate wells) was tested using an experimental procedure similar to that described above for testing inhibition on HPK1 kinase activity.

Table 6. Inhibition rate of DD02001A_trans on immune-associated protein kinase at 10 $\mu$M

| No. | Name of protein kinase | Inhibition rate (%) |
|---|---|---|
| 1 | JAK1 | 97.278 |
| 2 | CSF1R | 101.965 |
| 3 | SYK | 85.074 |
| 4 | LCK | 87.600 |
| 5 | LYNB | 82.948 |
| 6 | PI3K$\alpha$ | 79.095 |

DD02001A_trans did not exhibit significant inhibiting effects (inhibition rate <40%) on the majority of kinases, such as AKT1, HER2, EGFR, ERK1/2, ZAP70, IGF1R, ATR, MNK1/2, ROS1, CDK4, CDK7, CDK12, p38-alpha, IKK-beta, and CK1gamma1, etc., at a concentration of 10 $\mu$M, while exhibited highly selective inhibition on the immune-related protein kinases shown in Table 6, indicating that Compound DD02001A_trans possesses excellent kinase inhibition selectivity.

2.2.4 Determination of pharmacokinetic parameters of compounds

The compounds to be tested were administered intravenously (2 mg/kg) and by gavage (10 mg/kg) to three male SD rats, respectively, and the blood concentration of the compounds to be tested in plasma was quantified by liquid chromatography tandem mass spectrometry (LC-MS/MS), and the pharmacokinetic parameters were calculated to investigate the pharmacokinetic profiles of the compounds to be tested in the SD male rats. For the compounds to be tested, blood was taken at the time points of 0.25 h, 0.5 h, 1 h, 2 h, 4 h, 6 h, 8 h and 24 h after oral administration, and blood was taken at the time points of 0.083 h, 0.25 h, 0.5 h, 1 h, 2 h, 4 h, 6 h, 8 h and 24 h after intravenous administration. Subsequently, 50 μL of plasma sample was transferred to a 96-well plate and 250 μLACN (internal standard containing 260 ng/ml toluenesulfonylbutyramide) to precipitate proteins, and centrifuged at 4°C and 4000 rpm for 20 min, 150 μL of the supernatant was transferred to a new 96-well plate and mixed with 150 μL of 0.1% FA/water, and then 5 μL of the resulting solution was loaded on LC-MS/MS for analysis.

Table 7. Absolute oral bioavailability of DD02001A_trans

| Compound | Absolute oral bioavailability % |
|---|---|
| DD02001A trans | 61.3 |

2.2.5 Acute toxicity assay of DD02001A_trans

Mice were given DD02001A_trans (2000, 1500, 1000, and 500 mg/kg) by gavage in a single dose, respectively, and the responses of the mice after administration and their deaths within two weeks were recorded.

2.2.5.1 Animals: Kunming breed mice (purchased from Guangdong Provincial Laboratory Animal Centre, License No.: SCXK(GD)2018-0002), 10 mice, half male and half female, weighing 18-22 g. The mice were kept for three days to adapt to the environment. The mice were kept in cages (indoors) at a room temperature of 23 ± 1 °C, relative humidity of 40%-60%, and had *ad libitum* access to water and food.

2.5.1.2 Methods: The mice were fasted for 10 h before the experiment, and given each concentration of the tested drug (0.1 ml/10 g) by gavage at one time respectively, and were observed for 14 consecutive days, and symptoms of poisoning, the time of onset and duration of poisoning, and the death of the animals after the administration of the drug were recorded in detail. Necropsy was carried out on the dead animals in time to record the lesions. If obvious changes are visible to naked eyes, pathological section should be made.

According to the results of acute toxicity experiments, the maximum tolerated dosage of DD02001A_trans in mice by gavage is in the range of 1000-1500 mg/kg.

## Example 2. Detection of inhibition on IFN-β signalling pathway by HPK1

Interferon beta (IFN-β) is a protein encoded by the IFNB1 gene. Natural or recombinant IFN-β proteins have antiviral, antibacterial, and anticancer activities. To explore the intrinsic connection between HPK1 and IFN-β signalling pathway, HPK1 and IFN-β Luciferase Reporter plasmid were overexpressed in HEK293 cells. Cells were stimulated with Sendai virus (SeV) 24 hours after transfection and collected after 12 hours for the examination of IFN-β Luciferase activity. Results showed that overexpression of the HPK1(1-274) kinase domain significantly inhibited SeV-induced IFN-β transcription (Figure 1). Further, the expression of different amounts of HPK1 plasmid inhibited SeV-induced IFN-β transcription in a dose-dependent manner (Figure 2).

ISRE (IFN-stimulated response elements) can be bound by phosphorylated IRF3, thereby mediating IFN-β transcription, which is another important indicator of the antiviral pathway. Different doses of HPK1 and ISRE Luciferase Reporter plasmids were overexpressed in HEK293 cells, and the cells were stimulated with Sendai virus (SeV) 24 hours after transfection, and collected after 12 hours for examination of ISRE Luciferase activity. Results showed that HPK1 can inhibit SeV-induced ISRE transcription in a dose-dependent manner (Figure 3).

## Example 3. Assay *of in vitro* enzyme activity of the compounds of the present invention

The inhibiting activity of the compounds of the present invention (C1-C6) against HPK1 kinase was tested by the present inventors using ADP-GloTM method.

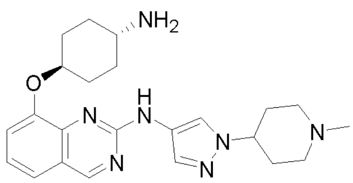

C1

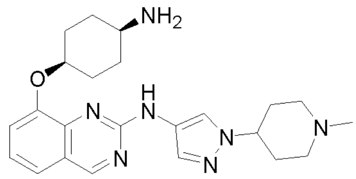

C2

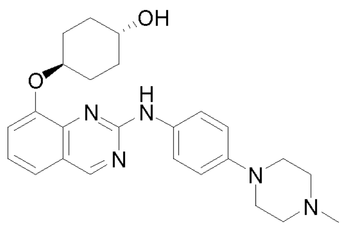

C3

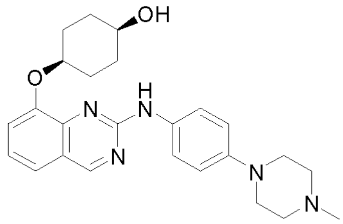

C4

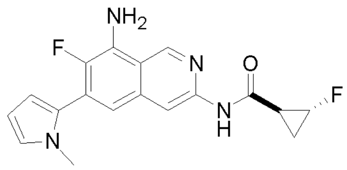

C5

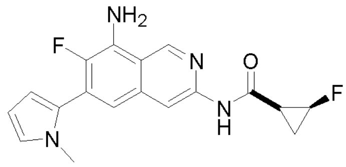

C6

ADP-Glo™ Kinase Assay is a luminescent ADP assay that provides a versatile, homogeneous, high-throughput screening method to measure kinase activity by quantifying the amount of ADP produced during the kinase reaction. The assay was performed in two steps; first, after the kinase reaction, an equal volume of ADP-Glo™ Reagent was added to quench the kinase reaction and consume the remaining ATP. Second, the Kinase Assay Reagent was added to concurrently convert ADP to ATP and allow measurement of newly synthesized ATP using the luciferase/luciferin reaction. The light produced was measured using a photometer. Luminescence can be correlated with ADP concentration by using an ATP to ADP conversion curve. All six small molecule inhibitors evaluated in this study exhibited good inhibiting activity against recombinant HPK1 kinase *in vitro,* with $IC_{50}$ below 0.1 μM (Table 1). Meanwhile, better selectivity was shown on kinases with closer homology such as GCK/TNIK/PDK1. These properties ensured that the compounds can specifically inhibit HPK1 enzymatic activity in cell or animal experiments.

**Table 1. Inhibiting activity of small molecule inhibitors on recombinant proteins *in vitro*[a]**

| Compound | HPK1 | GCK | TNIK | PDK1 |
|---|---|---|---|---|
| C1 | A | B | C | C |
| C2 | A | B | C | C |
| C3 | B | B | B | D |
| C4 | B | B | B | D |
| C5 | A | A | B | D |
| C6 | A | B | B | D |

[a] Activity level: A, $IC_{50} \leq 0.01$ μM; B, 0.01 μM $< IC_{50} \leq 0.1$ μM; C, 0.1μM$< IC_{50} \leq 1$ μM; D, $IC_{50} > 1$ μM

**Example 4. Restoration of HPK1-inhibited IFN-β expression by the Compounds of the present invention**

In view of the findings that HPK1 inhibits the antiviral IFN-β signalling pathway, the inventors explored whether a small molecule inhibitor of HPK1 can restore HPK1-inhibited **IFN-β** expression. As in the experimental procedure described above, HPK1 and **IFN-β** Luciferase Reporter plasmid were overexpressed in HEK293 cells, and 0.5 μM of

the compound was added 18 hours after transfection, the cells were stimulated with SeV after 24 hours, and the cells were collected after 12 hours for the examination of **IFN-β** Luciferase activity. Results showed that Compound C2\C3\C4 had better activity, followed by compounds C1 and C6 (Figure 4). Further, Compound C2 exhibited a dosage-dependent restoration of HPK1-inhibited IFN-β expression (Figure 5).

**Example 5. Oral maximum tolerated doses of the compounds of the invention**

The inventors tested representative compounds for oral maximum tolerated dose in mice. Results showed that the oral maximum tolerated dose for a single dose of Compound C1 was 337.5 mg/kg (Table 2) and the oral maximum tolerated dose for Compound C3 was 225 mg/kg (Table 3).

**Table 2. Oral acute toxicity test results of small molecule compound C1 in mice**

| Dose (mg/kg, P.O.) | Death | |
|---|---|---|
| | Male mice | Female mice |
| 225 | 0/5 | 0/5 |
| 337.5 | 0/5 | 0/5 |
| 506.3 | 2/5 | 1/5 |
| 759.4 | 4/5 | 4/5 |
| 1139.1 | 5/5 | 5/5 |

**Table 3. Oral acute toxicity test results of small molecule compound C3 in mice**

| Dose (mg/kg, P.O.) | Death | |
|---|---|---|
| | Male mice | Female mice |
| 225 | 0/5 | 0/5 |
| 337.5 | 1/5 | 2/5 |
| 506.3 | 3/5 | 2/5 |
| 759.4 | 3/5 | 5/5 |
| 1139.1 | 5/5 | 5/5 |

**Example 6. The compounds of the present invention are effective in treating the mouse MHV model**

To evaluate the antiviral efficacy of HPK1 small molecule inhibitors in an animal model, the inventors used a C57 mouse model infected with Mouse Hepatitis Virus (MHV), which is a murine coronavirus readily infecting the liver and the nervous system. 8-week-old female C57 mice (three in each group), were injected with a 4 * 104 pfu virus via hepatic *in situ* injection. After the inoculation with the virus, the mice were treated through the oral administration of drugs (10 mg/kg, BID). Three days after virus infection, the viral amount in liver tissues was detected by qPCR. Compound, C1, exhibited relatively significant antiviral efficacy, and effectively reduced the viral titre in the liver of mice (Figure 6). In the same virus-infected C57 mouse model, the oral administration of different doses of C1 (5 mg/kg, 20 mg/kg) twice a day showed a dose-dependent inhibiting effect on the viral titre in the liver (Figure 7) and serum Alanine aminotransferase (Figure 8) of the mice.

**Example 7. The compounds of the present invention are effective in treating feline infectious peritonitis**

Feline Infectious Peritonitis (FIP) is a fatal abnormal immune response in cats caused by a mutation of the feline coronavirus carried by cats. FIP is still an incurable disease with an indeterminate time to death after onset (but rarely more than a year). Although the name of the disease is "peritonitis", FIP is actually a multi-systemic inflammatory disease, and not all affected cats will necessarily show signs of peritonitis. Symptoms in affected cats can usually be divided into two categories: wet FIP and dry FIP, in which wet FIP accounts for about 70% of all cases, exhibiting fluid accumulation in the abdominal and thoracic cavities and abnormal bulging; and dry FIP cats will have different symptoms depending on the type of organ attacked by the virus. As of 2011, FIP has ranked as the number one fatal infectious disease in pet cats in developed countries.

A previous study (Ishida et al., Journal of Feline Medicine and Surgery, 2004) found that recombinant feline interferon (rFeIFN) had therapeutic effects on Feline Infectious Peritonitis. The inventor found that HPK1 inhibitors can activate the IFN-β signalling pathway. Therefore, the inventors hypothesized that HPK1 inhibitors might have therapeutic effects on Feline Infectious Peritonitis. To validate this hypothesis, the inventors conducted an animal clinical trial. Three animals were included in the experiment, all of them suffered from Feline Infectious Peritonitis, aged between 5-12 months, one was female and the other two were male (Table 4).

**Table 4. Animals enrolled in Feline Infectious Peritonitis experiment treated by HPK1 inhibitor antiviral treatment**

| Animal No. in the experiment | Sex | Age | Type of Feline Infectious Peritonitis (dry or wet? ) |
|---|---|---|---|
| 1 | Female | 6 months | Wet |
| 2 | Male | 5 months | Wet |
| 3 | Male | 12 months | Wet |

After being treated for 20 days with Compound C1 solution via injection (twice a day at a dose of 1.5 mg/kg), physiological indices of the experimental animals comprehensively improved (Table 5): (1) increase in appetite and thus body weight; (2) improvement in the mental status from depressed to active; (3) disappearance of ascites; (4) increase in the ratio of ALB/GLB; and (5) significant decrease in Feline serum amyloid A.

**Table 5. Changes in physiological indices before and after treating animals with Feline Infectious Peritonitis using small molecule Compound C1.**

| Physiological indices | Animal No. | Before treatment | After treatment |
|---|---|---|---|
| body weight (kg) | 1 | 1.5 | 2.15 |
| | 2 | 2.31 | 2.81 |
| | 3 | 2.9 | 3.2 |
| ALB/GLB | 1 | 0.5 | 0.7 |
| | 2 | 0.35 | 0.47 |
| | 3 | 0.4 | 0.5 |
| Feline serum amyloid A (fSAA, mg/L) | 1 | NA* | NA |
| | 2 | 70.47 | 15.14 |
| | 3 | 104.88 | 17.97 |
| Fluid in the abdominal and thoracic cavity | 1 | Significant ascites | disappearance of ascites |
| | 2 | Significant thoracic fluid, small amount of ascites | disappearance of ascites |
| | 3 | Significant thoracic fluid, dyspnea | Less thoracic fluid, improved dyspnea |
| *NA, not available | | | |

Therefore, a conclusion can be obtained that HPK1 small molecule inhibitors can effectively treat Feline Infectious Peritonitis caused by coronaviruses.

Conclusion:

The inventors have found that HPK1 can significantly inhibit the IFN-β signalling pathway. Based on previous studies, HPK1 also significantly inhibits the IFN-γ signalling pathway. The use of HPK1 inhibitors allows for anti-infective treatment from both IFN-β mediated antiviral effects and IFN-γ mediated T cell activation. Therefore, HPK1 inhibitors are therapeutically effective for human or animal diseases caused by all viruses in which IFN-β/IFN-γ has an effect, including but not limited to: Hepatitis B virus, Measles Virus, Sindbis Virus, West Nile Virus, Dengue Virus, Herpes Simplex Virus, Human Cytomegalovirus HCMV, Ebola Virus, HCV, Influenza A Virus, SARS-CoV, Zika Virus, HIV, Feline Infectious

Peritonitis Virus, Mouse Hepatitis Virus, Canine Coronavirus, Feline Calicivirus, Feline Leukemia Virus, Feline Immunodeficiency Virus, Feline Panleukopenia Virus, Avian Infectious Bronchitis Virus, Transmissible Gastroenteritis Virus, Porcine Epidemic Diarrhea Virus, Porcine Hemagglutinating Encephalomyelitis Virus, Bovine Coronavirus, and the like. HPK1 inhibitors are expected to be used as a novel broad-spectrum antiviral therapy for treating diseases induced by the above virus.

All documents mentioned herein are cited as references in the present application as if each document is cited individually as a reference. It is further to be understood that after reading the foregoing teachings of the present invention, a skilled person can make various alterations or modifications to the present invention, and such equivalent forms will likewise fall within the scope of the claims appended to the present application.

## Claims

1. Use of a HPK1 inhibitor in the preparation of a drug for treating or preventing an interferon-related disease, or for boosting immunity.

2. The use of claim 1, wherein said interferon-related disease is a viral infection.

3. The use of claim 1, wherein the HPK1 inhibitor is a compound shown in Formula I or a pharmaceutically acceptable salt, solvate, stereoisomer, tautomer, prodrug, metabolite or derivative thereof,

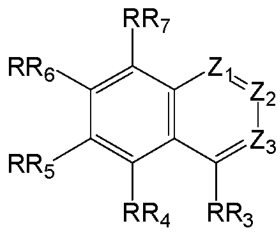

I

wherein $Z_1$ is selected from N or C-$RR_1$;
$Z_2$ and $Z_3$ are each selected from N or C-$RR_2$, wherein $Z_2$ and $Z_3$ are not identical;
$RR_1$ is selected from a hydrogen, halogen, cyano, hydroxy, substituted or unsubstituted $C_{1-6}$ alkyl, substituted or unsubstituted $C_{3-6}$ cycloalkyl, substituted or unsubstituted $C_{1-6}$ alkoxy, substituted or unsubstituted amino or substituted or unsubstituted acylamino;
$RR_2$ is selected from a substituted or unsubstituted hydroxyl, substituted or unsubstituted amino, or substituted or unsubstituted sulfhydryl;
$RR_3$ is selected from H, hydroxy, halogen, cyano, substituted or unsubstituted $C_{1-6}$ alkyl, substituted or unsubstituted $C_{3-8}$ cycloalkyl, substituted or unsubstituted $C_{1-6}$ alkoxy, substituted or unsubstituted amino, or substituted or unsubstituted acylamino;
$RR_4$, $RR_5$, $RR_6$, $RR_7$ are each independently selected from H, hydroxy, halogen, cyano, substituted or unsubstituted $C_{1-6}$ alkyl, substituted or unsubstituted $C_{3-8}$ cycloalkyl, substituted or unsubstituted $C_{1-6}$ alkoxy, substituted or unsubstituted amino, substituted or unsubstituted acylamino, substituted or unsubstituted $C_{5-10}$ heteroaryl, or $RR_8$-$Z_4$-, respectively;
$RR_8$ is selected from a substituted or unsubstituted alkyl, substituted or unsubstituted cycloalkyl, substituted or unsubstituted heterocyclyl, or substituted or unsubstituted hydroxy;
$Z_4$ is selected from -O-, -NH-, -S-, -SO-, -SO2-, carbonyl, carbonylamino or aminocarbonyl.

4. The use of claim 3, wherein the compound shown in Formula I is a compound shown in Formula 1-1:

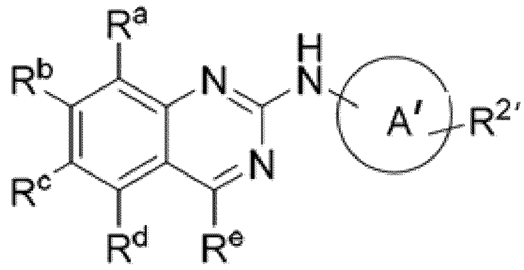

I-1

wherein one of $R^a$, $R^b$, $R^c$, $R^d$ is $R^{1'}$-X'- and the rest are each independently selected from H, hydroxyl, halogen, cyano, substituted or unsubstituted $C_{1-6}$ alkyl, substituted or unsubstituted $C_{3-8}$ cycloalkyl, or substituted or unsubstituted $C_{1-6}$ alkoxy;

$R^e$ is selected from H, hydroxy, halogen, cyano, substituted or unsubstituted $C_{1-6}$ alkyl, substituted or unsubstituted $C_{3-8}$ cycloalkyl, or substituted or unsubstituted $C_{1-6}$ alkoxy;

X' is selected from -O-, -NH-, -S-, -SO-, -SO2-, carbonyl, carbonylamino or aminocarbonyl;

$R^{1'}$ is a substituted or unsubstituted cycloalkyl, substituted or unsubstituted heterocycloalkyl or $R^{14'}$-O-$(CH_2)_m$-, wherein m is 0, 1, 2, 3, 4 or 5, and $R^{14'}$ is a substituted or unsubstituted $C_{1-6}$ alkyl or substituted or unsubstituted $C_{3-8}$ cycloalkyl;

ring A' is a substituted or unsubstituted aryl (preferably a substituted or unsubstituted phenyl) or substituted or unsubstituted 5-6-membered heteroaryl, wherein said heteroaryl has 1-4 heteroatoms selected from O, S or N;

$R^{2'}$ is selected from following substituents:

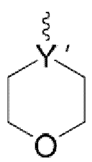 , 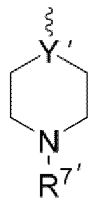 , 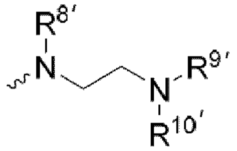 , 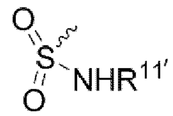 and 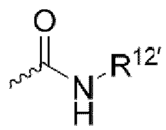 ;

wherein Y' is C or N, $R^{7'}$ is H, substituted or unsubstituted $C_{1-6}$ alkyl or substituted or unsubstituted $C_{3-8}$ cycloalkyl, $R^{8'}$ is H, substituted or unsubstituted $C_{1-6}$ alkyl or substituted or unsubstituted $C_{3-8}$ cycloalkyl, $R^{9'}$ and $R^{10'}$ are independently H, substituted or unsubstituted $C_{1-6}$ alkyl or substituted or unsubstituted $C_{3-8}$ cycloalkyl, $R^{11'}$ is H, substituted or unsubstituted amino, substituted or unsubstituted $C_{1-6}$ alkyl or substituted or unsubstituted $C_{3-8}$ cycloalkyl, and $R^{12'}$ is a substituted or unsubstituted 4-6-membered heterocyclyl.

**5.** The use of claim 4, wherein the compound shown in Formula I-1 is shown in Formula I-1-1,

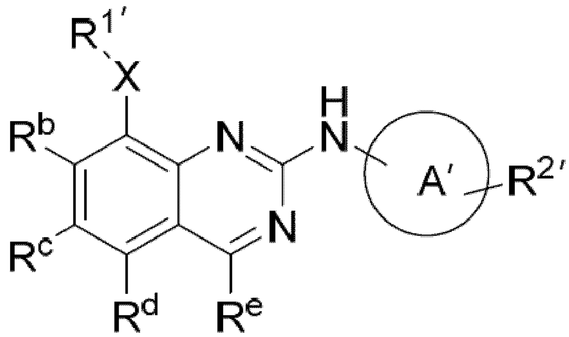

I-1-1

wherein $R^b$, $R^c$, $R^d$ and $R^e$ are each independently selected from H, hydroxyl, halogen, cyano, substituted or unsubstituted $C_{1-6}$ alkyl, substituted or unsubstituted $C_{3-8}$ cycloalkyl, or substituted or unsubstituted $C_{1-6}$ alkoxy;

X' is selected from -O-, -NH-, -S-, -SO-, $-SO_2-$, carbonyl, carbonylamino or aminocarbonyl;

$R^{1'}$ is a substituted or unsubstituted cycloalkyl, substituted or unsubstituted heterocycloalkyl or $R^{14'}$-O-$(CH_2)_m$-, wherein m is 0, 1, 2, 3, 4 or 5, and $R^{14'}$ is a substituted or unsubstituted $C_{1-6}$ alkyl or substituted or unsubstituted $C_{3-8}$ cycloalkyl;

ring A' is a substituted or unsubstituted aryl (preferably a substituted or unsubstituted phenyl) or substituted or unsubstituted 5-6-membered heteroaryl, wherein said heteroaryl has 1-4 heteroatoms selected from O, S or N; $R^{2'}$ is selected from following substituents:

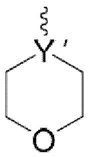 , 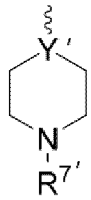 , 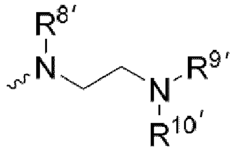 , 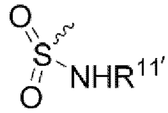 and 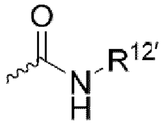 ;

wherein Y' is C or N, $R^{7'}$ is H, substituted or unsubstituted $C_{1-6}$ alkyl or substituted or unsubstituted $C_{3-8}$ cycloalkyl, $R^{8'}$ is H, substituted or unsubstituted $C_{1-6}$ alkyl or substituted or unsubstituted $C_{3-8}$ cycloalkyl, $R^{9'}$ and $R^{10'}$ are independently H, substituted or unsubstituted $C_{1-6}$ alkyl or substituted or unsubstituted $C_{3-8}$ cycloalkyl, $R^{11'}$ is H, substituted or unsubstituted amino, substituted or unsubstituted $C_{1-6}$ alkyl or substituted or unsubstituted $C_{3-8}$ cycloalkyl, and $R^{12'}$ is a substituted or unsubstituted 4-6-membered heterocyclyl.

**6.** The use of claim 5, wherein the compound shown in Formula I-1 is shown in Formula I-1-1-1 or I-1-1-2,

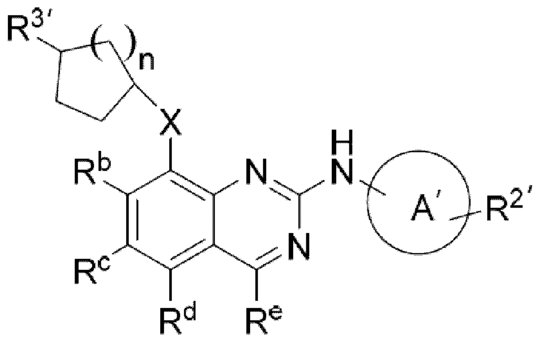

I-1-1-1

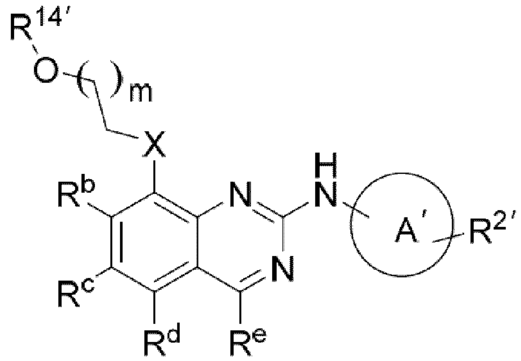

I-1-1-2

wherein $R^b$, $R^c$, $R^d$ and $R^e$ are each independently selected from H, hydroxyl, halogen, cyano, substituted or unsubstituted $C_{1-6}$ alkyl, substituted or unsubstituted $C_{3-8}$ cycloalkyl, or substituted or unsubstituted $C_{1-6}$ alkoxy;
X is selected from -O-, -NH-, -S-, -SO-, $-SO_2-$, carbonyl, carbonylamino or aminocarbonyl;
n is 1, 2, 3 or 4;
m is 0, 1, 2, 3, 4 or 5,
$R^{14'}$ is a substituted or unsubstituted $C_{1-6}$ alkyl or substituted or unsubstituted $C_{3-8}$ cycloalkyl;
ring A' is a substituted or unsubstituted aryl (preferably a substituted or unsubstituted phenyl) or substituted or unsubstituted 5-6-membered heteroaryl, wherein said heteroaryl has 1-4 heteroatoms selected from O, S or N;
$R^{2'}$ is selected from following substituents:

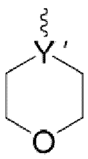 , 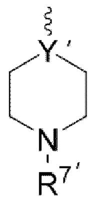 , 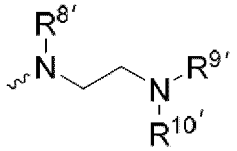 , 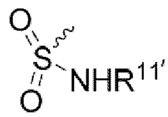 and 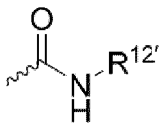 ;

wherein Y' is C or N, $R^{7'}$ is H, substituted or unsubstituted $C_{1-6}$ alkyl or substituted or unsubstituted $C_{3-8}$ cycloalkyl, $R^{8'}$ is H, substituted or unsubstituted $C_{1-6}$ alkyl or substituted or unsubstituted $C_{3-8}$ cycloalkyl, $R^{9'}$ and $R^{10'}$ are independently H, substituted or unsubstituted $C_{1-6}$ alkyl or substituted or unsubstituted $C_{3-8}$ cycloalkyl, $R^{11'}$ is H, substituted or unsubstituted amino, substituted or unsubstituted $C_{1-6}$ alkyl or substituted or unsubstituted $C_{3-8}$ cycloalkyl, and $R^{12'}$ is a substituted or unsubstituted 4-6-membered heterocyclyl.

**7.** The use of any one of claims 3-6, wherein the compound is selected from the following group:

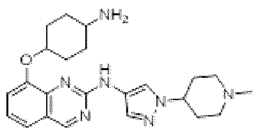
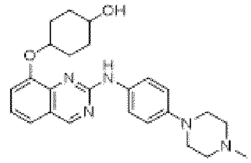
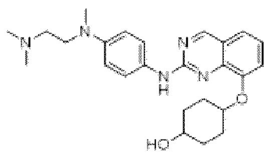
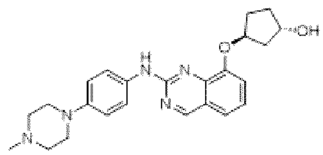
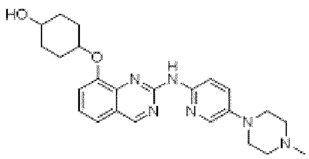
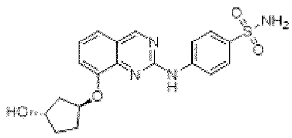
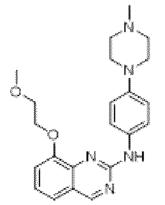
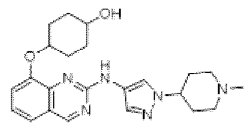
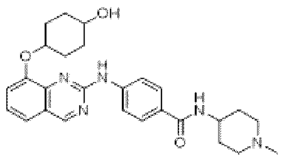
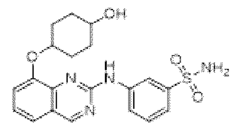
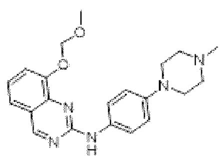
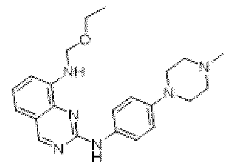
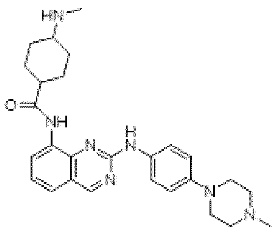
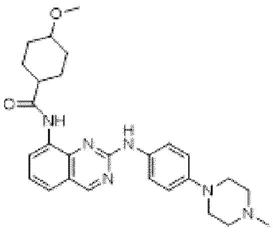
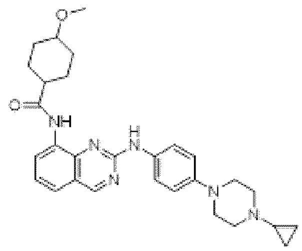
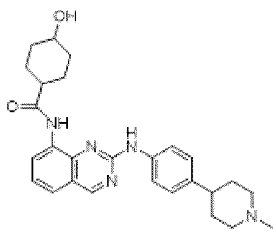
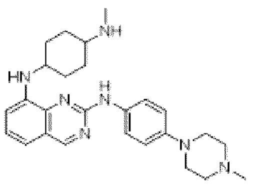
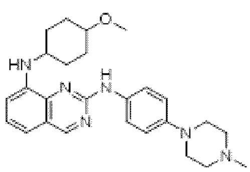
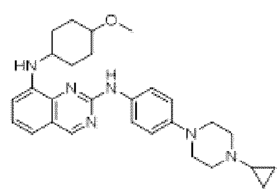
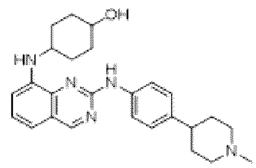
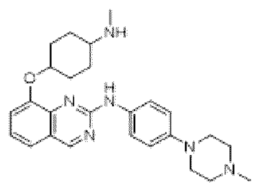
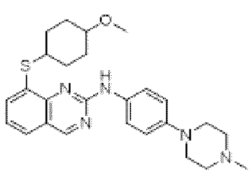
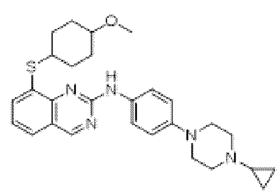
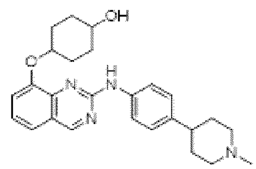
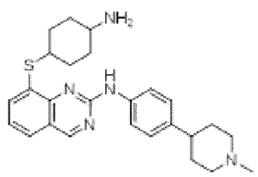
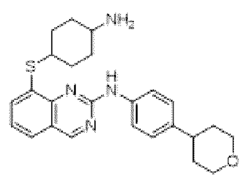
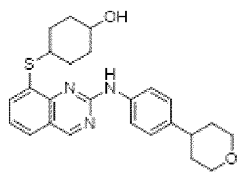
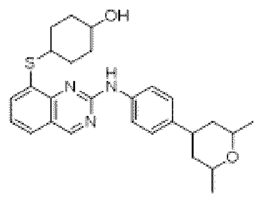

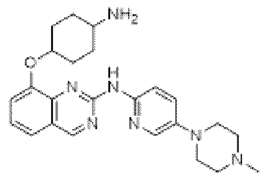
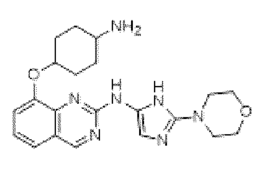
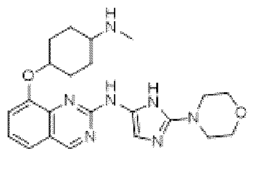
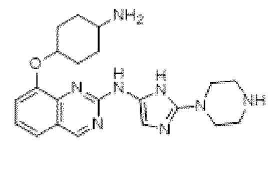

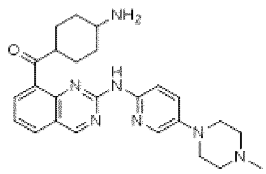
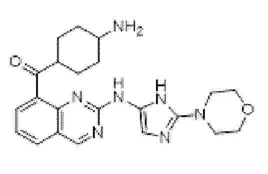
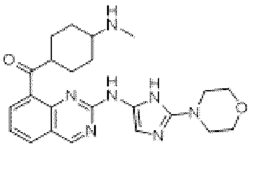
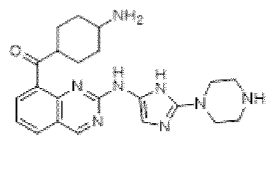

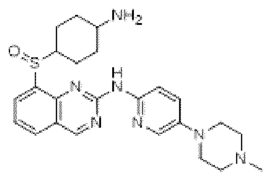
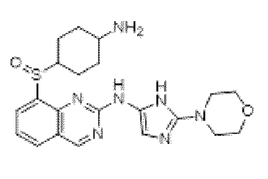
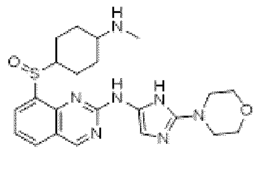
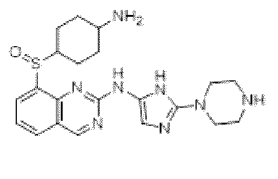

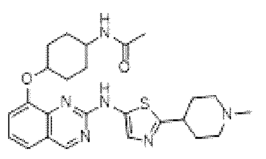
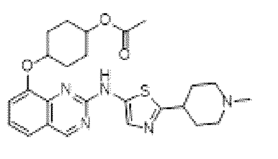
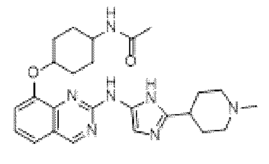
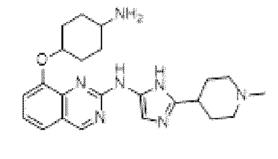

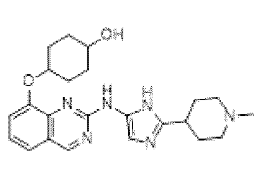
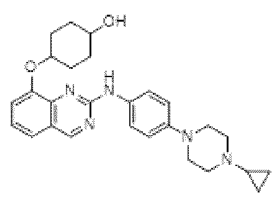
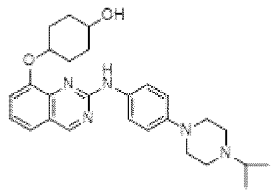
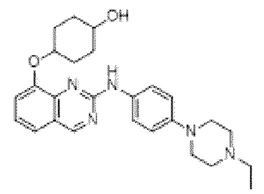

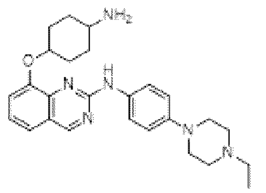
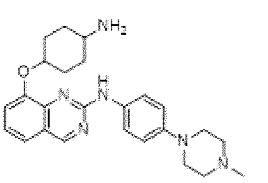
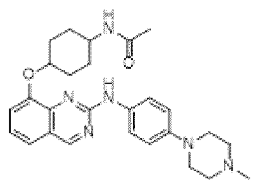
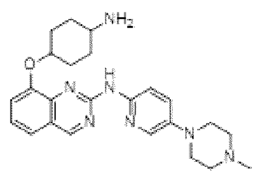

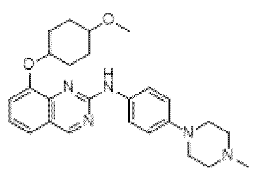
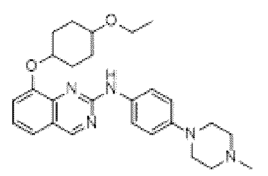
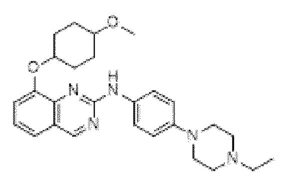
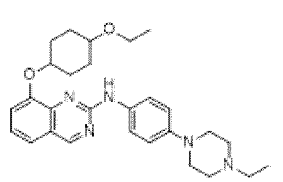

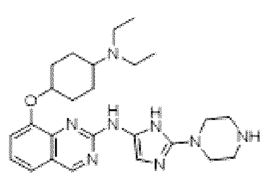
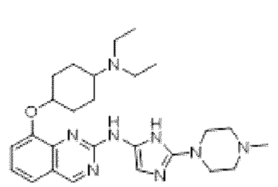
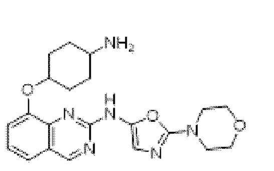
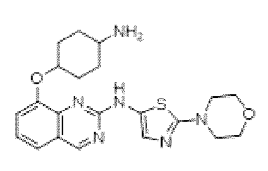

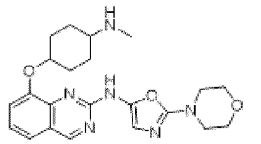 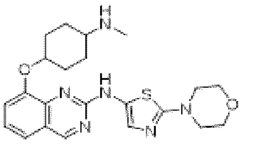 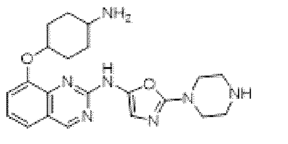 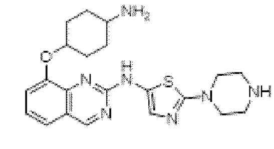

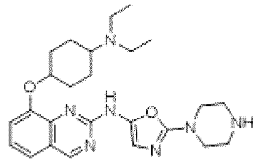 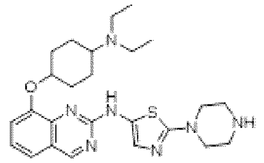 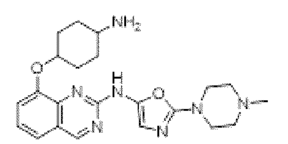 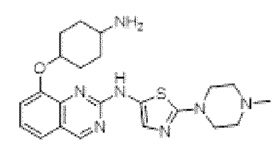

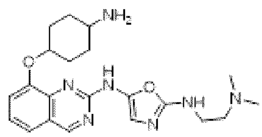 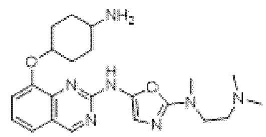 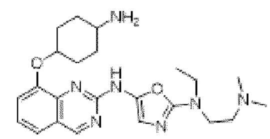 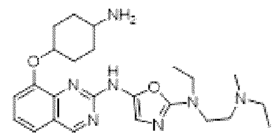

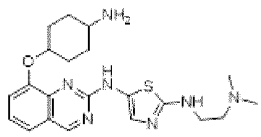 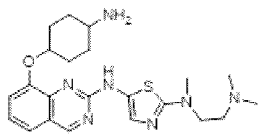 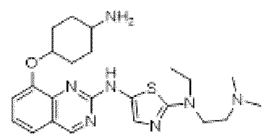 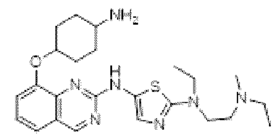

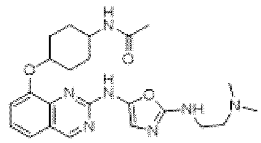 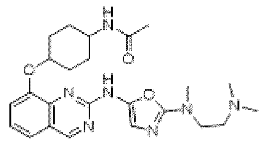 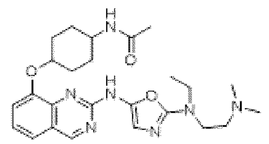 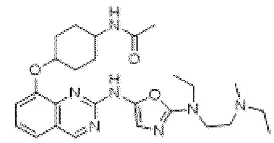

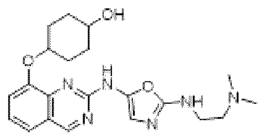 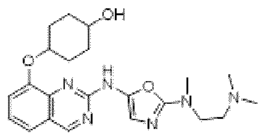 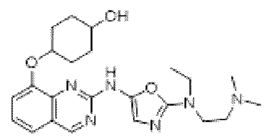 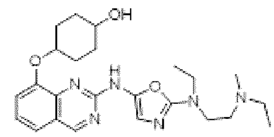

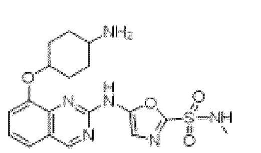 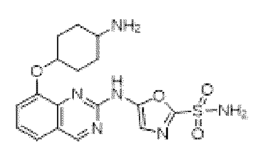 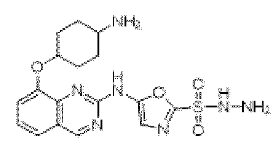 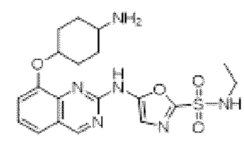

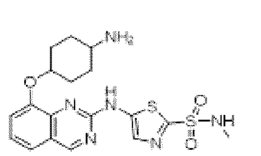 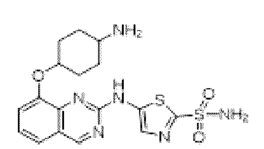 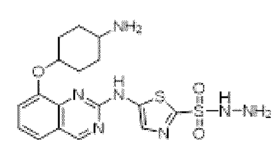 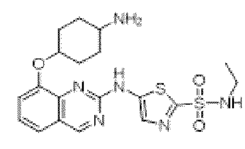

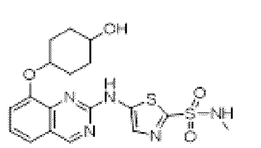 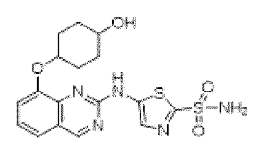 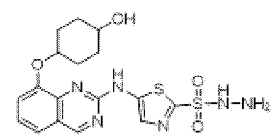 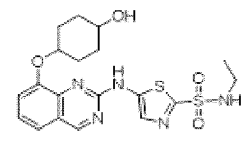

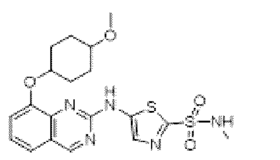
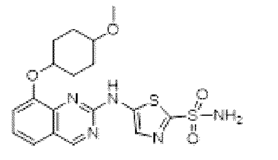
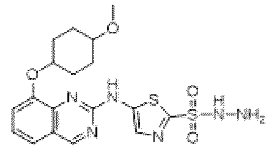
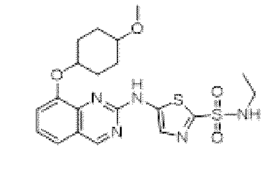
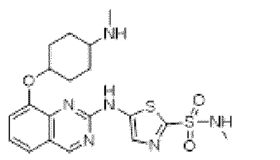
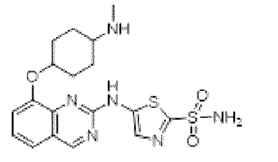
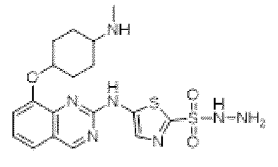
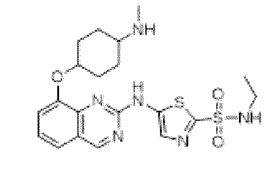
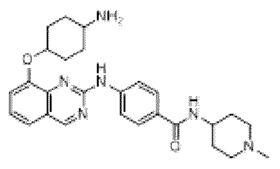
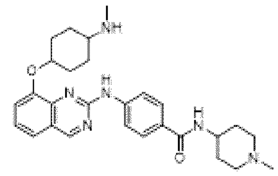
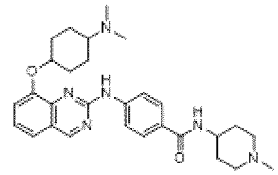
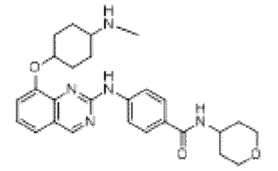
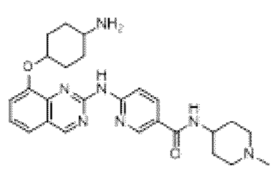
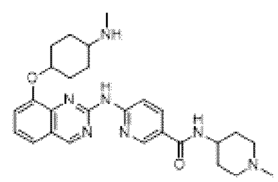
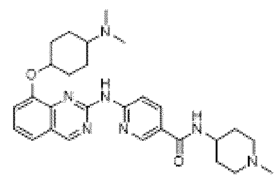
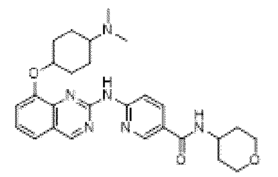
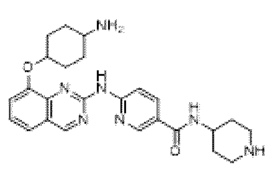
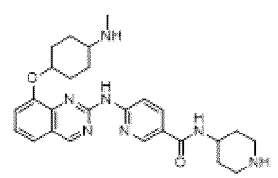
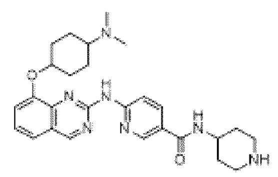
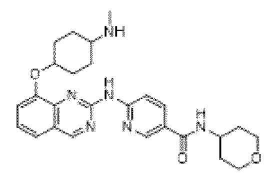
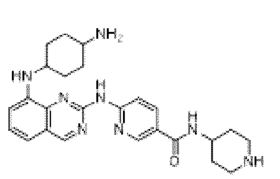
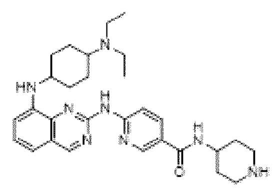
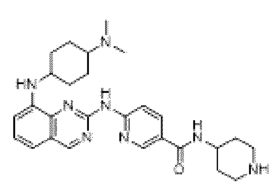
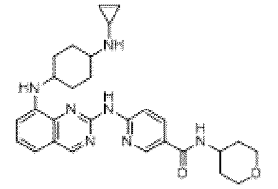
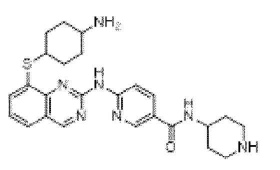
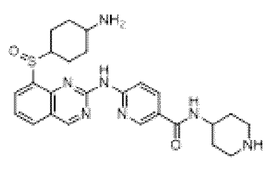
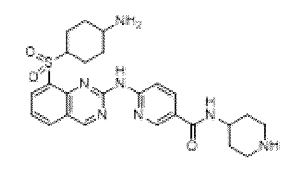
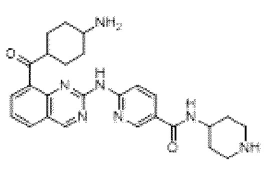
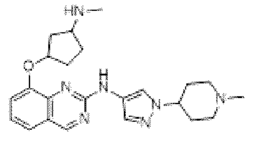
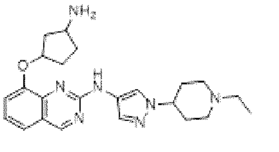
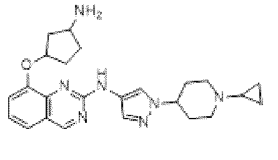
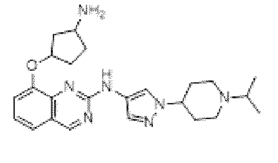

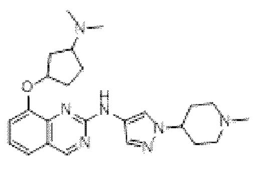
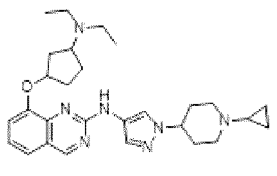
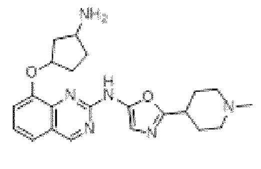
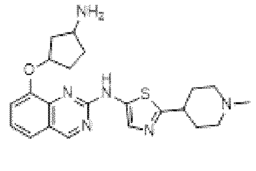

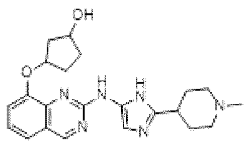
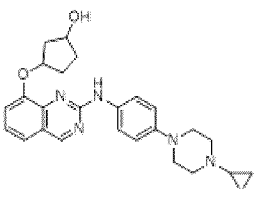
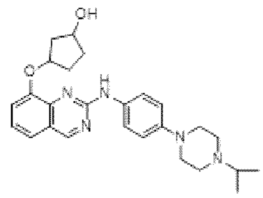
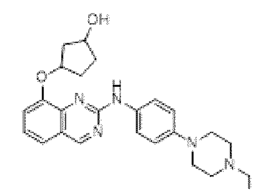

**8.** The use of claim 3, wherein the compound shown in Formula I is a compound shown in Formula I-2:

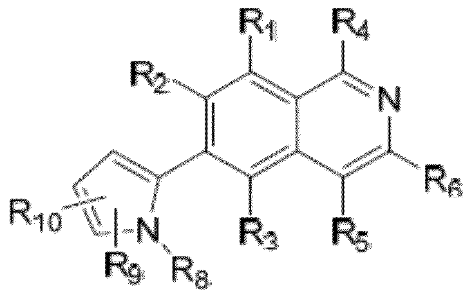

I-2

wherein $R_1$, $R_2$, $R_3$, $R_4$ and $R_5$ are each independently selected from: a hydrogen, halogen, cyano, hydroxy, substituted or unsubstituted $C_{1-6}$ alkyl, substituted or unsubstituted $C_{3-6}$ cycloalkyl, substituted or unsubstituted $C_{1-6}$ alkoxy, substituted or unsubstituted amino, or substituted or unsubstituted acylamino, and none of $R_1$ and $R_2$ is a hydrogen;
$R_6$ is:

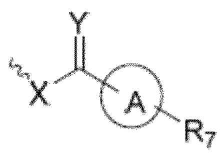

X is O or NH or S, Y is O or NH or S, A ring is a substituted or unsubstituted $C_{3-6}$ cycloalkyl or a substituted or unsubstituted $C_{6-10}$ aryl or a substituted or unsubstituted $C_{5-10}$ heteroaryl, said heteroaryl means an aryl comprising one or more N, O, or S heteroatoms, $R_7$ is a mono- or multi-substitution on various types of rings, including a halogen, cyano, hydroxyl, amino, amido, branched or straight chain $C_{1-6}$ alkyl, $C_{2-6}$ alkenyl, $C_{2-6}$ alkynyl, $C_{3-6}$ heterocyclyl, $C_{6-10}$ aryl, $C_{5-10}$ heteroaryl or -C(O)R', wherein said R' is a $C_{3-6}$ heterocyclyl, $C_{6-10}$ aryl, or $C_{5-10}$ heteroaryl, and said $C_{1-6}$ alkyl, $C_{2-6}$ alkenyl, $C_{2-6}$ alkynyl, $C_{3-6}$ heterocyclyl, $C_{6-10}$ aryl, and $C_{5-10}$ heteroaryl further comprise various types of mono- or multi-substitution including a hydrogen, hydroxyl, halogen, cyano, amino, $-CHF_2$, $-CF_3$, $-CH_3$, $-CH_2CH_2OH$, or $-CONH_2$; alternatively, $R_7$, together with the ring A, forms a substituted or unsubstituted fused ring group, bridged ring group, heterobridged ring group, spiro ring group or hetero spiro ring group;
$R_8$, $R_9$ and $R_{10}$ are each independently selected from:

i. a hydrogen, hydroxyl, halogen, cyano, amino, di($C_{1-6}$ alkyl)amino, mono($C_{1-6}$ alkyl)amino or $C_{1-6}$ alkoxy;
ii. a branched or straight chain $C_{1-6}$ alkyl, $C_{2-6}$ alkenyl, or $C_{2-6}$ alkynyl, and the alkyl, alkenyl and alkynyl further comprise various mono- or multi-substitution, including a hydrogen, hydroxyl, halogen, cyano, amino, $-CHF_2$, $-CF_3$, di($C_{1-6}$ alkyl)amino, mono($C_{1-6}$ alkyl)amino, $C_{3-6}$ cycloalkyl, or $C_{1-6}$ alkoxy;
iii. $C_{3-6}$ cycloalkyl, $C_{3-6}$ heterocycloalkyl, $C_{6-10}$ aryl or $C_{5-10}$ heteroaryl, wherein the cycloalkyl, heterocycloalkyl, aryl or heteroaryl comprises various mono- or multi-substitution, including a hydrogen, $C_{1-6}$ alkyl, hydroxyl, halogen, cyano, amino, $-CHF_2$ or $-CF_3$.

**9.** The use of claim 8, wherein the compound shown in Formula I-2 is shown in Formula I-2-1:

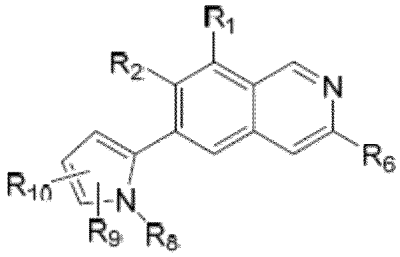

I-2-1

wherein $R_1$, $R_2$, $R_6$, $R_8$, $R_9$, and $R_{10}$ are each defined as in claim 6.

**10.** The use of claim 8, wherein the compound shown in Formula I-2 is shown in Formula I-2-2:

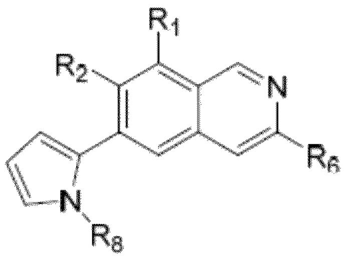

I-2-2

wherein $R_1$, $R_2$, $R_6$ and $R_8$ are each defined as in claim 8.

**11.** The use of claim 8, wherein the compound shown in Formula I-2 is shown in Formula I-2-3:

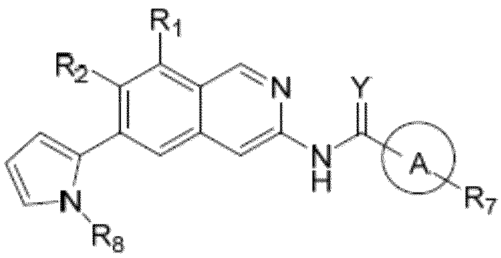

I-2-3

wherein $R_1$, $R_2$, $R_8$ and A are each defined as in claim 8.

**12.** The use of any one of claims 8-11, wherein the compound is selected from:

| No. | Structure | Name |
|---|---|---|
| 1 | | N-(8-amino-7-fluoro-6-(1-methyl-1H-pyrrole-2-yl)isoquinolin-3-yl)-2-fluorocyclopropan-1-carboxamide |
| 2 | | N-(8-amino-6-(1-ethyl-1H-pyrrole-2-yl)-7-fluoroi soquinolin-3-yl)-2-fluorocyclopropan-1-carboxamide |
| 3 | | N-(8-amino-7-fluoro-6-(1-isopropyl-1H-pyrrole-2-yl)isoquinolin-3-yl)-2-fluorocyclopropan-1-carboxamide |

(continued)

| No. | Structure | Name |
|---|---|---|
| 4 | | N-(8-amino-7-fluoro-6-(1-ethenyl-1H-pyrrole-2-yl)isoquinolin-3-yl)-2-fluorocyclopropan-1-carboxamide |
| 5 | | N-(8-amino-7-fluoro-6-(1-methyl-1H-pyrrole-2-yl)isoquinolin-3-yl)-4-morpholinobenzamide |
| 6 | | N-(8-amino-7-fluoro-6-(1-methyl-1H-pyrrole-2-yl)isoquinolin-3-yl)-4-(piperazin-1-yl)benzamide |
| 7 | 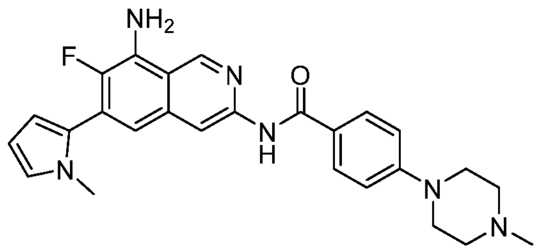 | N-(8-amino-7-fluoro-6-(1-methyl-1H-pyrrole-2-yl)isoquinolin-3-yl)-4-(4-methylpiperazin-1-yl) benzamide |
| 8 | 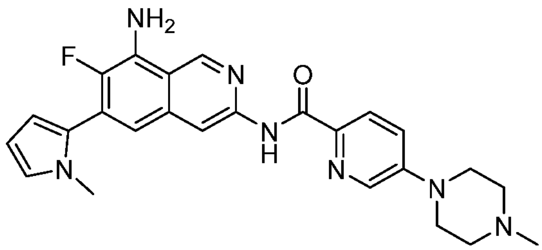 | N-(8-amino-7-fluoro-6-(1-methyl-1H-pyrrole-2-yl)isoquinolin-3-yl)-5-(4-methylpiperazin-1-yl) pyridinolinoamide |
| 9 | 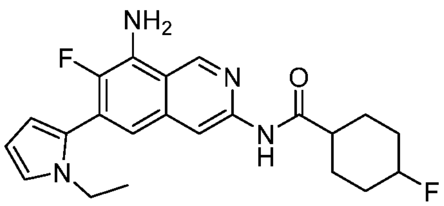 | N-(8-amino-6-(1-ethyl-1H-pyrrole-2-yl)-7-fluoroisoquinolin-3-yl)-4-fluorocyclohexan-1-carboxamide |
| 10 | 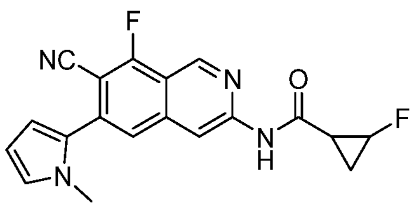 | N-(7 -cyano-8-fluoro-6-(1-methyl-1H-pyrrole-2-yl)isoquinolin-3-yl)-2-fluorocyclopropan-1-carboxamide |
| 11 | 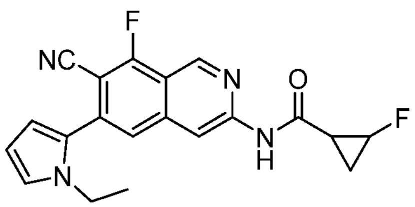 | N-(7-cyano-6-(1-ethyl-1H-pyrrole-2-yl)-8-fluoroi soquinolin-3-yl)-2-fluorocyclopropan-1-carboxamide |
| 12 | 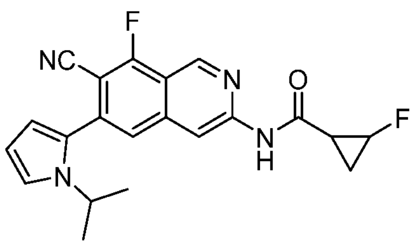 | N-(7-cyano-8-fluoro-6-(1-isopropyl-1H-pyrrole-2-yl)isoquinolin-3-yl)-2-fluorocyclopropan-1-carboxamide |

(continued)

| No. | Structure | Name |
|---|---|---|
| 13 | 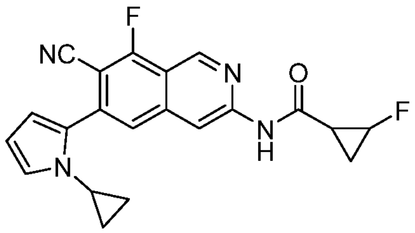 | N-(7-cyano-6-(1-cyclopropyl-1H-pyrrole-2-yl)-8-fluoroisoquinolin-3-yl)-2-fluorocyclopropan-1-carboxamide |
| 14 | 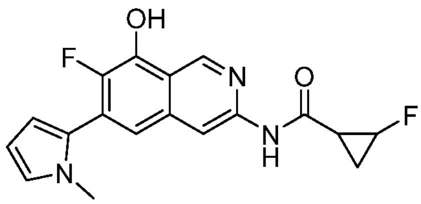 | 2-fluoro-N-(7-fluoro-8-hydroxy-6-(1-methyl-1H-pyrrole-2-yl)isoquinolin-3-yl)cyclopropan-1-carboxamide |
| 15 | 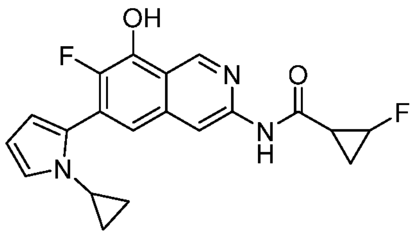 | N-(6-(1 -cyclopropyl-1H-pyrrole-2-yl)-7-fluoro-8-hydroxyisoquinolin-3-yl)-2-fluorocyclopropan-1-carboxamide |
| 16 | 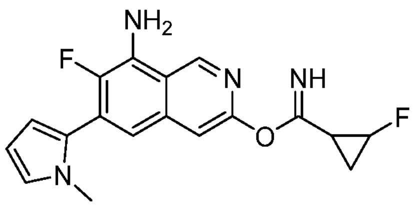 | 8-amino-7-fluoro-6-(1-methyl-1H-pyrrole-2-yl) isoquinolin-3-yl 2-fluorocyclopropan-1-carbamate |
| 17 | 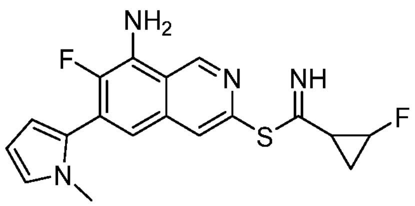 | 8-amino-7-fluoro-6-(1-methyl-1H-pyrrole-2-yl) isoquinolin-3-yl 2-fluorocyclopropan-1-carbonimidothioate |
| 18 | 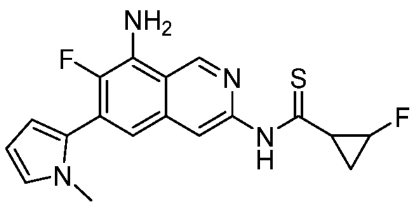 | N-(8-amino-7-fluoro-6-(1-methyl-1H-pyrrole-2-yl)isoquinolin-3-yl)-2-fluorocyclopropan-1-carbothioamide |
| 19 | 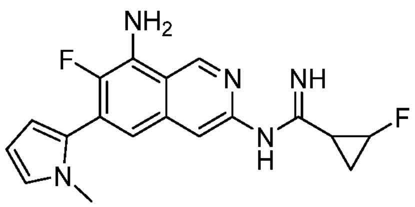 | N-(8-amino-7-fluoro-6-(1-methyl-1H-pyrrole-2-yl)isoquinolin-3-yl)-2-fluorocyclopropan-1-carboximide |

(continued)

| No. | Structure | Name |
|---|---|---|
| 20 | 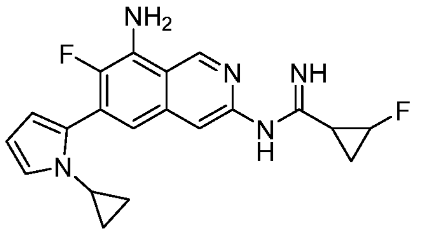 | N-(8-amino-6-(1-cyclopropyl-1H-pyrrole-2-yl)-7-fluoroisoquinolin-3-yl)-2-fluorocyclopropan-1-carboximide |
| 21 | 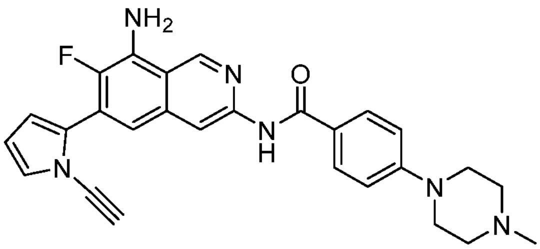 | N-(8-amino-6-(1-ethynyl-1H-pyrrole-2-yl)-7-fluoroisoquinolin-3-yl)-4-(4-methylpiperazin-1-yl)benzamide |
| 22 | 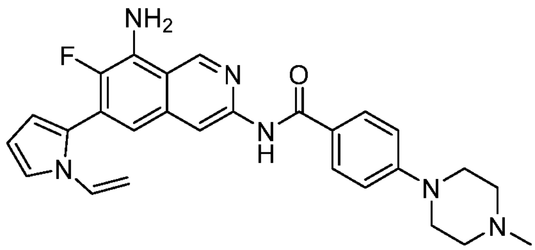 | N-(8-amino-7-fluoro-6-(1-ethenyl-1H-pyrrole-2-yl)isoquinolin-3-yl)-4-(4-methylpiperazin-1-yl) benzamide. |

**13.** The use of any one of claims 1-12, wherein the compound is

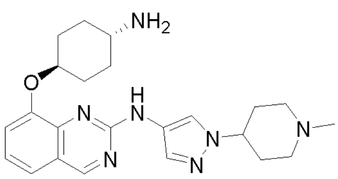

**C1**

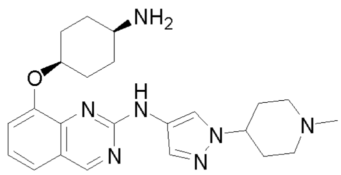

**C2**

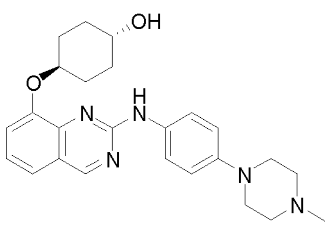

**C3**

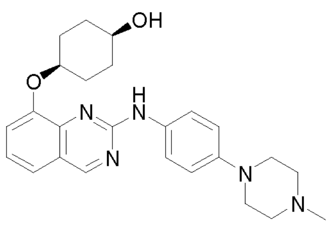

**C4**

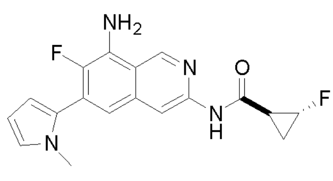

**C5**

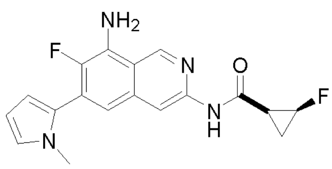

**C6**

.

**14.** The use of any one of claims 2-13, wherein the virus is selected from Hepatitis B virus, Measles Virus, Sindbis Virus, West Nile Virus, Dengue Virus, Herpes Simplex Virus, Human Cytomegalovirus HCMV, Ebola Virus, hepatitis C virus, Influenza A Virus, SARS-CoV, Zika Virus, HIV, Feline Infectious Peritonitis Virus, Mouse Hepatitis Virus, Canine Coronavirus, Feline Calicivirus, Feline Leukemia Virus, Feline Immunodeficiency Virus, Feline Panleukopenia Virus, Avian Infectious Bronchitis Virus, Transmissible Gastroenteritis Virus, Porcine Epidemic Diarrhea Virus, Porcine Hemagglutinating Encephalomyelitis Virus, Bovine Coronavirus, and the like; preferably, Feline Infectious Peritonitis Virus, Mouse Hepatitis Virus, Herpes Simplex Virus, SARS-CoV, Zika Virus, Feline Infectious Peritonitis Virus, Canine Coronavirus, Feline Calicivirus, Avian Infectious Bronchitis Virus, Porcine Epidemic Diarrhea Virus.

**15.** A pharmaceutical composition comprising a compound of any one of claims 1-13 or a pharmaceutically acceptable salt, solvate, stereoisomer, tautomer, prodrug, metabolite or derivative thereof, and one or more other antiviral drugs, as well as optionally a pharmaceutically acceptable excipient.

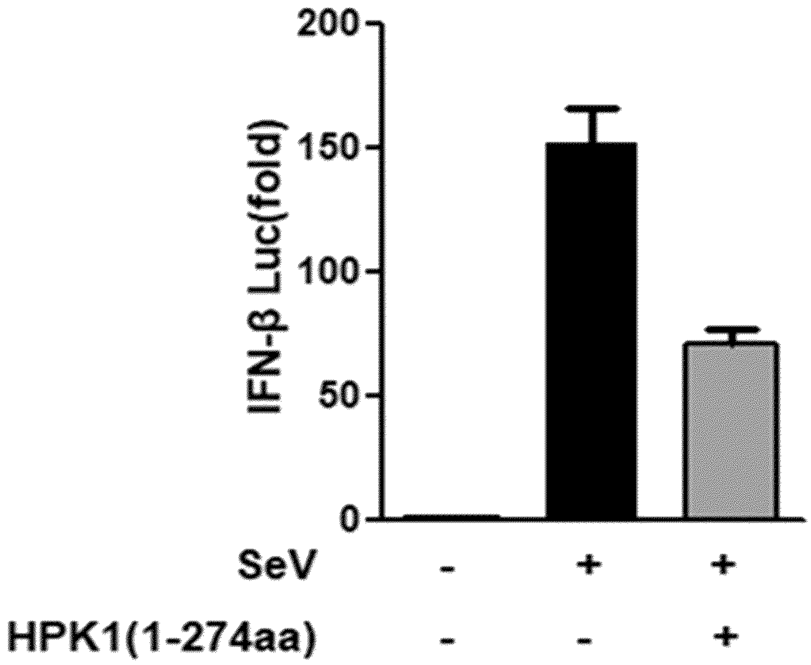
200
150
100
50
0
IFN-β Luc(fold)
SeV
HPK1(1-274aa)
-
+
+
-
-
+

Fig. 1

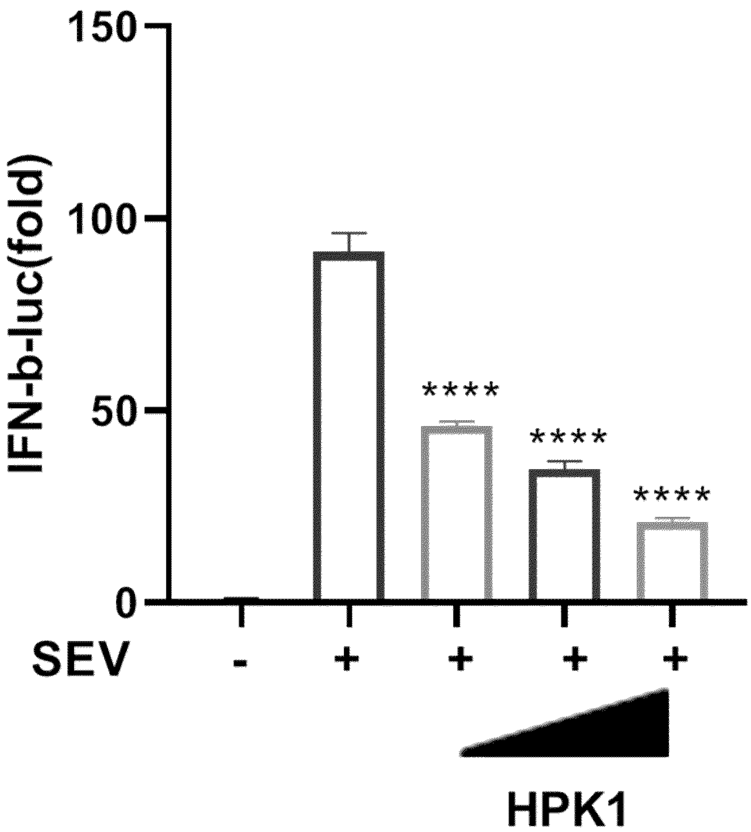
150
100
50
0
IFN-b-luc(fold)
****
****
****
SEV
-
+
+
+
+
HPK1

Fig. 2

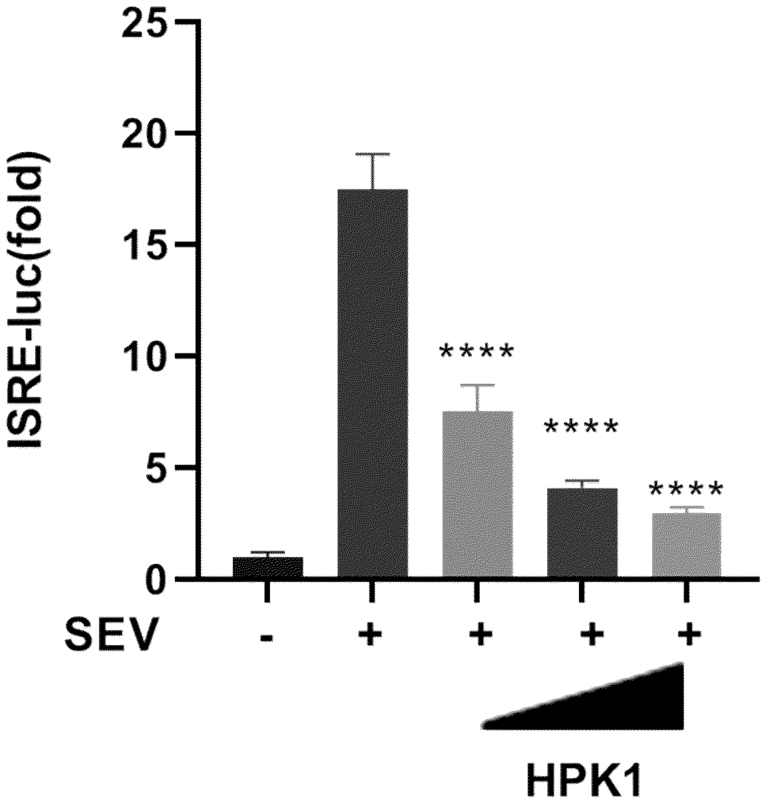
25
20
15
10
5
0
ISRE-luc(fold)
****
****
****
SEV
-
+
+
+
+
HPK1

Fig. 3

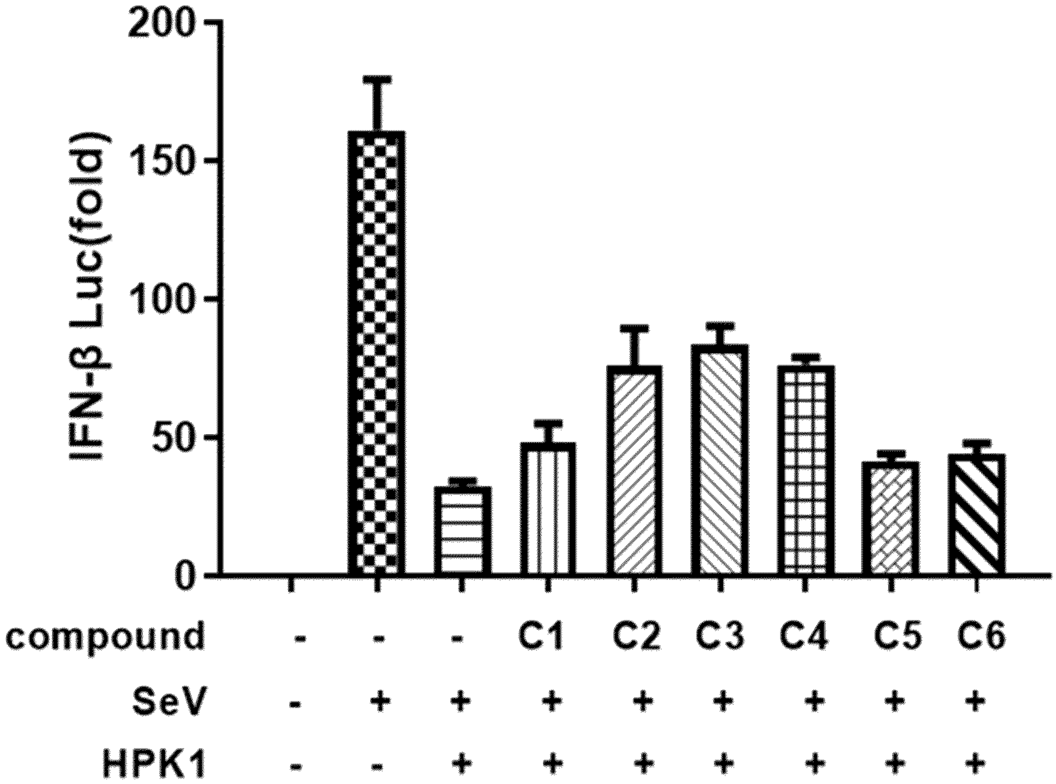
200
150
100
50
0
IFN-β Luc(fold)
compound
-
-
-
C1
C2
C3
C4
C5
C6
SeV
-
+
+
+
+
+
+
+
+
HPK1
-
-
+
+
+
+
+
+
+

Fig. 4

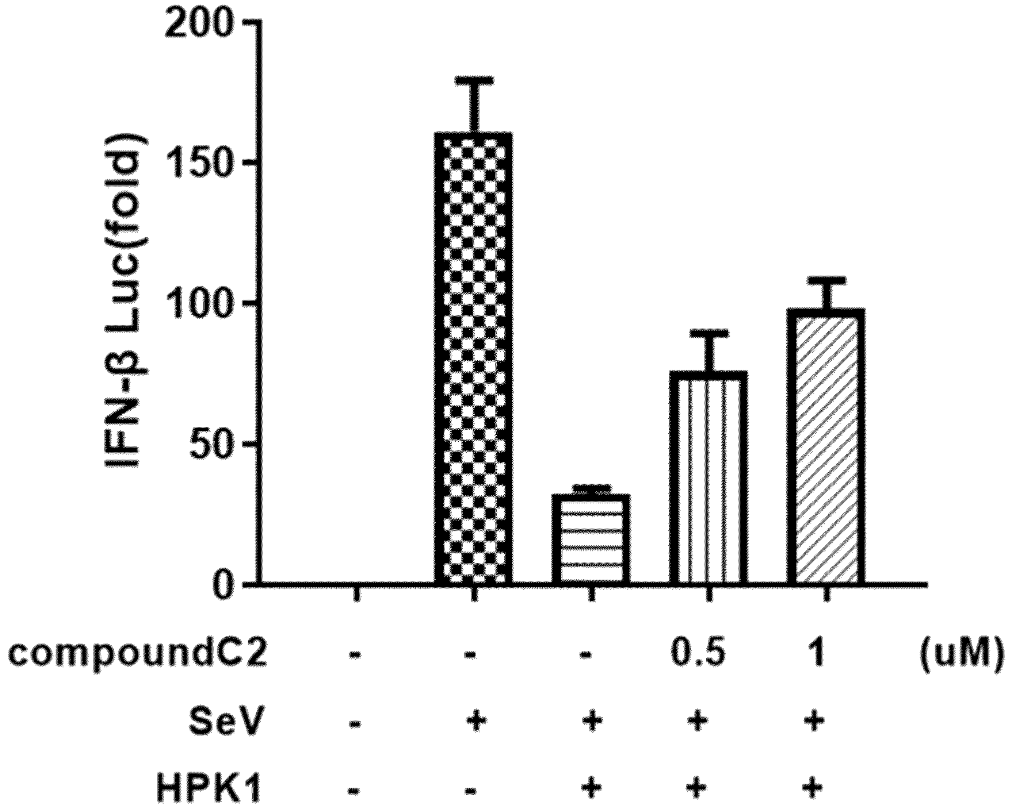
IFN-β Luc(fold)
200
150
100
50
0
compoundC2 - - - 0.5 1 (uM)
SeV - + + + +
HPK1 - - + + +

Fig. 5

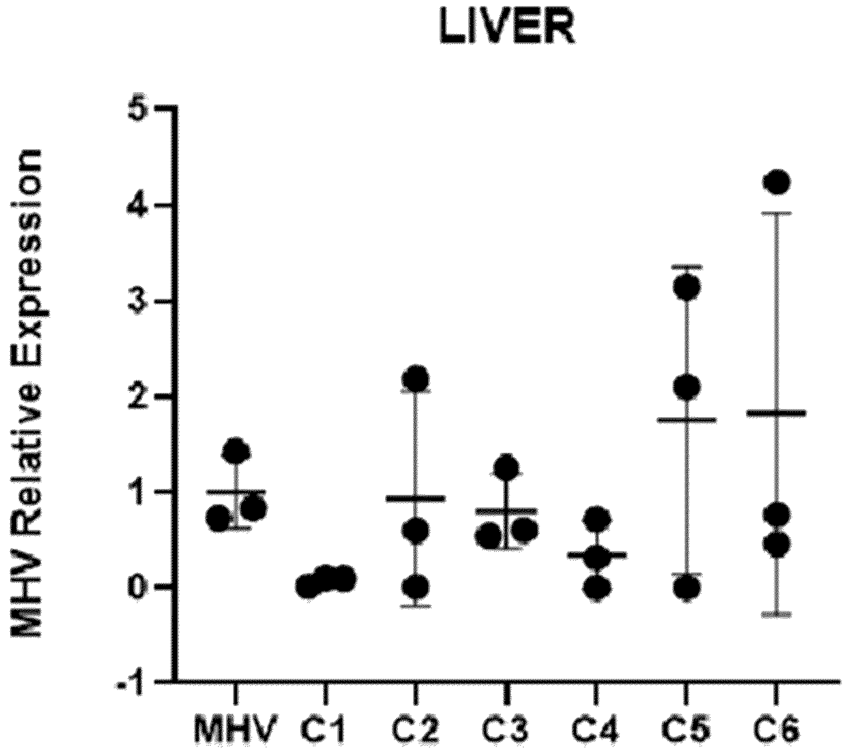
LIVER
MHV Relative Expression
5
4
3
2
1
0
-1
MHV C1 C2 C3 C4 C5 C6

Fig. 6

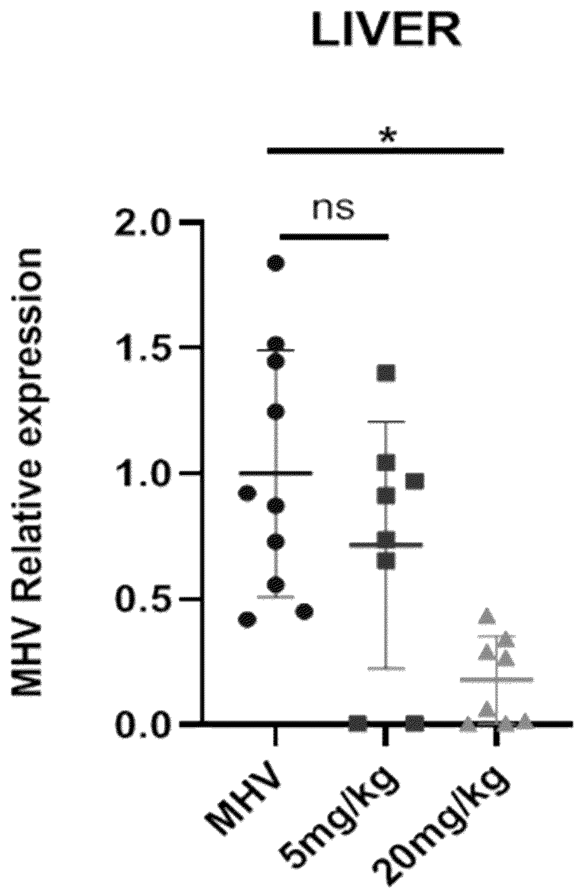
LIVER
*
ns
MHV Relative expression
2.0
1.5
1.0
0.5
0.0
MHV
5mg/kg
20mg/kg

Fig. 7

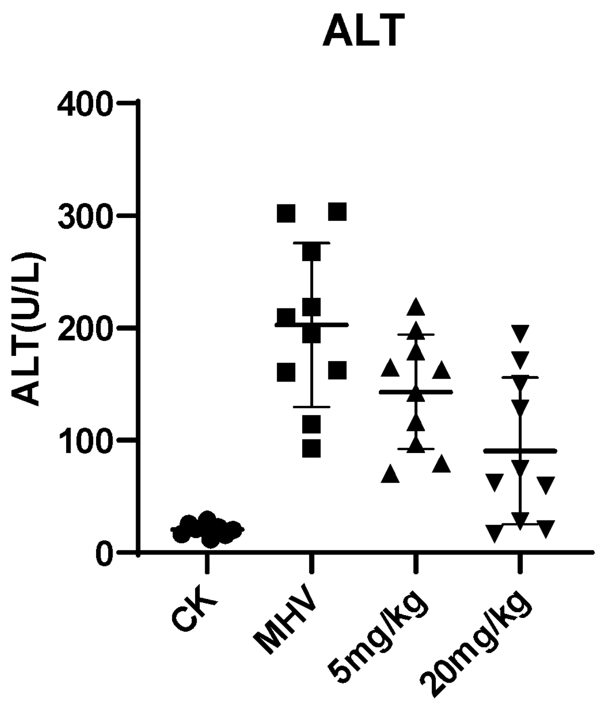
ALT
ALT(U/L)
400
300
200
100
0
CK
MHV
5mg/kg
20mg/kg

Fig. 8

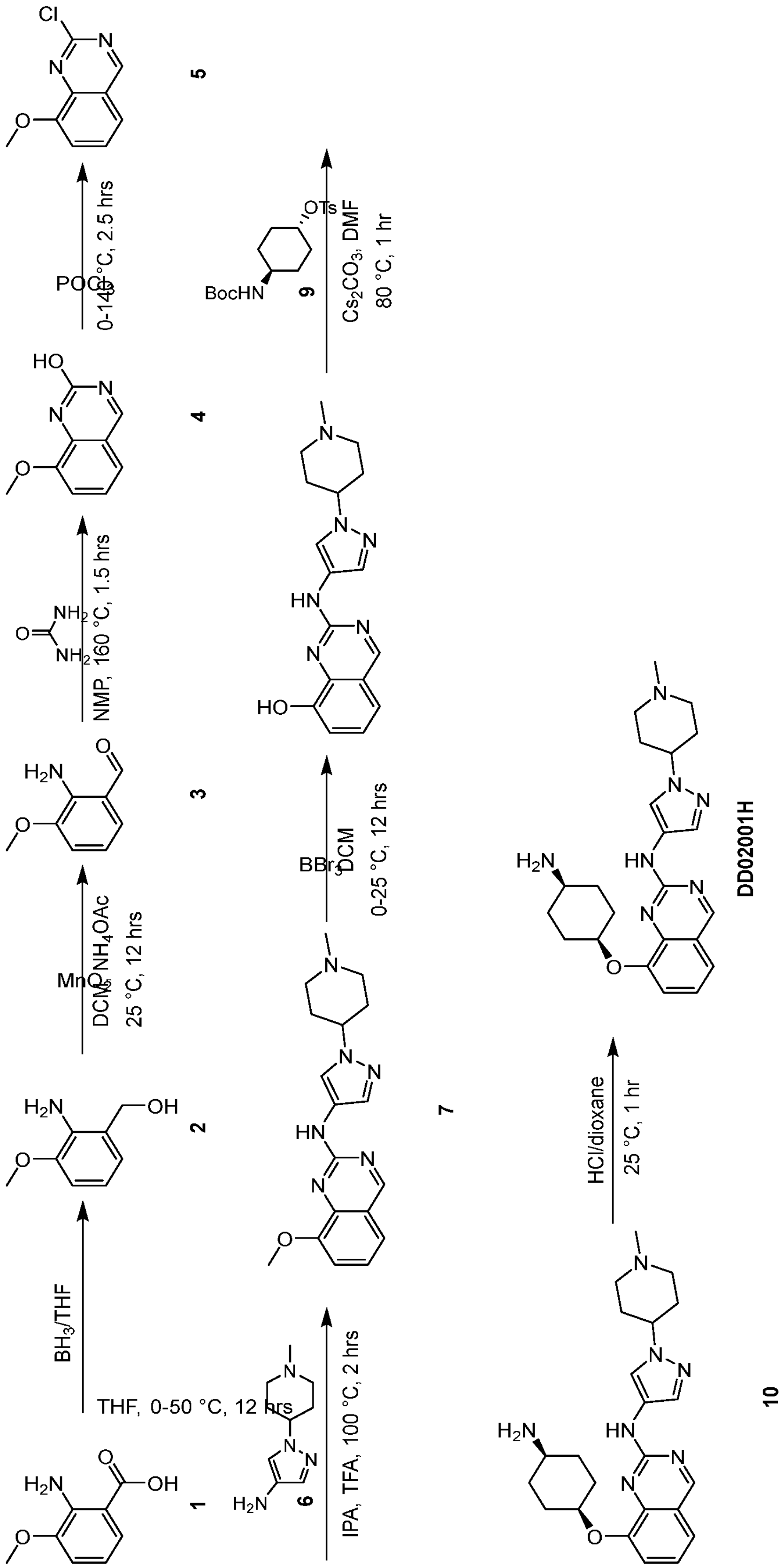
1
BH3/THF
THF, 0-50 °C, 12 hrs
2
MnO2, NH4OAc
DCM, 25 °C, 12 hrs
3
NMP, 160 °C, 1.5 hrs
4
POCl3
0-140 °C, 2.5 hrs
5
6
IPA, TFA, 100 °C, 2 hrs
7
BBr3, DCM
0-25 °C, 12 hrs
9
Cs2CO3, DMF
80 °C, 1 hr
10
HCl/dioxane
25 °C, 1 hr
DD02001H

Fig. 9

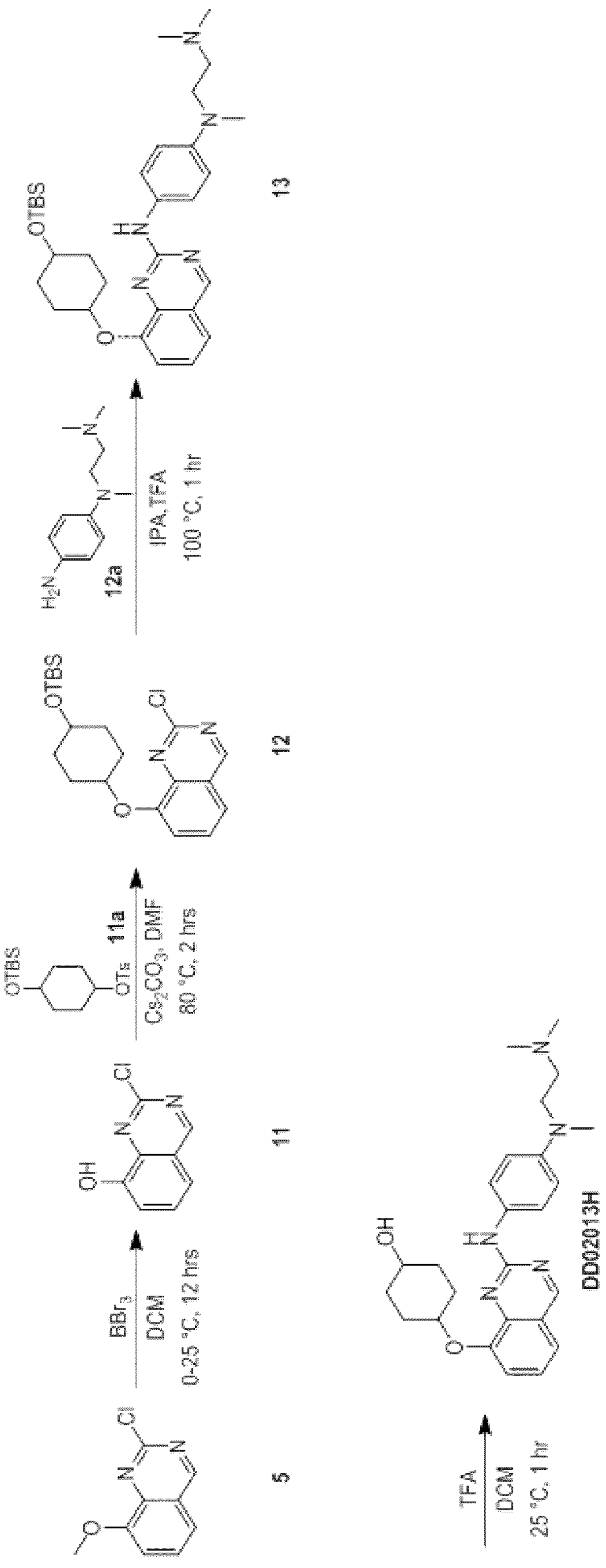


5 11 12 13 DD02013H

Fig. 10

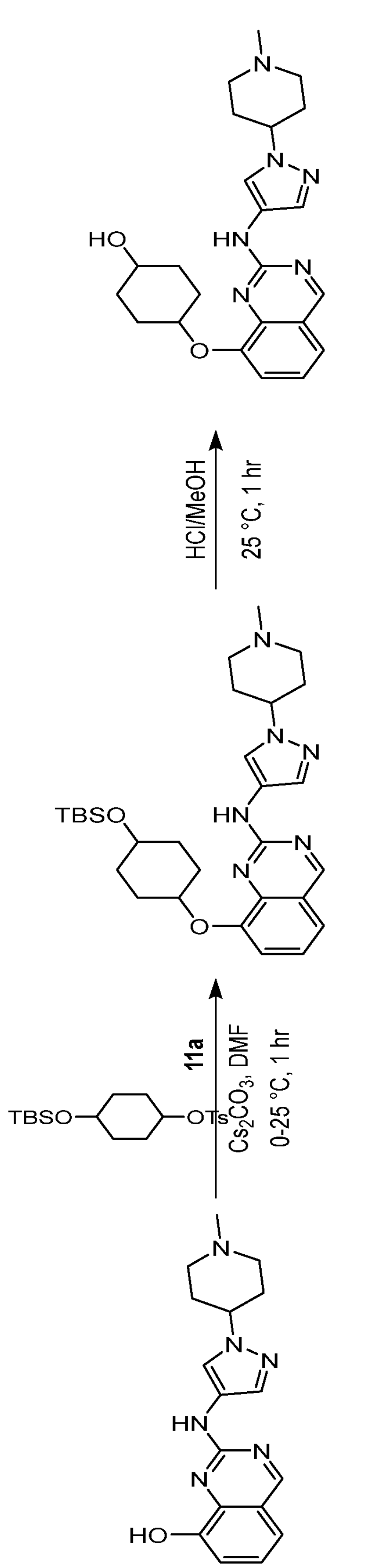


8

14

DD02014H

Fig. 11

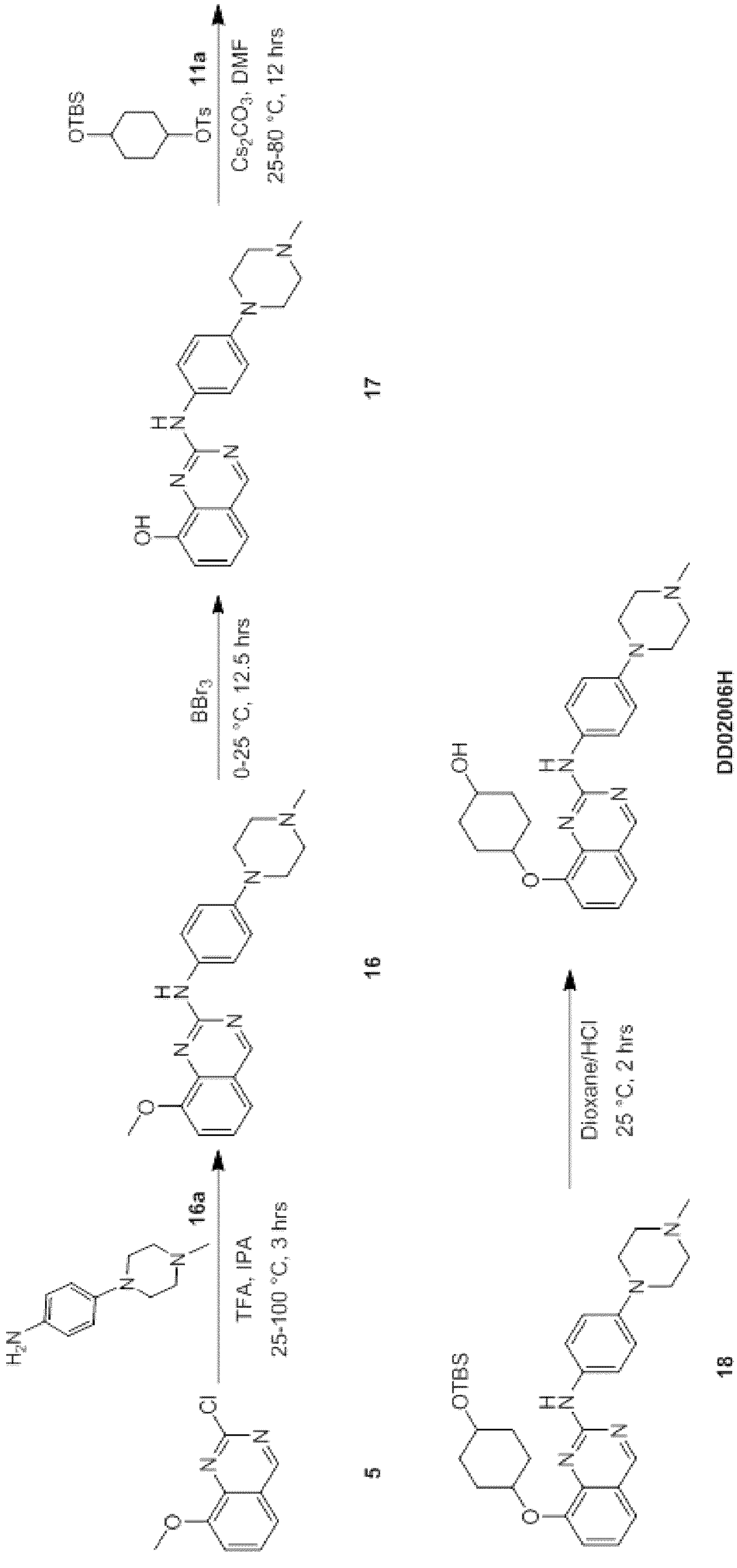


5

16

17

18

DD02006H

Fig. 12

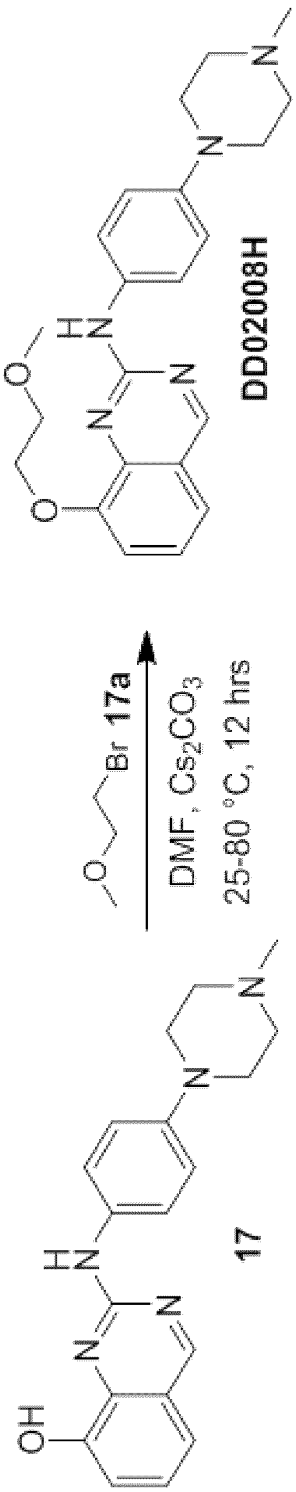


17

DD02008H

Fig. 13

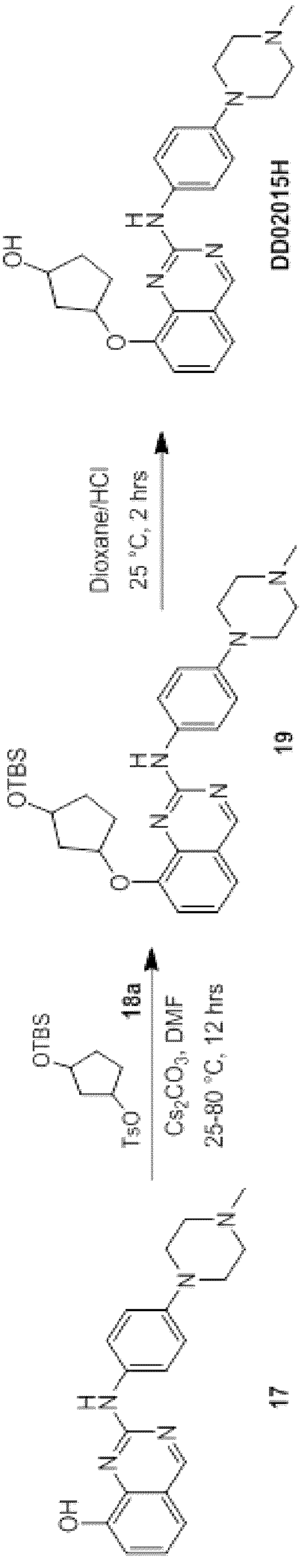
17
18a
$Cs_2CO_3$, DMF
25-80 °C, 12 hrs
19
Dioxane/HCl
25 °C, 2 hrs
DD02015H

Fig. 14

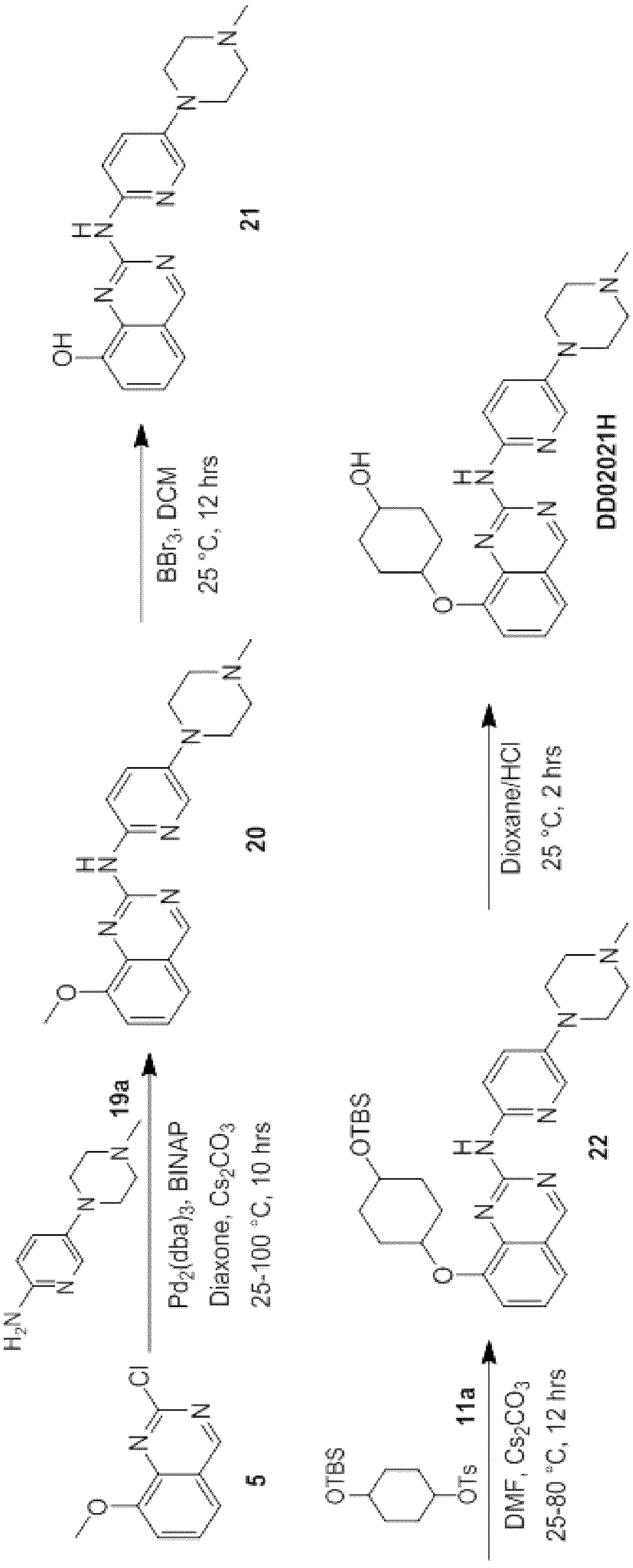
5
19a
Pd$_2$(dba)$_3$, BINAP
Diaxone, Cs$_2$CO$_3$
25-100 °C, 10 hrs
20
BBr$_3$, DCM
25 °C, 12 hrs
21
11a
DMF, Cs$_2$CO$_3$
25-80 °C, 12 hrs
22
Dioxane/HCl
25 °C, 2 hrs
DD02021H

Fig. 15

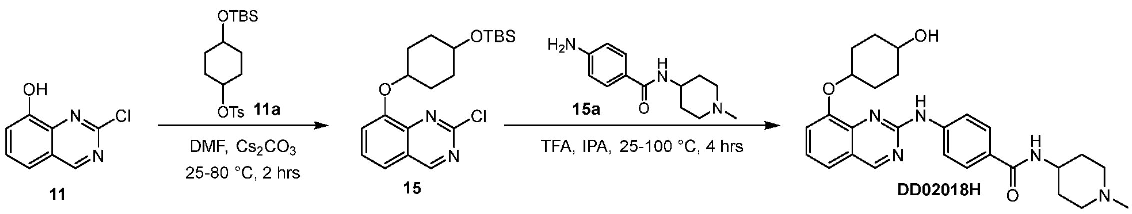


11 15 DD02018H

Fig. 16

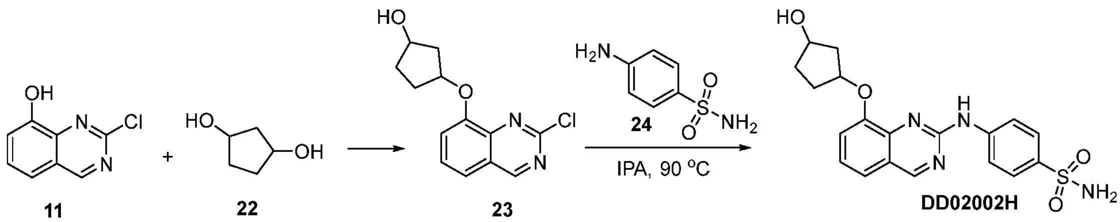


11 22 23 DD02002H

Fig. 17

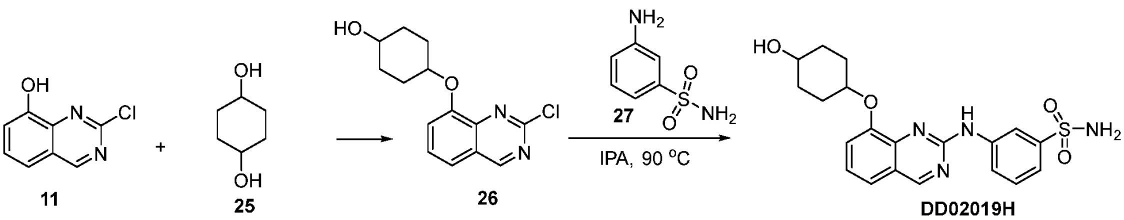


11 25 26 DD02019H

Fig. 18

| INTERNATIONAL SEARCH REPORT | International application No. PCT/IB2023/051675 |
|---|---|

**A. CLASSIFICATION OF SUBJECT MATTER**

A61K31/517(2006.01)i; A61K31/5377(2006.01)i; A61K31/4725(2006.01)i; A61K31/496(2006.01)i; A61P31/12(2006.01)i

According to International Patent Classification (IPC) or to both national classification and IPC

**B. FIELDS SEARCHED**

Minimum documentation searched (classification system followed by classification symbols)

IPC: A61K A61P

Documentation searched other than minimum documentation to the extent that such documents are included in the fields searched

Electronic data base consulted during the international search (name of data base and, where practicable, search terms used)

CNTXT, WPABS, WPABSC, OETXT, VEN, CJFD, 中国药物专利数据库, CHINESE PHARMACEUTICAL PATENT DATABASE, CNKI, 百度学术, BAIDU SCHOLAR, Medline, ISI-WEB OF SCIENCE, REGISTRY, CAPLUS: 结构式检索, search for structural formula, 喹唑啉, 造血祖细胞激酶, 病毒, HPK1, MAP4K1, quinazoline, virus

**C. DOCUMENTS CONSIDERED TO BE RELEVANT**

| Category* | Citation of document, with indication, where appropriate, of the relevant passages | Relevant to claim No. |
|---|---|---|
| PX | CN 114315798 A (SHENZHEN INTELLIGENT MEDICINE INFORMATION TECHNOLOGY LIMITED COMPANY) 12 April 2022 (2022-04-12) see claims 1-10 | 15 (in part) |
| X | KR 20210143131 A (KOREA RESEARCH INSTITUTE OF CHEMICAL TECHNOLOGY et al.) 26 November 2021 (2021-11-26) see claims 1-11, description embodiment 2-1 | 1-7 (in part), 13-15 (in part) |
| X | CN 101454294 A (NOVARTIS AG) 10 June 2009 (2009-06-10) see claims 1-27 | 15 (in part) |
| X | CN 105358549 A (CARNA BIOSCIENCES INC. et al.) 24 February 2016 (2016-02-24) see claims 1-5 | 15 (in part) |
| A | CN 113797202 A (ZHUHAI YUFAN BIOTECHNOLOGIES CO., LTD.) 17 December 2021 (2021-12-17) see claims 1, 12-13, description paragraphs 0005-0006, 0081-0111 | 1-2 (in part), 14-15 (in part) |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **28 June 2023** | **14 July 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/IB2023/051675**

| C. DOCUMENTS CONSIDERED TO BE RELEVANT | | |
|---|---|---|
| Category* | Citation of document, with indication, where appropriate, of the relevant passages | Relevant to claim No. |
| A | CN 110709392 A (F. HOFFMANN-LA ROCHE AG) 17 January 2020 (2020-01-17) see claims 1-60 | 1-15 (in part) |

Form PCT/ISA/210 (second sheet) (July 2022)

**INTERNATIONAL SEARCH REPORT**

International application No.
**PCT/IB2023/051675**

**Box No. III Observations where unity of invention is lacking (Continuation of item 3 of first sheet)**

This International Searching Authority found multiple inventions in this international application, as follows:

Invention 1: Claims 1-3 (in part), claims 4-7 (in part), and claims 13-15 (in part), wherein in a compound of formula 1, Z1 is N, Z2 is C, Z3 is N, and the use of the compound is to treat or prevent virus infection.

Invention 2: Claims 1-3 (in part), claims 4-7 (in part), claim 13 (in part), and claim 15 (in part), wherein in a compound of formula 1, Z1 is N, Z2 is C, Z3 is N, and the use of the compound is to treat or prevent other interferon-related diseases other than virus infection.

Invention 3: Claims 1-3 (in part), claims 4-7 (in part), claim 13 (in part), and claim 15 (in part), wherein in a compound of formula 1, Z1 is N, Z2 is C, Z3 is N, and the use of the compound is to improve immunity.

Invention 4: Claims 1-3 (in part) and claims 14-15 (in part), wherein in a compound of formula 1, Z1 is N, Z2 is N, Z3 is C, and the use of the compound is to treat or prevent virus infection.

Invention 5: Claims 1-3 (in part) and claim 15 (in part), wherein in a compound of formula 1, Z1 is N, Z2 is N, Z3 is C, and the use of the compound is to treat or prevent other interferon-related diseases other than virus infection.

Invention 6: Claims 1-3 (in part) and claim 15 (in part), wherein in a compound of formula 1, Z1 is N, Z2 is N, Z3 is C, and the use of the compound is to improve immunity.

Invention 7: Claims 1-3 (in part), claims 8-12 (in part), and claims 13-15 (in part), wherein in a compound of formula 1, Z1 is C, Z2 is N, Z3 is C, and the use of the compound is to treat or prevent virus infection.

Invention 8: Claims 1-3 (in part), claims 8-12 (in part), claim 13 (in part), and claim 15 (in part), wherein in a compound of formula 1, Z1 is C, Z2 is N, Z3 is C, and the use of the compound is to treat or prevent other interferon-related diseases other than virus infection.

Invention 9: Claims 1-3 (in part), claims 8-12 (in part), claim 13 (in part), and claim 15 (in part), wherein in a compound of formula 1, Z1 is C, Z2 is N, Z3 is C, and the use of the compound is to improve immunity.

Invention 10: Claims 1-3 (in part) and claims 14-15 (in part), wherein in a compound of formula 1, Z1 is C, Z2 is C, Z3 is N, and the use of the compound is to treat or prevent virus infection.

Invention 11: Claims 1-3 (in part) and claim 15 (in part), wherein in a compound of formula 1, Z1 is C, Z2 is C, Z3 is N, and the use of the compound is to treat or prevent other interferon-related diseases other than virus infection.

Invention 12: Claims 1-3 (in part) and claim 15 (in part), wherein in a compound of formula 1, Z1 is C, Z2 is C, Z3 is N, and the use of the compound is to improve immunity.

The compounds involved in the 12 inventions have different main structures which are all known chemical structures, and have different pharmaceutical uses, so that the 12 inventions do not have a same or similar special technical feature, and do not meet the requirement of unity of invention (PCT Rule 13.2).

**INTERNATIONAL SEARCH REPORT**

International application No.
**PCT/IB2023/051675**

**Box No. III Observations where unity of invention is lacking (Continuation of item 3 of first sheet)**

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **claims 1-3 (in part), claims 4-7 (in part), claims 13-15 (in part)**

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2022)

**INTERNATIONAL SEARCH REPORT**
**Information on patent family members**

International application No.
**PCT/IB2023/051675**

| Patent document cited in search report | Publication date (day/month/year) | Patent family member(s) | Publication date (day/month/year) |
|---|---|---|---|
| CN 114315798 A | 12 April 2022 | None | |
| KR 20210143131 A | 26 November 2021 | None | |
| CN 101454294 A | 10 June 2009 | GT 200800202 A | 25 June 2009 |
| | | PE 20080059 A1 | 05 March 2008 |
| | | JP 2009532486 A | 10 September 2009 |
| | | SMAP 200800059 A | 12 November 2008 |
| | | SMP 200800059 B | 07 September 2009 |
| | | AR 060358 A1 | 11 June 2008 |
| | | TW 200808739 A | 16 February 2008 |
| | | US 2010048561 A1 | 25 February 2010 |
| | | US 7932262 B2 | 26 April 2011 |
| | | EP 2004615 A2 | 24 December 2008 |
| | | KR 20080111114 A | 22 December 2008 |
| | | ECSP 088823 A | 30 December 2008 |
| | | TNSN 08375 A1 | 29 December 2009 |
| | | AU 2007235339 A1 | 18 October 2007 |
| | | AU 2007235339 A8 | 20 November 2008 |
| | | NO 20084665 L | 06 January 2009 |
| | | BRPI 0710521 A2 | 13 March 2012 |
| | | EA 200870415 A1 | 27 February 2009 |
| | | CA 2648529 A1 | 18 October 2007 |
| | | MA 30358 B1 | 01 April 2009 |
| | | WO 2007117607 A2 | 18 October 2007 |
| | | WO 2007117607 A3 | 21 December 2007 |
| | | WO 2007117607 A9 | 06 March 2008 |
| | | CR 10427 A | 27 January 2009 |
| | | MX 2008012852 A | 28 November 2008 |
| | | ZA 200808307 B | 27 January 2010 |
| CN 105358549 A | 24 February 2016 | KR 20160093543 A | 08 August 2016 |
| | | KR 102338568 B1 | 13 December 2021 |
| | | JP 6492012 B2 | 27 March 2019 |
| | | ES 2924480 T3 | 07 October 2022 |
| | | WO 2015083833 A1 | 11 June 2015 |
| | | EP 3078660 A1 | 12 October 2016 |
| | | EP 3078660 A4 | 17 May 2017 |
| | | EP 3078660 B1 | 13 July 2022 |
| | | US 2016264555 A1 | 15 September 2016 |
| | | US 9682961 B2 | 20 June 2017 |
| | | IN 201503339 P3 | 08 July 2016 |
| | | JP WO2015083833 A1 | 16 March 2017 |
| | | IN 353313 B | 11 December 2020 |
| CN 113797202 A | 17 December 2021 | CA 3182850 A1 | 23 December 2021 |
| | | WO 2021254118 A1 | 23 December 2021 |
| | | BR 112022025714 A2 | 03 January 2023 |
| | | AU 2021290594 A1 | 02 February 2023 |
| | | KR 20230026387 A | 24 February 2023 |
| | | EP 4154881 A1 | 29 March 2023 |
| CN 110709392 A | 17 January 2020 | IL 268994 A | 31 October 2019 |
| | | CR 20190424 A | 04 November 2019 |

Form PCT/ISA/210 (patent family annex) (July 2022)

**INTERNATIONAL SEARCH REPORT**
**Information on patent family members**

International application No.
**PCT/IB2023/051675**

| Patent document cited in search report | Publication date (day/month/year) | Patent family member(s) | Publication date (day/month/year) |
|---|---|---|---|
| | | WO 2018183964 A1 | 04 October 2018 |
| | | RU 2019133646 A | 30 April 2021 |
| | | RU 2019133646 A3 | 30 July 2021 |
| | | PE 20200008 A1 | 06 January 2020 |
| | | CA 3054161 A1 | 04 October 2018 |
| | | CL 2020000856 A1 | 21 August 2020 |
| | | EP 3601259 A1 | 05 February 2020 |
| | | EP 3601259 B1 | 23 February 2022 |
| | | US 2021163417 A1 | 03 June 2021 |
| | | US 11566003 B2 | 31 January 2023 |
| | | MA 48994 A | 05 February 2020 |
| | | PH 12019502258 A1 | 06 July 2020 |
| | | SG 11201908940 UA | 30 October 2019 |
| | | CO 2019009927 A2 | 30 September 2019 |
| | | TW 201843139 A | 16 December 2018 |
| | | SG 10201914112 VA | 30 March 2020 |
| | | BR 112019019555 A2 | 22 April 2020 |
| | | AU 2018243770 A1 | 19 September 2019 |
| | | KR 20190135029 A | 05 December 2019 |
| | | MX 2019010302 A | 21 November 2019 |
| | | JP 2020515603 A | 28 May 2020 |
| | | JP 7129420 B2 | 01 September 2022 |
| | | CL 2019002734 A1 | 17 January 2020 |
| | | US 2018282282 A1 | 04 October 2018 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2020255022 A


### Non-patent literature cited in the description


- **HERNANDEZ et al.** *Cell Reports*, 2018
- **BERGE et al.** Pharmaceutical Salts. *J. Pharm. Sci.*, 1977, vol. 66, 1-19